(19) Europäisches Patentamt
European Patent Office
Office européen des brevets

(11) EP 0 920 441 B1

(12) **EUROPÄISCHE PATENTSCHRIFT**

(45) Veröffentlichungstag und Bekanntmachung des Hinweises auf die Patenterteilung:
**29.01.2003 Patentblatt 2003/05**

(21) Anmeldenummer: **97943814.0**

(22) Anmeldetag: **20.08.1997**

(51) Int Cl.7: **C07J 53/00**, A61K 31/565, C07J 41/00, C07J 43/00, A61K 31/58

(86) Internationale Anmeldenummer:
**PCT/EP97/04517**

(87) Internationale Veröffentlichungsnummer:
**WO 98/007740 (26.02.1998 Gazette 1998/08)**

(54) **7ALPHA-(EPSILON-AMINOALKYL)-ESTRATRIENE, VERFAHREN ZU DEREN HERSTELLUNG, PHARMAZEUTISCHE PRÄPARATE, DIE DIESE 7ALPHA-(EPSILON-AMINOALKYL)-ESTRATRIENE ENTHALTEN SOWIE DEREN VERWENDUNG ZUR HERSTELLUNG VON ARZNEIMITTELN**

7ALPHA-(EPSILON-AMINOALKYL)-ESTRATRIENES, PROCESS FOR PREPARING THE SAME, PHARMACEUTICAL PREPARATIONS CONTAINING SAID 7ALPHA-(EPSILON-AMINOALKYL)-ESTRATRIENES AND THEIR USE FOR PREPARING MEDICAMENT

7ALPHA-(EPSILON-AMINOALKYL)-ESTRATRIENES, LEUR PROCEDE DE PRODUCTION, PREPARATIONS PHARMACEUTIQUES LES CONTENANT, AINSI QUE LEUR UTILISATION POUR LA FABRICATION DE MEDICAMENTS

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **20.08.1996 DE 19635525**

(43) Veröffentlichungstag der Anmeldung:
**09.06.1999 Patentblatt 1999/23**

(73) Patentinhaber: **SCHERING AKTIENGESELLSCHAFT**
**13353 Berlin (DE)**

(72) Erfinder:
- **BOHLMANN, Rolf**
  **14055 Berlin (DE)**
- **BITTLER, Dieter**
  **13503 Berlin (DE)**
- **HEINDI, Josef**
  **14129 Berlin (DE)**
- **HEINRICH, Nikolaus**
  **12159 Berlin (DE)**
- **HOFMEISTER, Helmut**
  **13465 Berlin (DE)**
- **KÜNZER, Hermann**
  **13347 Berlin (DE)**
- **SAUER, Gerhard**
  **13465 Berlin (DE)**
- **HEGELEHARTUNG, Christa**
  **10179 Berlin (DE)**
- **LICHTNER, Rosemarie**
  **10823 Berlin (DE)**
- **NISHINO, Yukishige**
  **14089 Berlin (DE)**
- **PARCZYK, Karsten**
  **12207 Berlin (DE)**
- **SCHNEIDER, Martin**
  **13469 Berlin (DE)**

(56) Entgegenhaltungen:
**EP-A- 0 138 504** **EP-A- 0 367 576**
**EP-A- 0 410 554** **DE-A- 4 218 743**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- **A. FRENCH ET AL: "A synthesis of 7.alpha.-substituted estradiols: synthesis and biological evaluation of a 7.alpha.-pentyl-substituted BODIPY fluorescent conjugate and a fluorine-18-labeled 7.alpha.-pentylestradiol analogue" STEROIDS: STRUCTURE, FUNCTION, AND REGULATION., Bd. 58, Nr. 4, April 1993, MA US, Seiten 157-169, XP002051815**
- **CHEMICAL ABSTRACTS, vol. 104, no. 21, 26.Mai 1986 Columbus, Ohio, US; abstract no. 186697, MUEHLENBRUCH B ET AL: "Synthesis of estradiol haptens" XP002051816 & ARCH. PHARM., Bd. 319, Nr. 2, 1986, Seiten 177-183,**


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft substituierte 7α-(ξ-Aminoalkyl)-estratriene der allgemeinen Formel I

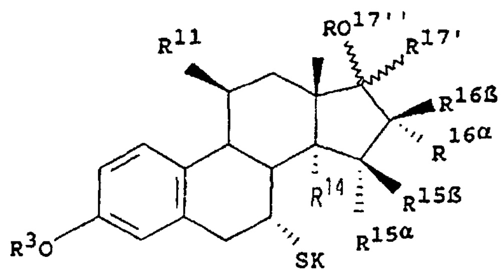

(I)

worin
die Seitenkette SK einen Rest der Teilformel

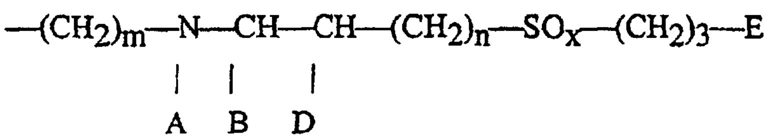

wobei

m 4, 5 oder 6,
n 0,1 oder 2,
x 0, 1 oder 2,
A ein Wasserstoffatom oder eine $C_{1-5}$-Alkylgruppe,
B und D je ein Wasserstoffatom, oder
A und B gemeinsam eine Alkylengruppe $—(CH_2)_p—$ mit p= 2, 3, 4 oder 5 und D ein Wasserstoffatom oder
A und D gemeinsam eine Alkylengruppe $—(CH_2)_q—$ mit q= 2, 3 oder 4 und B ein Wasserstoffatom, und
E ein unsubstituierter oder ein- bis fünffach fluorierter Ethylrest ist, oder der terminale Substituent $-(CH_2)_3$-E in der Seitenkette durch einen gegebenenfalls substituierten Aryl- oder Heteroarylrest, der direkt oder über eine Mono-, Di- oder Trimethylengruppe an das Schwefelatom gebunden ist, ersetzt ist,

$R^3$ ein Wasserstoffatom, einen Kohlenwasserstoffrest mit bis zu 8 Kohlenstoffatomen oder einen Rest der Teilformel $R^{3'}$—C(O)—, worin $R^{3'}$ ein Wasserstoffatom oder ein Kohlenwasserstoffrest mit bis zu 8 Kohlenstoffatomen oder ein Phenylrest ist,
$R^{11}$ ein Wasserstoffatom, ein Halogenatom oder eine Nitrooxygruppe—$O\text{-}NO_2$,
$R^{14}$, $R^{15\alpha}$, $R^{15\beta}$, $R^{16\alpha}$ und $R^{16\beta}$ je ein Wasserstoffatom oder
$R^{14}$ und $R^{15\alpha}$ eine zusätzliche Bindung oder eine Methylenbrücke, oder
$R^{15\beta}$ eine Methylgruppe und $R^{15\alpha}$ ein Wasserstoffatom, oder
$R^{15\alpha}$ und $R^{15\beta}$ jeweils eine Methylgruppe, oder
$R^{15\beta}$ und $R^{16\beta}$ gemeinsam eine Methylenbrücke, oder
$R^{16\alpha}$ oder $R^{16\beta}$ ein Halogenatom oder
$R^{16\alpha}$ und $R^{16\beta}$ gemeinsam eine Methylidengruppe
und die übrigen der Substituenten $R^{14}$, $R^{15\alpha}$, $R^{15\beta}$, $R^{16\alpha}$ und $R^{16\beta}$ je ein Wasserstoffatom,
$R^{17'}$ in α- oder β-Position ein Wasserstoffatom, eine $C_{1-5}$-Alkyl-, $C_{2-5}$-Alkenyl- oder $C_{2-5}$-Alkinylgruppe oder eine Trifluonriethylgrupp und
$R^{17''}$ ein Wasserstoffatom oder einen Rest der Teilformel $R^{17'''}$—C(O)—, worin $R^{17'''}$ ein Wasserstoffatom oder ein Kohlenwasserstoffrest mit bis zu 8 Kohlenstoffatomen ist, oder,

wenn $R^{17'}$ sich in der α-Position befindet, $R^{17'}$ gemeinsam mit $R^{14}$ eine Ethanobrücke bedeutet,
mit der Maßgabe, daß, wenn nicht A und B gemeinsam für $-(CH_2)_p-$ oder A und D gemeinsam für $-(CH_2)_q-$ stehen,

mindestens einer der Substituenten $R^{11}$, $R^{14}$, $R^{15\alpha}$, $R^{15\beta}$, $R^{16\alpha}$ und $R^{16\beta}$, nicht ein Wasserstoffatom ist, sowie deren physiologisch verträgliche Additionssalze mit organischen und anorganischen Säuren.

Die vorliegende Erfindung betrifft außerdem diese Verbindungen der allgemeinen Formel I sowie deren physiologisch verträgliche Additionssalze mit organischen und anorganischen Säuren enthaltende pharmazeutische Präparate sowie deren Verwendung zur Herstellung von Arzneimitteln.

Vorzugsweise ist in den Verbindungen der allgemeinen Formel I das Stickstoffatom in der Seitenkette durch 5 Methylengruppen vom Kohlenstoffatom 7 des Steroidgerüsts getrennt.

Steht A für eine Alkylgruppe mit bis zu 5 Kohlenstoffatomen, ist dies eine Methyl-, Ethyl-, Propyl-, Isopropyl-, Butyl-, Isobutyl-, tert.-Butyl-, Pentyl-, Isopentyl-, oder Neopentylgruppe; als A ist eine Methylgruppe bevorzugt.

Der Index n kann den Wert 0, 1 oder 2 annehmen, wobei wenn A ein Wasserstoffatom oder eine Alkylgruppe mit bis zu 5 Kohlenstoffatomen ist, der Wert 1 für n bevorzugt ist, so daß das Stichstoffatom und das Schwefelatom durch 3 Methylengruppen getrennt sind.

Das Stickstoffatom kann Bestandteil eines 4 bis 7 gliedrigen bzw. 5 bis 7 gliedrigen Heterocyclus sein, der in 2- bzw. 3-Position mit dem Rest der Seitenkette $-(CH_2)_n-SO_x-(CH_2)_3-E$ substituiert ist.

Vorzugsweise sind A und B gemeinsam eine Trimethylengruppe, d.h. sie bilden gemeinsam mit dem Stickstoffatom und dessen benachbartem Kohlenstoffatom einen in 2-Position substituierten Pyrrolidinring.
In letzterem Fall ist für n der Wert 0 und für x der Wert 0 bevorzugt.

Das Schwefelatom in der Seitenkette kann als einfache Schwefelbrücke (Sulfid), als Sulfon oder Sulfoxid vorliegen. Die Sulfide sind bevorzugt.

Als Rest E kommen ein unsubstituierter oder ein ein- bis fünffach fluorierter Ethylrest infrage; der Perfluorrest ist für E bevorzugt.
Wenn der terminale Substituent $-(CH_2)_3-E$ in der Seitenkette durch einen gegebenenfalls substituierten Aryl- oder Heteroarylrest, der direkt oder über eine Mono-, Di- oder Trimethylengruppe an das Schwefelatom gebunden ist, ersetzt ist, so ist der Arylrest vorzugsweise ein Phenylrest; im Falle eines Heteroarylrestes ist dies vorzugsweise ein 2-Furyl- oder 2-Thienylrest. Als Substituent an diesem Aryl- oder Heteroarylrest kann beispielsweise eine Pentafluorethyl- oder Trifluormethylgruppe stehen, vorzugsweise eine Trifluormethylgruppe und diese wiederum bevorzugt in 4-Stellung eines Phenylrestes. Ein 2-Furyl- oder 2-Thienylrest ist vorzugsweise durch eine Methylengruppe vom Schwefelatom getrennt.

Beim Substituenten $R^3$ am 3-Sauerstoffatom handelt es sich in erster Linie um ein Wasserstoffatom. Die Hydroxygruppe kann aber auch mit einem gerad- oder verzweigtkettigen, gesättigten oder ungesättigten Kohlenwasserstoffrest mit bis zu 8 Kohlenstoffatomen, wie z.B. einer Methyl-, Ethyl-, Propyl-, Isopropyl-, Butyl-, Isobutyl-, tert. -Butyl-, Pentyl,- Isopentyl-, Neopentyl-, Heptyl-, Hexyl- oder Octylrest verethert oder mit einem Acylrest $R^{3'}—C(O)—$, worin $R^{3'}$ ein Wasserstoffatom oder ein Kohlenwasserstoffrest mit bis zu 8 Kohlenstoffatomen oder ein Phenylrest ist, verestert sein.

Beim Substituenten $R^{11}$ kann es sich um ein Wasserstoffatom, ein Halogenatom (F, Cl, Br, I) oder eine Nitrooxygruppe handeln; ein Fluoratom ist bevorzugt.

Steht $R^{11}$ für ein Wasserstoffatom oder stehen A und B nicht gemeinsam für $—(CH_2)_p—$ oder A und D nicht gemeinsam für $—(CH_2)_q—$, so weist der D-Ring eine Substitution aus der Gruppe 14,15-Doppelbindung, 14α,15α-Methylen, 15β-Methyl, 15,15-Dimethyl, 15β,16β-Methylen, 16α- oder 16β-Halogen und hiervon insbesondere 16α-Fluor, 16-Methyliden oder 14α,17α-Ethano auf. Eine 15β-Methylgruppe oder 16α-Fluoratom ist bevorzugt zu nennen.

$R^{17'}$ kann α- oder β-ständig sein.

Im Falle einer $C_{1-5}$-Alkylgruppe ist dies eine Methyl-, Ethyl-, Propyl-, Isopropyl-, Butyl-, Isobutyl-, tert-Butyl-, Pentyl-, Isopentyl-oder Neopentylgruppe. Als $C_{2-5}$-Alkenylgruppe ist beispielsweise der Vinyl- oder Allylrest zu nennen. Exemplarische Vertreter für eine $C_{2-5}$-Alkinylgruppe sind der Ethinyl- und 1-Propinylrest.
Ist $R^{17'}$ α-ständig, steht hierfür insbesondere ein Wasserstoffatom, eine Methyl- oder Trifluormethylgruppe oder eine gemeinsam mit $R^{14}$ gebildete Ethanobrücke.
Für $R^{17'}$ in der β-Position ist in erster Linie ein Wasserstoffatom und eine Methylgruppe zu nennen.

Insbesondere sind diejeniegen Verbindungen der allgemeinen Formel I bevorzugt, worin die Seitenkette SK entweder ein Rest der Teilformel $—(CH_2)_5—N(CH_3)—(CH_2)_3—SO_x— (CH_2)_3—C_2F_5$ mit x= 0, 1 oder 2 oder $—(CH_2)_5—N(A)—(CHB)—CH_2—S—CH_2)_3— C_2F_5$ mit $A + B = —(CH_2)_3—$ ist. In letzterem Fall weisen die Verbindungen vorzugsweise ein 17α-Wasserstoffatom auf.

Zur Bildung von Säureadditionssalzen sind anorganische und organische Säuren geeignet, wie sie dem Fachmann zur Bildung physiologisch verträglicher Salze bekannt sind. Als Additionssalze mit Säuren sind insbesondere die Hydrochloride und die Methansulfonate zu nennen.

Die nachstehenden Verbindungen sind erfindungsgemäß besonders bevorzugt:

14,17-Ethano-7α-{5-[N-Methyl-N-3-(4,4,5,5,5-pentafluorpentylthio)-propylamino]-pentyl}-estra-1,3,5(10)-trien-3,17β-diol

14,17-Ethano-7α-{5-[N-Methyl-N-3-(4,4,5,5,5-pentafluorpentansulfinyl)-propylamino]-pentyl}-estra-1,3,5(10)-trien-3,17β-diol
3,17β-Diacetoxy-14α,17α-ethano-7α-{5-[N-methyl-N-3-(4,4,5,5,5-pentafluorpentylthio)-propylamino]-pentyl}-estra-1,3,5(10)-trien
14,17-Ethano-7α-{5-[N-methyl-N-3-(4,4,5,5,5-pentafluorpentansulfonyl)-propylamino]-pentyl}-estra-1,3,5(10)-trien-3,17β-diol
17α-Trifluormethyl-7α-{5-[N-methyl-N-3-(4,4,5,5,5-pentafluorpentylthio)-propylamino]-pentyl}-estra-1,3,5(10)-trien-3,17β-diol
15β,16β-Methano-17α-methyl-7α-{5-[N-methyl-N-3-(4,4,5,5,5-pentafluor-pentylthio)-propylamino]-pentyl}-estra-1,3,5(10)-trien-3,17β-diol
15β,16β-Methano-17α-methyl-7α-{5-[N-methyl-N-3-(4,4,5,5,5-pentafluorpentansulfinyl)-propylamino]-pentyl}-estra-1,3,5(10)-trien-3,17β-diol
15β,16β-Methano-7α-{5-[N-methyl-N-3-(4,4,5,5,5-pentafluor-pentylthio)-propylamino]-pentyl}-estra-1,3,5(10)-trien-3,17β-diol
15β,16β-Methano-7α-{5-[N-methyl-N-3-(4,4,5,5,5-pentafluor-pentansulfinyl)-propylamino]-pentyl}-estra-1,3,5(10)-trien-3,17β-diol
15β-Methyl-7α-{5-[N-methyl-N-3-(4,4,5,5,5-pentafluor-pentylthio)-propylamino]-pentyl}-estra-1,3,5(10)-trien-3,17β-diol
15β,17α-Dimethyl-7α-{5-[N-methyl-N-3-(4,4,5,5,5-pentafluor-pentylthio)-propylamino]-pentyl}-estra-1,3,5(10)-trien-3,17β-diol
11β-Fluor-7α-{5-[N-methyl-N-3-(4,4,5,5,5-pentafluorpentylthio)-propylamino]-pentyl}-estra-1,3,5(10)-trien-3,17β-diol
11β-Fluor-7α-{5-[N-methyl-N-3-(4,4,5,5,5-pentafluorpentansulfinyl)-propylamino]-pentyl}-estra-1,3,5(10)-trien-3,17β-diol
11β-Fluor-7α-{5-[N-methyl-N-3-(4,4,5,5,5-pentafluorpentansulfonyl)-propylamino]-pentyl}-estra-1,3,5(10)-trien-3,17β-diol
16α-Fluor-17α-methyl-7α-{5-[N-methyl-N-3-(4,4,5,5,5-pentafluorpentylthio)-propylamino]-pentyl} -estra-1,3,5(10)-trien-3,17β-diol
16α-Fluor-17β-methyl-7α- {5-[N-methyl-N-3-(4,4,5,5,5-pentafluorpentylthio)-propylamino]-pentyl}-estra-1,3,5(10)-trien-3,17α-diol
16α-Fluor-17α-methyl-7α-{5-[N-methyl-N-3-(4,4,5,5,5-pentafluorpentansulfinyl)-propylamino]-pentyl}-estra-1,3,5(10)-trien-3,17β-diol
16α-Fluor-17α-methyl-7α-{5-[N-methyl-N-3-(4,4,5,5,5-pentafluorpentansulfonyl)-propylamino]-pentyl}-estra-1,3,5(10)-trien-3,17β-diol
16α-Fluor-7α-{5-[N-methyl-N-3-(4,4,5,5,5-pentafluorpentylthio)-propylamino]-pentyl}-estra-1,3,5(10)-trien-3,17β-diol
16α-Fluor-7α-{5-[N-methyl-N-3-(4,4,5,5,5-pentafluorpentylthio)-propylamino]-pentyl}-estra-1,3,5(10)-trien-3,17α-diol
16α-Fluor-7α-{5-[N-methyl-N-3-(4,4,5,5,5-pentafluorpentansulfinyl)-propylamino]-pentyl}-estra-1,3,5(10)-trien-3,17β-diol
16α-Fluor-7α-{5-[N-methyl-N-3-(4,4,5,5,5-pentafluorpentansulfonyl)-propylamino]-pentyl}-estra-1,3,5(10)-trien-3,17β-diol
7α-{5-[N-Methyl-N-3-(4,4,5,5,5-pentafluorpentylthio)-propylamino]-pentyl}-estra-1,3,5(10),14-tetraen-3,17β-diol
7α-{5-[N-Methyl-N-3-(4,4,5,5,5-pentafluorpentansulfinyl)-propylamino]-pentyl}-estra-1,3,5(10),14-tetraen-3,17β-diol
7α-{5-[N-Methyl-N-3-(4,4,5,5,5-pentafluorpentansulfonyl)-propylamino]-pentyl}-estra-1,3,5(10),14-tetraen-3,17β-diol
7α-{5-[(2S)-2-(4,4,5,5,5-Pentafluorpentylthiomethyl)-pyrrolidin-1-yl]-pentyl}-estra-1,3,5(10)-trien-3,17β-diol
7α-{5-[(2R)-2-(4,4,5,5,5-Pentafluorpentylthiomethyl)-pyrrolidin-1-yl]-pentyl}-estra-1,3,5(10)-trien-3,17β-diol
17α-Methyl-7α-{5-[2-(4,4,5,5,5-pentafluorpentylthiomethyl)-pyrrolidin-1-yl]-pentyl}-estra-1,3,5(10)-trien-3,17β-diol
11β-Fluor-7α-{5-[2-(4,4,5,5,5-pentafluorpentylthiomethyl)-pyrrolidin-1-yl]-pentyl}-estra-1,3,5(10)-trien-3,17β-diol
11β-Fluor-17α-methyl-7α-{5-[(2S)-2-(4,4,5,5,5-pentafluorpentylthiomethyl)-pyrrolidin-1-yl]-pentyl}-estra-1,3,5(10)-trien-3,17β-diol
11β-Fluor-17α-methyl-7α-{5-[(2S)-2-(4,4,5,5,5-pentafluorpentansulfinylmethyl)-pyrrolidin-1-yl]-pentyl}-estra-1,3,5(10)-trien-3,17β-diol
11β-Fluor-17α-methyl-7α-{5-[(2S)-2-(4,4,5,5,5-pentafluorpentansulfonylmethyl)-pyrrolidin-1-yl]-pentyl}-estra-1,3,5(10)-trien-3,17β-diol

7α-{5-[(2S)-2-(4,4,5,5,5-Pentafluorpentansulfinylmethyl)-pyrrolidin-1-yl]-pentyl-estra-1,3,5(10)-trien-3,17β-diol
7α-{5-[(2S)-2-(4,4,5,5,5-Pentafluorpentansulfonylmethyl)-pyrrolidin-1-yl]-pentyl-estra-1,3,5(10)-trien-3,17β-diol
11β-Fluor-17α-methyl-7α-{5-[N-methyl-N-3-(4,4,5,5,5-pentafluorpentylthio)-propylamino]-pentyl}-estra-1,3,5(10)-trien-3,17β-diol
11β-Fluor-17α-methyl-7α-{5-[N-methyl-N-3-(4,4,5,5,5-pentafluorpentansulfinyl)-propylamino]-pentyl}-estra-1,3,5(10)-trien-3,17β-diol
11β-Fluor-17α-methyl-7α-{5-[N-methyl-N-3-(4,4,5,5,5-pentafluorpentansulfonyl)-propylamino]-pentyl}-estra-1,3,5(10)-trien-3,17β-diol
11β-Fluor-7α-{5-[N-methyl-N-2-(4,4,5,5,5-pentafluorpentansulfonyl)-ethylamino]-pentyl}-estra-1,3,5(10)-trien-3,17β-diol
11β-Fluor-7α-{5-[N-methyl-N-3-(4,4,5,5,5-pentafluorpentylthio)-propylamino]-pentyl}-3-hydroxy-estra-1,3,5(10)-trien-17-on
11β-Fluor-7α-{6-[N-methyl-N-3-(4,4,5,5,5-pentafluorpentylthio)-propylamino]-hexyl}-estra-1,3,5(10)-trien-3,17β-diol
11β-Fluor-7α-{6-[N-methyl-N-3-(4,4,5,5,5-pentafluorpentansulfinyl)-propylamino]-hexyl}-estra-1,3,5(10)-trien-3,17β-diol
11β-Fluor-7α-(5-{[N-3-(furan-2-ylmethylthio)-propyl]-N-methyl-amino}-pentyl)-estra-1,3,5(10)-trien-3,17β-diol
11β-Fluor-7α-(5-{N-methyl-[N-3-(thiophen-2-ylmethylthio)-propyl]-amino}-pentyl)-estra-1,3,5(10)-trien-3,17β-diol
11β-Fluor-7α-{5-[(2S)-2-(4-trifluormethyphenylthiomethyl)-pyrrolidin-1-yl]-pentyl}-estra-1,3,5(10)-trien-3,17β-diol
11β-Fluor-17α-methyl-7α-{5-[(2S)-2-(4,4,5,5,5-pentafluorpentansulfinylmethyl)-pyrrolidin-1-yl]-pentyl}-estra-1,3,5(10)-trien-3,17β-diol
11β-Fluor-17α-methyl-7α-{5-[(2R)-2-(4,4,5,5,5-pentafluorpentylthiomethyl)-pyrrolidin-1-yl]-pentyl}-estra-1,3,5(10)-trien-3,17β-diol
11β-Fluor-7α-{5-[(2S)-2-(4,4,5,5,5-pentafluorpentylthiomethyl)-pyrrolidin-1-yl]-pentyl}-estra-1,3,5(10)-trien-3,17β-diol
11β-Fluor-7α-{5-[(2S)-2-(4,4,5,5,5-pentafluorpentansulfinylmethyl)-pyrrolidin-1-yl]-pentyl}-estra-1,3,5(10)-trien-3,17β-diol
11β-Fluor-7α-{5-[(2S)-2-(4,4,5,5,5-pentafluorpentansulfonylmethyl)-pyrrolidin-1-yl]-pentyl}-estra-1,3,5(10)-trien-3,17β-diol

Die Verbindungen der allgemeinen Formel I stellen Verbindungen mit sehr starker antiestrogener Wirksamkeit dar.
Bei den erfindungsgemäßen Verbindungen handelt es sich zum einen Teil um reine Antiestrogene oder zum anderen Teil um sogenannte Partialantagonisten, d. h. um Antiestrogene mit estrogener Partialwirkung wie das Tamoxifen oder das Raloxifen. Die agonistische, estrogene Wirkung ist aber in den erfindungsgemäßen Verbindungen in jedem Fall deutlich schwächer ausgeprägt als beim Tamoxifen. Im Gegensatz zum Tamoxifen tritt bei den Partialantagonisten der allgemeinen Formel I deren agonistische, estrogene Wirkung gewebeselektiv auf. Insbesondere tritt die agonistische Wirkung am Knochen, im Herz-Kreislaufsystem und im ZNS (Zentrales Nervensystem) auf. Keine agonistische Wirkung tritt insbesondere am Uterus auf.

Verbindungen mit antiestrogenen Eigenschaften, d.h. Stoffe mit Hemmwirkungen gegenüber Estrogenen, sind bereits zahlreich beschrieben worden.
Als den vorliegenden Verbindungen der allgemeinen Formel I strukturell am nächsten kommende Verbindungen sind zum einen die aus der EP-A 0 138 504 hervorgehenden Steroid-Derivate anzusehen, und von diesen insbesondere das 7α-[9-(4,4,5,5,5-Pentafluorpentylsulfinyl)-n-nonyl]-estra-1,3,5(10)-trien-3,17β-diol (EP-A 0 138 504, Seite 58, vorletzte Verbindung). Diese Verbindung befindet sich gegenwärtig in klinischer Entwicklung für hormonabhängige Tumoren (Brustkrebs) und stellt die derzeit beste bekannte Verbindung, d.h. diejeniege mit der stärksten antiestrogenen Wirksamkeit, dieser Steroid-Derivate dar.

Pharmazeutische Zusammensetzungen, die Sexualsteroid-Inhibitoren enthalten, welche ein steroidales Grundgerüst, das eine 7α-Seitenkette bei gleichzeitiger Anwesenheit mindestens eines weiteren Substituenten in Position 14, 15 oder 16 aufweist, sind Gegenstand der EP-A 0 376 576 und sind ebenfalls als nächstliegender Stand der Technik in Betracht zu ziehen.

Eine Vielzahl verschiedenartigster Verbindungen - u. a. solche steroidalen Ursprungs als auch solche mit 2-Phenylindol-Grundgerüst - die als Antiestrogen wirken und/oder die Estrogenbiosynthese unterdrücken, werden in der WO 93/10741 offenbart.

Weitere steroidale Antiestrogene sind in den EP-AS 0 384 842 und 0 629 635 beschrieben, die einen 11β-Phenylrest tragen.

Bei den erfindungsgemäßen Verbindungen handelt es sich um Antiestrogene mit stärkerer antiestrogener Wirkung als das schon erwähnte 7α-[9-(4,4,5,5,5-Pentafluorpentylsulfinyl)-n-nonyl]-estra-1,3,5(10)-trien-3,17β-diol.

Die Verbindungen der allgemeinen Formel I gemäß vorliegender Anmeldung zeichnen sich im Vergleich zu den bereits bekannten Steroid-Derivaten gemäß der EP-A 0 138 504 und EP-A 0 367 576 durch neuartige Seitenketten am Kohlenstoffatom 7 des Steroid-Gerüsts aus. Diese Strukturmodifikation führt zu besonders hoch antiestrogen wirksamen Verbindungen, wie in Transaktivierungstests nachgewiesen wurde.
Gegenüber den Verbindungen der EP-A 0 138 504 unterscheiden sich die Verbindungen der allgemeinen Formel I außerdem hinsichtlich der Substitution am Kohlenstoffatom 11 und/oder des D-Ringes (außer im Fall, daß A und B gemeinsam für $-(CH_2)_p-$ oder A und D gemeinsam für $-(CH_2)_q-$ stehen). Im Vergleich zu den Verbindungen der EP-A 0 367 576 können die Verbindungen der allgemeinen Formel I am Kohlenstoffatom 11 und/oder im D-Ring dieselben oder auch andere Substituenten tragen.
Durch den Disclaimer in der Definition der allgemeinen Formel I werden aus deren Umfang in der nicht-vorveröffentlichten DE P 196 22 457 beschriebene Verbindungen ausgenommen.

Die antiestrogene Wirkung der erfindungsgemäßen Verbindungen wurde in Transaktivierungsassays bestimmt [Demirpence E., Duchesne M.-J., Badia E., Gagne D. und Pons M.: MVLN Cells: A Bioluminescent MCF-7-Derived Cell Line to study the Modulation of Estrogenic Activity; J. Steroid. Molec. Biol. Vol. 46, No. 3, 355 - 364 (1993) sowie Berry M., Metzger D. Chambon P.: Role of the two activating domains of the estrogen receptor in the cell-type and promoter-context dependent agonistic activity of the anti-estrogen 4-hydroxytamoxifen; The EMBO Journal Vol. 9, 2811 - 2818 (1990)].

Die Hela-Zellen sind transient mit humanem Estrogenrezeptor-Expressionsvektor (HEGO) und Vit-TK-CAT Reportergen und die MVLN-Zellen stabil mit dem Reportergen Vit-TK-LUC transfiziert. Es wurde die estrogene Wirkstärke in Gegenwart von 0,1 nM Estradiol bestimmt.

Die $IC_{50}$-Werte fiir die neuen Verbindungen liegen im nanomolaren Bereich. In der Helasowie der MVLN-Zellinie ergeben sich fiir die Verbindungen der Beispiele 12, 15, 18, 25, 29, 31 und 36 sowie fiir 7α-[9-(4,4,5,5,5-pentafluorpentylsulfinyl)-n-nonyl]-estra-1,3,5(10)-trien-3,17β-diol folgende $IC_{50}$-Werte (Testdurchführung gemäß der oben angegebenen Literaturstellen):

| Verbindung | $IC_{50}$ [nM] | |
|---|---|---|
| | Hela-Zellen | MVLN-Zellen |
| Beispiel 12 | 0,06 | 2,4 |
| Beispiel 15 | 0,06 | 1,4 |
| Beispiel 18 | 0,05 | 0,13 |
| Beispiel 25 | 0,06 | 0,15 |
| Beispiel 29 | 0,13 | 0,2 |
| Beispiel 31 | 0,1 | 0,2 |
| Beispiel 36 | 0,05 | 0,4 |
| Referenz: | | |
| 7α-[9-(4,4,5,5,5-Pentafluorpentansulfinyl)-nonyl]-estra-1,3,5(10)-trien-3,17β-diol | 0,5 | 6,0 |

In-vivo Tests belegen ebenfalls die Überlegenheit der erfindungsgemäßen Verbindungen gegenüber dem 7α-[9-(4,4,5,5,5-Pentafluorpentansulfinyl)-nonyl]-estra-1,3,5(10)-trien-3,17β-diol. Die nachfolgend beschriebenen Tests wurden durchgeführt:

1. Uteruswachstumstest bei der infantilen Ratte, p.o. (Test auf antiestrogene Wirkung)

2. Tumortests: Antitumorwirkung auf das hormonabhängige Mammakarzinom DMBA*-induziertes Mammakarzinom bei der Ratte

**1. Uteruswachstumstest bei der infantilen Ratte (antiestrogene Wirkung)**

Prinzip der Methode

Bei Nagern reagiert der Uterus auf die Applikation von estrogenen mit einer Gewichtszunahme (sowohl Proliferation als auch Wassereinlagerung). Dieses Wachstum ist durch gleichzeitige Gäbe antiestrogen-wirkender Verbindungen dosisabhängig zu hemmen.

**Versuchsdurchführung**

Tiere:

Infantile weibliche Ratten im Gewicht von 35-45 g bei Versuchsbeginn, pro Dosis 5-6 Tiere.

Formulierung und Applikation der Substanzen:

Für die p.o. Applikation werden die Substanzen in 1 Teil Ethanol (E) gelöst und mit 9 Teilen Erdnußöl (EÖ) aufgefüllt.

Versuchsansatz

Die gerade von den Müttern abgesetzten jungen Ratten werden zur Eingewöhnung einen Tag vor Behandlungsbeginn geliefert und sofort mit Futter - auch in dem Tierkäfig - versorgt. Die Behandlung erfolgt dann täglich einmal über 3 Tage in Kombination mit 0,5 µg Estradiolbenzoat (EB). EB wird immer subcutan (s.c.) appliziert, während die Testsubstanz p.o. (peroral) verabreicht wird. 24 Stunden nach der letzten Applikation werden die Tiere gewogen, getötet und die Uteri entnommen. Von den präparierten Uteri werden die Feuchtgewichte (ohne Inhalt) ermittelt.

Kontrollen

negative Kontrolle: Vehikel (E/EÖ), 0,2 ml/Tier/Tag
positive Kontrolle: 0,5 µg EB/0,1 ml/Tier/Tag

Auswertung

Von den relativen Organgewichten (mg/100 g Körpergewicht) werden für jede Gruppe die Mittelwerte mit Standardabweichung (X+SD), sowie die Signifikanz der Unterschiede zur Kontrollgruppe (EB) im Dunnett-Test ($p<0.05$) ermittelt. Die Berechnung der Hemmung (in %) gegenüber der EB-Kontrolle erfolgt mit einem Programm. Die relativen Wirksamkeiten der Prüfsubstanzen werden durch eine Kovarianz- und Regressionsanalyse ermittelt.

**Antiuterotrophe Wirkung an der Ratte**

| Verbindung aus Beispiel | 0,1 mg/kg p.o. % Hemmung | IC50 |
|---|---|---|
| 12 | 91 | 0.01 |
| 15 | 33 | 0.24 |
| 18 | 16 | > 0.30 |
| 29 | 80 | 0.01 |
| 31 | 71 | 0.04 |
| 36 | 62 | 0.03 |
| Referenz ZM 182 780 | 8 | 0.39 |

Diese Ergebnisse demonstrieren die viel höhere antiestrogene Wirksamkeit der Verbindungen der allgemeinen Formel I im Vergleich zur Verbindung 7α-[9-(4,4,5,5,5-Pentafluorpentylsulfinyl)-n-nonyl]-estra-1,3,5(10)-trien-3,17β-diol jeweils nach oraler Applikation.
Die erfindungsgemäßen Verbindungen zeichnen sich durch eine bessere Bioverfügbarkeit nach oraler Applikation aus.

* = Dimethylbenzanthrazen

## 2. Tumortest

**Beeinflussung des Tumorwachstums im DMBA*-Modell bei der Ratte (DMBA-Tumormodell)**

Biologische Grundlage

Das Wachstum des DMBA-induzierten Mammatumors der Ratte ist weitgehend von Estrogenen und Prolaktin abhängig. Wirksame Antiestrogene, Antigestagene und Aromatasehemmer führen zur Hemmung des Tumorwachsturns. Substanzen, die antigonadotrope und androgene Eigenschaften besitzen, entfalten ebenfalls tumorhemmende Wirkung.

Tiermaterial

45 - 47 Tage alte weibliche Ratten (Sprague-Dawley, Züchter ZIH oder Möllegard), pro Gruppe 8 - 10 Tiere.

Versuchsansatz

Die Tiere erhalten einmalig 10 mg DMBA oral. Anschließend werden die Tiere einmal wöchentlich palpatorisch auf Tumorentwicklung untersucht. 6 bis 10 Wochen nach der DMBA-Behandlung entwickeln sich etwa 1 bis 10 Tumoren pro Tier. Die Tumorgröße wird einmal wöchentlich mit Hilfe einer Schiebelehre bestimmt. Hat mindestens ein Tumor eine definierte Größe (150 $mm^2$ Tumorfläche) erreicht, erfolgt die Ovariektomie der Tiere bzw. beginnt die Behandlung der Tiere mit der Testsubstanz. Die Behandlung erfolgt zumeist täglich über ca. 28 Tage (Details Abwicklung siehe Versuchsplan). Die Tumorgröße wird weiterhin 1 x /Woche ermittelt.

Auswertung

Die Gesamttumorgröße pro Tier wird vor Beginn der Behandlung ermittelt (Vorwerte). Für jede Gruppe wird dann der Mittelwert der prozentualen Veränderungen der Tumorgröße, bezogen auf die initialen Werte, errechnet. Außerdem wird der Prozentsatz an Tieren pro Gruppe ermittelt, deren Tumoren jeweils (1) total zurückgegangen (totale Regression), teilweise zurückgegangen (partiale Regresssion), (3) unverändert (keine Veränderung) bzw. (4) weiter vergrößert (Vergrößerung) sind.

Die ermittelten Werte werden im Dunnett-Test auf Signifikanz getestet und grafisch dargestellt.

**Testergebnis**

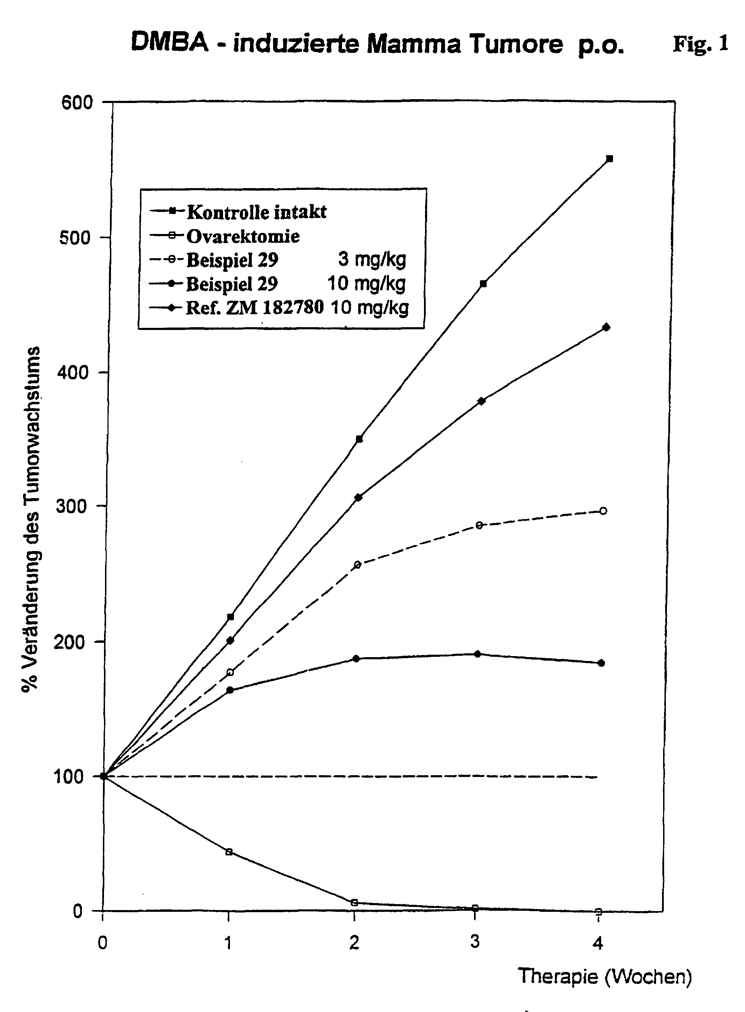

Bei einer oralen Dosis von 3 mg/kg/Tag hemmt 11β-Fluor-7α-{5-[2-(4,4,5,5,5-pentafluorpentylthiomethyl)-pyrrolidin-1-yl]-pentyl}-estra-1,3,5(10)-trien-3,17β-diol (Verbindung des Beispiels 29) das Tumorwachstum stärker als eine orale Dosis von 10 mg/kg/Tag 7α-[9-(4,4,5,5,5-Pentafluorpentansulfinyl)-nonyl]-estra-1,3,5(10)-trien-3,17β-diol, welches bei dieser Dosis nur eine geringfügige Wirkung im Vergleich zur intakten Kontrolle ausübt. Ovarktomie führt zu einer totalen Remission der Tumoren (Figur 1).

Die Verbindungen wirken hemmend auf das Wachstum von hormonabhängigen Tumorzellen, insbesondere hemmen sie das Wachstum von estrogen-abhängigen menschlichen Mammatumorzellen (MCF-7).

Die antiproliferative Aktivität der neuen Verbindungen in Mammakarzinom-Zellinien ist höher als die des 7α-[9-(4,4,5,5,5-Pentafluorpentylsulfinyl)-n-nonyl]-estra-1,3,5(10)-trien-3,17β-diols.

Als reine Antiestrogene im Sinne vorliegender Erfindung sind solche Verbindungen der allgemeinen Formel I anzusehen, die im nachstehenden in-vitro Test auf estrogene Wirkung keine oder nur geringfügige agonistische Wirkung zeigen (bis etwa 10 % der Wirkung von Estradiol).

Die estrogene Partialwirkung wurde dabei ebenfalls durch Transaktivierungsassays bestimmt. HeLa Zellen wurden mit humanem Estrogenrezeptor-Expressionsvektor (HEGO) und einem Reportergen rPR-TK-CAT transfiziert. Dieses Reportergen enthält das "Estrogen Responsive Element" des Kaninchen Progesteronrezeptorgens (+698/+729-Region) vor einem TK-CAT Gen (Savouret J.F., Bailly A., Misrahi M., Rauch C., Redeuilh G., Chauchereau A., Milgrom E., Characterization of the hormone responsive element involved in the regulation of the progesterone receptor gene. EMBO J. 10, 1875 - 1883 (1991).

Es wurde die estrogene Wirkstärke bei einer Konzentration von 1µM bestimmt.

*9,10-Dimethyl-1,2-benzanthracen

| Verbindung aus Beispiel | Aktivierung des rPR-TK-Promotors [% Estradiol]* |
|---|---|
| 12 | - 11 |
| 15 | - 25 |
| 18 | - 24 |
| 25 | - 21 |
| 29 | 10 |
| 31 | - 5 |
| 36 | - 6 |
| Referenz ZM 182780 | - 15 |

*Ein negativer Wert bedeutet Suppression der Reportergen-Aktivität unterhalb die Werte der Kontrollen

Die erfindungsgemäßen Verbindungen, insbesondere wenn es reine Antiestrogene sind, eignen sich zur Therapie von estrogen-abhängigen Erkrankungen, zum Beispiel Mammacarcinom (second-line Therapie des Tamoxifen-resistenten Mammacarcinoms; zur adjuvanten Behandlung des Mammacarcinoms anstelle von Tamoxifen), Endometriumcarcinom, Prostatahyperplasie, anovulatorische Infertilität und Melanom.
Die reinen Antiestrogene der allgemeinen Formel I können außerdem als Komponente in den in der EP 346 014 B1 beschriebenen Produkten verwendet werden, die ein Estrogen und ein reines Antiestrogen enthalten, und zwar zur gleichzeitigen, sequentiellen oder getrennten Verwendung für die selektive Estrogentherapie peri- oder postmenopausaler Frauen.
Die Verbindungen der allgemeinen Formel I, insbesondere wenn es sich um reine Antiestrogene handelt, können gemeinsam mit Antigestagenen (kompetitiven Progesteronantagonisten) zur Behandlung hormonabhängiger Tumoren verwendet werden (EP 310 542 A).
Weitere Indikationen, in denen die Verbindungen der allgemeinen Formel zum Einsatz kommen können, ist der männliche Haarausfall, eine diffuse Alopecie, eine durch eine Chemotherapie hervorgerufene Alopecie sowie Hirsutismus (Hye-Sun Oh und Robert C. Smart, Proc. Natl. Acad. Sci. USA, 93 (1996) 12525 - 12530).
Außerdem können die Verbindungen der allgemeinen Formel I zur Herstellung von Medikamenten zur Behandlung der Endometriose und von Endometrialkarzinomen verwendet werden.
Femer kann man die Verbindungen der allgemeinen Formel I zur Herstellung pharmazeutischer Zusammensetzungen für die männliche und weibliche Fertilitätskontrolle einsetzen (männliche Fertilitätskontrolle: DE-A 195 10 862.0).

Diejenigen Verbindungen der allgemeinen Formel I mit gewebeselektiver estrogener Partialwirkung können in erster Linie zur Prophylaxe und Therapie der Osteoporose und zur Herstellung von Präparaten für die Substitutionstherapie in der Prae-, Peri- und Postmenopause (HRT) Verwendung finden (Black, L.J., Sato,M.,Rowley, E.R., Magee, D.E., Bekele, A., Williams, D.C., Cullinan, G.J., Bendele, R., Kauffinan, R.F., Bensch, W.R., Frolik, C.A., Termine, J.D. and Bryant, H.U.: Raloxifene [LY 139481 HCl] prevents bone loss and reduces serum cholesterol without causing uterine hypertrophy in ovariectornized rats; J. Clin. Invest. 93: 63 - 69, 1994). Die estrogene Partialwirkung tritt ausschließlich am gewünschten Zielorgan auf.

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere zur Behandlung von estrogenabhängigen Krankheiten und Tumoren und von Arzneimitteln für die Hormonsubstitutions-Therapie (HRT).

Die erfindungsgemäßen Verbindungen und die Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen oder deren Säureadditionssalze, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 u. ff.; H.v. Czetsch-Lindenwald, Hilfsstoffe für Pharmazie und angrenzende Gebiete; Pharm. Ind. Heft 2, 1961, Seite 72 u. ff.; Dr. H.P. Fiedler, Lexikon der Hilfsstoffe für Pharmazie, Kosmetik und angrenzende Gebiete Cantor KG. Aulendorf in Württemberg 1971.

Die Verbindungen können oral oder parenteral, beispielsweise intraperitoneal, intramuskulär, subkutan oder perkutan, verabreicht werden. Die Verbindungen können auch in das Gewebe implantiert werden. Die zu verabreichende Menge der Verbindungen schwankt innerhalb eines weiten Bereichs und kann jede wirksame Menge abdecken. In Abhängigkeit des zu behandelnden Zustandes und der Art der Verabreichung kann die Menge der verabreichten Verbindung 0,1-25 mg/kg Körpergewicht, vorzugsweise 0,5-5 mg/kg Körpergewicht, je Tag betragen. Beim Menschen entspricht dies einer täglichen Dosis von 5 bis 1250 mg.
Die bevorzugte tägliche Dosierung beim Menschen ist 50 bis 200 mg. Dies gilt insbesondere für die Tumortherapie.

Zur oralen Verabreichung kommen Kapseln, Pillen, Tabletten, Dragèes usw. infrage. die Dosierungseinheiten können neben dem Wirkstoff einen pharmazeutisch verträglichen Träger, wie zum Beispiel Stärke, Zucker, Sorbit, Gelantine, Gleitmittel, Kieselsäure, Talkum usw., enthalten. Die einzelnen Dosierungseinheiten für die orale Applikation können beispielsweise 5 bis 500 mg des Wirkstoffs enthalten.

Um eine bessere Bioverfügbarkeit des Wirkstoffes zu erreichen, können die Verbindungen auch als Cyclodextrinclathrate formuliert werden. Hierzu werden die Verbindungen mit α-, β-oder γ-Cyclodextrin oder Derivaten von diesen umgesetzt (PCT/EP95/02656).
Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvennittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Die erfindungsgemäßen Verbindungen können wie nachstehend beschrieben hergestellt werden. Die nachfolgenden Beispiele dienen der näheren Erläuterung der Erfindung. Durch analoge Vorgehensweise unter Verwendung analoger Reagenzien zu den in den Beispielen enthaltenen Angaben lassen sich alle Verbindungen der allgemeinen Formel I erhalten.
Die Verseifung von Estergruppierungen sowieVeresterung und Veretherung freier Hydroxygruppen erfolgt jeweils nach etablierten Verfahren der organischen Chemie.
Durch Beachtung der unterschiedlichen Reaktivität der veresterten und freien 3- und 17-Hydroxygruppe lassen sich die 3,17-Diester selektiv in 3-Position spalten und die 3-Hydroxy-17-acyloxy-Verbindung läßt sich dann gezielt in 3-Position weiter funktionalisieren; genauso gut ist es möglich, die 3,17-Dihydroxyverbindung selektiv nur in 3-Position zu verestern oder zu verethern und dann gezielt in 17-Position einen anderen als bereits in 3-Stellung sich befindenden Rest einzuführen.
Die Säureadditionssalze der Verbindungen der allgemeinen Formel I lassen sich ebenfalls nach gängigen Verfahren aus den Verbindungen der allgemeinen Formel I herstellen.

Die nachfolgenden Beispiele dienen der näheren Erläuterung der Erfindung:

Beispiel 1

**14,17-Ethano-7α-{5-[N-Methyl-N-3-(4,4,5,5,5-pentafluorpentylthio)-propylamino]-pentyl}-estra-1,3,5(10)-trien-3,17β-diol**

**a) 7α-(5-*tert*. Butyldimethylsilyloxypentyl)-estr-4-en-3,17-dion**

In 70 ml abs. Tetrahydrofuran werden 15,1 g Mg-Späne mit 175,6 g 1-Brom-5-*tert*-butyldimethylsilyloxypentan [Tetrahedron Letters 23, **1982,** 40, 4147-4150] gelöst in 600 ml abs. Tetrahydrofuran zum Grignardreagenz umgesetzt. In diese auf -20°C gekühlte Lösung werden unter einem Stickstoffstrom 59 g Kupfer-(I)-iodid gegeben und anschließend innerhalb einer Stunde 50 g Estra-4,6-dien-3,17-dion [Steroids Vol. 1, **1963**, 233-249] gelöst in 300 ml abs. THF zugetropft. Zur Aufarbeitung werden 37,5 ml Essigsäure zugetropft, die Reaktionsmischung mit Essigester verdünnt, mit gesättigter Ammoniumchloridlösung, Wasser und Natriumhydrogencarbonatlösung gewaschen und getrocknet. Der nach dem Eindampfen erhaltene Rückstand wird an Kieselgel chromatographiert. Man erhält 35,4 g 7α-(5-tert-Butyldimethylsilyloxypentyl)-estr-4-en-3,17-dion. $[\alpha]_D^{22}$ = +52.8° (c = 0.535% in Chloroform)

**b) 7α-(5-Hydroxypentyl)-estr-4-en-3,17-dion**

Eine Lösung von 125,4 g 7α-(5-*tert*-Butyldimethylsilyloxypentyl)-estr-4-en-3,17-dion in 625 ml Methanol und 347 ml Wasser wird mit 694 ml Eisessig 2,5 Stunden bei 50 °C gerührt. Nach dem Eindampfen bei 60 °C i. Vak. werden

94,1 g rohes 7α-(5-Hydroxypentyl)-estr-4-en-3,17-dion als Öl erhalten.

**c) 7α-(5-Acetoxypentyl)-estr-4-en-3,17-dion**

Eine Lösung von 94 g rohem 7α-(5-Hydroxypentyl)-estr-4-en-3,17-dion in 620 ml Pyridin wird mit 310 ml Acetanhydrid langsam versetzt und 2 h bei 25 °C gerührt. Dann wird unter Eiskühlung mit 116 ml Wasser langsam versetzt, mit 3 1 Diethylether verdünnt, die organ. Phase nach dem Waschen mit Natruimhydrogencarbonatlösung, getrocknet und eingedampft. Der Rückstand wird an Kieselgel chromatographiert und es werden 84,4 g 7α-(5-Acetoxypentyl)-estr-4-en-3,17-dion als Öl erhalten.

**d) 7α-(5-Acetoxypentyl)-3-hydroxy-estra-1,3,5(10)-trien-17-on**

Zu einer Lösung von 82,3 g 7α-(5-Acetoxypentyl)-estr-4-en-3,17-dion in 936 ml Acetonitril werden bei 80 °C Badtemperatur 17,8 g Lithiumbromid und 92,83 g Kupfer-II-bromid gegeben. Nach 10 Minuten bei 80 °C Badtemperatur wird die Reaktionslösung abgekühlt, dreimal mit Essigester extrahiert, mit Wasser und Natriumhydrogencarbonatlösung gewaschen und getrocknet. Der nach dem Eindampfen erhaltene Rückstand wird an Kieselgel chromatographiert und es werden 60,4 g 7α-(5-Acetoxypentyl)-3-hydroxy-estra-1,3,5(10)-trien-17-on als Öl erhalten.

**e) 3-Acetoxy-7α-(5-acetoxypentyl)-estra-1,3,5(10)-trien-17-on**

Eine Lösung von 60,4 g 7α-(5-Acetoxypentyl)-3-hydroxy-estra-1,3,5(10)-trien-17-on in 300 ml Pyridin wird mit 150 ml Acetanhydrid 1 Stunde bei Raumtemperatur gerührt. Dann wird mit einer Eis / Wasser / Kochsalz / Salzsäure-Mischung gefällt, mit Essigester aufgenommen, mit Natriumhydrogencarbonat- sowie Kochsalzlösung neutralgewaschen und über Natriumsulfat getrocknet und i.Vak. eingeengt. Man erhält 63,9 g 3-Acetoxy-7α-(5-acetoxypentyl)-estra-1,3,5(10)-trien-17-on als Öl.

**f) 3-Acetoxy-7α-(5-acetoxypentyl)-17,17-ethylendioxy-estra-1,3,5(10)-trien**

Eine Lösung von 63,9 g 3-Acetoxy-7α-(5-acetoxypentyl)-estra-1,3,5(10)-trien-17-on in 460 ml Dichlormethan wird mit 460 ml Ethylenglycol, 155 ml Trimethylorthoformiat und 1,2 g *para*-Toluolsulfonsäure 3 Stunden bei 50 °C Badtemperatur gerührt. Dann wird mit Dichlormethan verdünnt, mit Natriumhydrogencarbonat- sowie Kochsalzlösung gewaschen und über Natriumsulfat getrocknet, i.Vak. eingeengt und an Kieselgel mit Hexan / Essigester chromatographiert. Man erhält 63,2 g 3-Acetoxy-7α-(5-acetoxypentyl)-17,17-ethylendioxy-estra-1,3,5(10)-trien als Öl.

**g) 3-Acetoxy-7α-(5-acetoxypentyl)-16α-brom-17,17-ethylendioxy-estra-1,3,5(10)-trien**

Eine Lösung von 63,2 g 3-Acetoxy-7α-(5-acetoxypentyl)-17,17-ethylendioxy-estra-1,3,5(10)-trien in 630 ml Tetrahydrofuran wird bei 0 °C mit 61,7 g Pyridinhydrobromidperbromid portionsweise versetzt und 2 Stunden bei 0 °C gerührt. Dann wird eine Lösung von 15 g Natriumsulfid in 70 ml Wasser zugesetzt, mit Essigester verdünnt, mit Natriumhydrogencarbonat- sowie Kochsalzlösung gewaschen und über Natriumsulfat getrocknet und i.Vak. eingeengt. Man erhält 74,1 g 3-Acetoxy-7α-(5-acetoxypentyl)-16α-brom-17,17-ethylendioxy-estra-1,3,5(10)-trien als Öl

**h) 17,17-Ethylendioxy-7α-(5-hydroxypentyl)-estra-1,3,5(10),15-tetraen-3-ol**

Eine Lösung von 74,1 g 3-Acetoxy-7α-(5-acetoxypentyl)-16α-brom-17,17-ethylendioxy-estra-1,3,5(10)-trien in 740 ml Dimethylsulfoxid und 74 ml Methanol wird mit 74 g Kaliumhydroxid 7,5 Stunden bei 85 °C Badtemperatur gerührt. Dann wird mit Eis / Wasser / Kochsalz gefällt, mit Essigester aufgenommen, neutralgewaschen, über Natriumsulfat getrocknet, i.Vak. eingeengt und an Kieselgel mit Hexan / Essigester chromatographiert. Man erhält 36,12 g reines 17,17-Ethylendioxy-7α-(5-hydroxypentyl)-estra-1,3,5(10),15-tetraen-3-ol als Schaum.

**i) 3-Hydroxy-7α-(5-hydroxypentyl)-estra-1,3,5(10),15-tetraen-17-on**

Eine Lösung von 36,12 g 17,17-Ethylendioxy-7α-(5-hydroxypentyl)-estra-1,3,5(10),15-tetraen-3-ol in 958 ml Aceton und 111 ml Wasser wird 2 Stunden bei Raumtemperatur mit 2,76 g *para*-Toluolsulfonsäure gerührt. Dann wird i. Vak. auf 1/3 des Volumens eingeengt, mit Essigester aufgenommen, neutralgewaschen, über Natriumsulfat getrocknet und i.Vak. eingeengt. Man erhält 31,7 g 3-Hydroxy-7α-(5-hydroxypentyl)-estra-1,3,5(10),15-tetraen-17-on als Kristalle vom Schmelzpunkt 194-196 °C.

**j) 3,17-Diacetoxy-7α-(5-acetoxypentyl)-estra-1,3,5(10),14,16-pentaen**

Eine Lösung von 15,7 g 3-Hydroxy-7α-(5-hydroxypentyl)-estra-1,3,5(10),15-tetraen-17-on in 330 ml Essigsäureanhydrid wird mit 4,6 g *para*-Toluolsulfonsäure 4 Stunden bei Raumtemperatur gerührt. Dann wird mit Pyridin / Wasser / Kochsalz gefällt, in Essigester aufgenommen, mit Natriumhydrogencarbonat- sowie Kochsalzlösung gewaschen, über Natriumsulfat getrocknet, i.Vak. eingeengt und an Kieselgel mit Hexan / Essigester chromatographiert. Man erhält 11,1 g reines 3,17-Diacetoxy-7α-(5-acetoxypentyl)-estra-1,3,5(10),14,16-pentaen als Öl.

**k) 3,17β-Diacetoxy-7α-(5-acetoxypentyl)-14α,17α-etheno-estra-1,3,5(10)-trien**

11,0 g 3,17-Diacetoxy-7α-(5-acetoxypentyl)-estra-1,3,5(10),14,16-pentaen in 120 ml Benzol wird bei 300 bar und 175 °C 6,5 Tage mit Ethen behandelt. Dann wird in Essigester aufgenommen, mit Natriumhydrogencarbonat- sowie Kochsalzlösung gewaschen, über Natriumsulfat getrocknet, i.Vak. eingeengt und an Kieselgel mit Hexan / Essigester chromatographiert. Man erhält 8,4 g 3,17β-Diacetoxy-7α-(5-acetoxypentyl)-14α,17α-etheno-estra-1,3,5(10)-trien als Schaum.

**l) 3,17β-Diacetoxy-7α-(5-acetoxypentyl)-14α,17α-ethano-estra-1,3,5(10)-trien**

Eine Lösung von 8,4 g 3,17β-Diacetoxy-7α-(5-acetoxypentyl)-14α,17α-etheno-estra-1,3,5(10)-trien in 200 ml Essigester wird mit 1.5 g Palladium (10%) auf Kohle eine Stunde bei Raumtemperatur unter Wasserstoff geschüttelt. Dann wird über Celite abgesaugt, mit Essigester nachgewaschen, i. Vak. eingeengt und an Kieselgel mit Hexan / Essigester chromatographiert. Man erhält 6,0 g 3,17β-Diacetoxy-7α-(5-acetoxypentyl)-14α,17α-ethano-estra-1,3,5(10)-trien als Schaum.

**m) 14α,17α-Ethano-7α-(5-hydroxypentyl)-estra-1,3,5(10)-trien-3,17β-diol**

Eine Lösung von 6,0 g 3,17β-Diacetoxy-7α-(5-acetoxypentyl)-14α,17α-ethano-estra-1,3,5(10)-trien in 100 ml einer 1 molaren Kaliumhydroxydlösung wird 7,5 Stunden bei Raumtemperatur stehengelassen. Dann wird auf 1 molare Salzsäure gegeben, dreimal mit Essigester extrahiert, mit Natriumhydrogencarbonat- sowie Kochsalzlösung gewaschen, über Natriumsulfat getrocknet, i.Vak. eingeengt und aus Aceton / Hexan umkristallisiert. Man erhält 4,57 g 14α, 17α-Ethano-7α-(5-hydroxypentyl)-estra-1,3,5(10)-trien-3,17β-diol als farblose Kristalle vom Schmelzpunkt 63-65 °C.

**n) 3-Benzyloxy-14α,17α-ethano-7α-(5-hydroxypentyl)-estra-1,3,5(10)-trien-17β-ol**

Eine Lösung von 4,5 g 14α,17α-Ethano-7α-(5-hydroxypentyl)-estra-1,3,5(10)-trien-3,17β-diol in 90 ml Acetonitril wird mit 1,91 g Kaliumcarbonat und 1,53 ml Benzylbromid 6,5 Stunden bei 80 °C Badtemperatur gerührt. Dann wird i. Vak. auf 1/3 des Volumens eingeengt, auf Wasser gegeben, dreimal mit Essigester extrahiert, neutralgewaschen, über Natriumsulfat getrocknet, i.Vak. eingeengt und an Kieselgel mit Dichlormethan / Aceton chromatographiert. Man erhält 4,8 g 3-Benzyloxy-14α,17α-ethano-7α-(5-hydroxypentyl)-estra-1,3,5(10)-trien-17β-ol als Schaum.

**o) 3-Benzyloxy-14α,17α-ethano-7α-(5-tosyloxypentyl)-estra-1,3,5(10)-trien-17β-ol**

Eine Lösung von 4,8 g 3-Benzyloxy-14α,17α-ethano-7α-(5-hydroxypentyl)-estra-1,3,5(10)-trien-17β-ol in 50 ml Pyridin wird bei 0 °C mit 3,63 g Toluolsulfonsäureanhydrid 4 Stunden gerührt. Dann wird mit Essigester verdünnt, mit 2 molarer Salzsäure extrahiert, neutralgewaschen, über Natriumsulfat getrocknet, i.Vak. eingeengt und an Kieselgel mit Hexan / Essigester chromatographiert. Man erhält 4,75 g 3-Benzyloxy-14α,17α-ethano-7α-(5-tosyloxypentyl)-estra-1,3,5(10)-trien-17β-ol als Schaum.

**p) 3-Benzyloxy-14α,17α-ethano-7α-{5-[N-methyl-N-3-(4,4,5,5,5-pentafluorpentylthio)-propylamino]-pentyl}-estra-1,3,5(10)-trien-17β-ol**

Eine Lösung von 4,7 g 3-Benzyloxy-14α,17α-ethano-7α-(5-tosyloxypentyl)-estra-1,3,5(10)-trien-17β-ol in 100 ml Dimethylformamid wird mit 2,7 g Methyl-[3-(4,4,5,5,5-pentafluorpentylthio)-propyl]-amin 4 Stunden bei 80 °C Badtemperatur gerührt. Dann wird auf Wasser gegeben, dreimal mit Essigester extrahiert, mit Kochsalzlösung gewaschen, über Natriumsulfat getrocknet, i.Vak. eingeengt und an Kieselgel mit Dichlormethan / Methanol chromatographiert. Man erhält 3,8 g 3-Benzyloxy-14α,17α-ethano-7α-{5-[N-methyl-N-3-(4,4,5,5,5-pentafluorpentylthio)-propylamino]-pentyl)-estra-1,3,5(10)-trien-17β-ol als Öl.

**q) 14α,17α-Ethano-7α-{5-[N-methyl-N-3-(4,4,5,5,5-pentafluorpentylthio)-propylamino]-pentyl}-estra-1,3,5(10)-trien-3,17β-diol**

Eine Lösung von 3,7 g 3-Benzyloxy-14α,17α-ethano-7α-{5-[N-methyl-N-3-(4,4,5,5,5-pentafluorpentansulfinyl)-propylamino]-pentyl}-estra-1,3,5(10)-trien-17β-ol in 65 ml Dichlormethan wird bei 0 °C mit 2,1 ml N,N-Dimethylanilin 5 Minuten gerührt, mit 2,75 g wasserfreiem Aluminiumchlorid versetzt und 3,5 Stunden bei 0 °C gerührt. Dann wird mit gesättigter, wäßriger Kaliumnatriumtartratlösung versetzt, auf Wasser gegeben, dreimal mit Dichlormethan extrahiert, neutralgewaschen, über Natriumsulfat getrocknet und i.Vak. eingeengt. Man erhält 6,3 g Rohprodukt, das man an Kieselgel mit Dichlormethan / Methanol chromatographiert. Man erhält 2,85 g reines 14α,17α-Ethano-7α-{5-[N-methyl-N-3-(4,4,5,5,5-pentafluorpentylthio)-propylamino]-pentyl}-estra-1,3,5(10)-trien-3,17β-diol als Kristalle vom Schmelzpunkt 63-67 °C, $[\alpha]_D^{22}$ = +19,4° (c = 0.505% in Chloroform).

Beispiel 2

**14,17-Ethano-7α-{5-[N-Methyl-N-3-(4,4,5,5,5-pentafluorpentansulfinyl)-propylamino]-pentyl}-estra-1,3,5(10)-trien-3,17β-diol**

Eine Lösung von 1,0 g 14α,17α-Ethano-7α-{5-[N-methyl-N-3-(4,4,5,5,5-pentafluorpentylthio)-propylamino]-pentyl}-estra-1,3,5(10)-trien-3,17β-diol in 37,5 ml Methanol und 1,78 ml Wasser wird mit 381 mg Natriumperiodat 5 Stunden bei Raumtemperatur gerührt. Dann wird auf Wasser gegeben, dreimal mit Dichlormethan extrahiert, neutralgewaschen, über Natriumsulfat getrocknet und i.Vak. eingeengt. Man erhält 985 mg Rohprodukt, das an Kieselgel mit Dichlormethan / Methanol chromatographiert wird. Man erhält 514,3 mg reines 14,17-Ethano-7α-{5-[N-Methyl-N-3-(4,4,5,5,5-pentafluorpentansulfinyl)-propylamino]-pentyl}-estra-1,3,5(10)-trien-3,17β-diol als Kristalle vom Schmelzpunkt 84-86 °C; $[\alpha]_D^{22}$ = +13,0° (c = 0,5% in Chloroform).

Beispiel 3

**3,17β-Diacetoxy-14α,17α-ethano-7α-{5-[N-methyl-N-3-(4,4,5,5,5-pentafluorpentylthio)-propylamino]-pentyl}-estra-1,3,5(10)-trien**

Eine Lösung von 600 mg 14α,17α-Ethano-7α-{5-[N-methyl-N-3-(4,4,5,5,5-pentafluorpentylthio)-propylamino]-pentyl}-estra-1,3,5(10)-trien-3,17β-diol in 2 ml Pyridin. und 1 ml Acetanhydrid wird mit 5 mg Dimethylaminopyridin 4,5 Stunden bei Raumtemperatur gerührt. Dann wird mit Essigester verdünnt, mit Wasser und Kochsalzlösung gewaschen, über Natriumsulfat getrocknet und i.Vak. eingeengt. Man erhält 680 mg 3,17β-Diacetoxy-14α,17α-ethano-7α-{5-[N-methyl-N-3-(4,4,5,5,5-pentafluorpentylthio)-propylamino]-pentyl}-estra-1,3,5(10)-trien als Öl.

Beispiel 4

**14,17-Ethano-7α-{5-[N-methyl-N-3-(4,4,5,5,5-pentafluorpentansulfonyl)-propylamino]-pentyl}-estra-1,3,5(10)-trien-3,17β-diol**

Eine Lösung von 650 mg 3,17β-Diacetoxy-14α,17α-ethano-7α-{5-[N-methyl-N-3-(4,4,5,5,5-pentafluorpentylthio)-propylamino]-pentyl}-estra-1,3,5(10)-trien in 15 ml Eisessig wird mit 1,5 g Natriumperborat tetrahydrat 3 Stunden bei Raumtemperatur gerührt. Dann wird auf Wasser gegeben, dreimal mit Dichlormethan extrahiert, neutralgewaschen, über Natriumsulfat getrocknet, i.Vak. eingeengt, in 10 ml 0,2 molarer methanolischer Kaliumhydroxydlösung aufgenommen, 24 Stunden bei Raumtemperatur gerührt, auf Wasser gegeben, dreimal mit Dichlormethan extrahiert, neutralgewaschen, über Natriumsulfat getrocknet, i.Vak. eingeengt, in 20 ml Essigester suspendiert, mit 10 ml Zinn(II) chloridlösung versetzt, 4 Stunden bei Raumtemperatur gerührt, mit Essigester verdünnt, mit Kochsalzlösung gewaschen, über Natriumsulfat getrocknet, i.Vak. eingeengt und an Kieselgel mit Hexan / Essigester chromatographiert. Man erhält 300 mg reines 14,17-Ethano-7α-{5-[N-methyl-N-3-(4,4,5,5,5-pentafluorpentansulfonyl)-propylamino]-pentyl}-estra-1,3,5(10)-trien-3,17β-diol als Kristalle vom Schmelzpunkt 55-58 °C; $[\alpha]_D^{22}$ = +19,4° (c = 0,51% in Chloroform).

Beispiel 5

**17α-Trifluormethyl-7α-{5-[N-methyl-N-3-(4,4,5,5,5-pentafluorpentylthio)-propylamino]-pentyl}-estra-1,3,5(10)-trien-3,17β-diol**

**a) 17β-Acetoxy-7α-{5-tert. butyl-dimethylsilyloxypentyl)-estr-4-en-3-on**

In 200 ml abs. THF werden 22,9 g Mg-Späne mit 261 g 1-Brom-5-*tert*-butyldimethylsilyloxypentan [Tetrahedron Letters 23, 1982, 40, 4147-4150] gelöst in 250 ml abs. THF zum Grignardreagenz umgesetzt. In diese auf-20°C gekühlte Lösung werden unter einem Stickstoffstrom 92,9 g Kupfer-(I)-iodid gegeben und anschließend innerhalb einer Stunde 73,5 g 17β-Acetoxyestra-4,6-dien-3-on [J. Am. Chem. Soc. 80, 1958, 2596-2597) gelöst in 300 ml abs. THF zugetropft. Zur Aufarbeitung werden 61,2 ml Essigsäure zugetropft, die Reaktionsmischung mit Essigester verdünnt, mit gesättigter Ammoniumchloridlösung, Wasser und Natriumhydrogencarbonatlösung gewaschen und getrocknet. Der nach dem Eindampfen erhaltene Rückstand wird an Kieselgel chromatographiert. Man erhält 48 g 17β-Acetoxy-7α-(5-*tert*-butyl-dimethylsilyloxypentyl)-estr-4-en-3-on.

**b) 17β-Acetoxy-7α-(5-hydroxypentyl)-estr-4-en-3-on**

Eine Lösung von 48 g 17β-Acetoxy-7α-(5-*tert*-butyl-dimethylsilyloxypentyl}-estr-4-en-3-on in 350 ml Methanol wird mit 35 ml einer 8 vol.%iger Schwefelsäure 30 Minuten bei Raumtemperatur stehengelassen. Die Lösung wird mit Diethylether verdünnt, mit Wasser neutral gewaschen, getrocknet. Nach dem Eindampfen werden 37,7 g 17β-Acetoxy-7α-(5-hydroxypentyl)-estr-4-en-3-on als Öl erhalten.

**c) 17β-Acetoxy-7α-(5-acetoxypentyl)-estr-4-en-3-on**

Eine Lösung von 37,7 g 17β-Acetoxy-7α-(5-hydroxypentyl)-estr-4-en-3-on in 160 ml Pyridin wird mit 80 ml Acetanhydrid langsam versetzt und 16 h bei 25 °C gerührt. Dann wird mit Essigester verdünnt, die organ. Phase nach dem Waschen mit Natruimhydrogencarbonatlösung, getrocknet und eingedampft. Der Rückstand wird an Kieselgel chromatographiert und es werden 26,6 g 17β-Acetoxy-7α-(5-acetoxypentyl)-estr-4-en-3-on als Öl erhalten. $[\alpha]_D^{22}$ = +20,0° (c = 0,51% in Chloroform)

**d) 17β-Acetoxy-7α-(5-acetoxypentyl)-estra-1,3,5(10)-trien-3-ol**

Zu einer auf 80°C erwärmten Lösung von 26,6 g 17β-Acetoxy-7α-(5-acetoxypentyl)-estr-4-en-3-on in 260 ml Acetonitril wird eine Lösung von 5,18 g Lithiumbromid und 26,73 g Kupfer-II-bromid in 260 ml Acetonitril innerhalb 30 Min unter Rühren zugetropft. Nach beendeter Zugabe wird die Reaktionslösung abgekühlt, mit Diethylether verdünnt, mit Wasser und Natriumhydrogencarbonatlösung gewaschen und getrocknet. Der nach dem Eindampfen erhaltene Rückstand wird an Kieselgel chromatographiert und es werden 21,3 g 17β-Acetoxy-7α-(5-acetoxypentyl)-1,3,5(10)-estratrien-3-ol als Öl erhalten. $[\alpha]_D^{22}$ = +28,9° (c = 0,535% in Chloroform)

**e) 17β-Acetoxy-7α-(5-acetoxypentyl)-3-(tetrahydropyran-2-yloxy)-estra-1,3,5(10)-trien**

Eine Lösung von 21,3 g 17β-Acetoxy-7α-(5-acetoxypentyl)-estra-1,3,5(10)-trien-3-ol in 213 ml Tetrahydrofuran wird mit 21,3 ml 3,4-Dihydro-2H-pyran und 1,065 g *p*-Toluolsulfonsäure 8 Stunden bei Raumtemperatur stehengelassen. Die Reaktionslösung wird mit 3 ml Pyridin versetzt, dann mit Diethylether verdünnt, mit Wasser gewaschen und getrocknet. Der nach dem Eindampfen erhaltenen Rückstand wird an Kieselgel chromatographiert und es werden 24,3 g 17β-Acetoxy-7α-(5-acetoxypentyl)-3-(tetrahydropyran-2-yloxy)-estra-1,3,5(10)-trien als Öl erhalten. $[\alpha]_D^{22}$ = +31,5° (c = 0,535% in Chloroform)

**f) 17β-Acetoxy-7α-(5-hydroxypentyl)-3-(tetrahydropyran-2-yloxy)-estra-1,3,5(10)-trien**

Eine Lösung von 10,2 g 17β-Acetoxy-7α-(5-acetoxypentyl)-3-(tetrahydropyran-2-yloxy)-estra-1,3,5(10)-trien in 205 ml Methanol wird mit 33,7 ml Natronlauge 45 Minuten bei 15 °C gerührt. Dann wird mit Diethylether verdünnt, mit Wasser gewaschen, über Natriumsulfat getrocknet und i.Vak. eingeengt. Man erhält 5.6 g17β-Acetoxy-7α-(5-hydroxypentyl)-3-(tetrahydropyran-2-yloxy)-estra-1,3,5(10)-trien. $[\alpha]_D^{22}$ = +32,2° (c = 0.505% in Chloroform)

**g) 17β-Acetoxy-3-(tetrahydropyran-2-yloxy)-7α-(5-*p*-toluolsulfonyloxypentyl)-estra-1,3,5(10)-trien**

Eine Lösung von 5,5 g 17β-Acetoxy-7α-(5-hydroxypentyl)-3-(tetrahydropyran-2-yloxy)-estra-1,3,5(10)-trien in 47 ml Pyridin wird mit 5,5 g *p*-Toluolsulfonsäureanhydrid 45 Minuten bei Raumtemperatur stehengelassen. Anschließend wird die Reaktionslösung im Eisbad abgekühlt, mit 4 ml Wasser versetzt und 45 Minuten nachgerührt. Es wird dann mit Essigester verdünnt, mit Wasser gewaschen, getrocknet und eingedampft. Es werden 8,2 g 17β-Acetoxy-3-(tetrahydropyran-2-yloxy)-7α-(5-*p*-toluolsulfonyloxypentyl)-estra-1,3,5,(10)-trien als Öl erhalten.

**h) 17β-Acetoxy-7α-(5-methylaminopentyl)-3-(tetrahydropyran-2-yloxy)-estra-1,3,5(10)-trien**

In eine Lösung von 8,2 g 17β-Acetoxy-3-(tetrahyropyran-2-yloxy)-7α-(5-*p*toluolsulfonyloxypentyl)-estra-1,3,5 (10)-trien in 80 ml Tetrahydrofuran werden in ein Druckrohr 6,3 g Methylamin bei Eiskühlung einkondensiert. Das verschlossene Druckrohr wird dann 6 Stunden auf 60°C erhitzt. Nach dem Abkühlen wird i. Vakuum zur Trockene eingedampft und der Rückstand an Kieselgel chromatographiert. Es werden 5,1 g 17β-Acetoxy-7α-(5-methylaminopentyl)-3-(tetrahydropyran-2-yloxy)-estra-1,3,5(10)-trien als Öl erhalten. $[\alpha]^{22}_{D}$ = +29,7° (c = 0.535% in Chloroform)

**i) 17β-Acetoxy-7α-{5-[N-methyl-N-3-(4,4,5,5,5-pentafluorpentyl-thio)-propylamino]-pentyl}-3-(tetrahydropyran-2-yloxy)-estra-1,3,5(10)-trien**

Eine Lösung von 1,64 g 17β-Acetoxy-7α-(5-methylaminopentyl)-3-(tetrahydropyran-2-yloxy)-estra-1,3,5(10)-trien in 25 ml abs. DMF wird mit 159 mg 80%igem Natriumhydrid unter Stickstoff 2 Stunden bei Raumtemperatur gerührt. Es werden dann 1,43 g 3-Chlorpropyl-4,4,5,5,5-pentafluorpentylsulfid in 7 ml abs. DMF zugetropft und anschließend 22 Stunden bei 80°C nachgerührt. Die Reaktionslösung wird dann mit Essigester verdünnt, mit Wasser gewaschen, getrocknet, eingedampft und der Rückstand an Kieselgel chromatographiert. Es werden 820 mg 17β-Acetoxy-7α-{5-[N-methyl-N-3-(4,4,5,5,5-pentafluorpentylthio)-propylamino]-pentyl}-3-(tetrahydropyran-2-yloxy-estra-1,3,5(10)-trien als Öl erhalten.
$[\alpha]^{22}_{D}$ = +21,5° (c = 0,51% in Chloroform)

**j) 7α-{5-[N-Methyl-N-3-(4,4,5,5,5-pentafluor-pentylthio)-propylamino]-pentyl}-3-(tetrahydropyran-2-yloxy)-estra-1,3,5(10)-trien-17β-ol**

Eine Lösung von 790 mg 17β-Acetoxy-7α-{5-[N-Methyl-N-3-(4,4,5,5,5-pentafluor-pentylthio)-propylamino]-pentyl}-3-(tetrahydropyran-2-yloxy)-estra-1,3,5(10)-trien in 8 ml Methanol und 3 ml THF wird mit 430 mg Kaliumcarbonat 18 Stunden bei Raumtemperatur gerührt. Die Reaktionslösung wird mit Diethylether verdünnt, mit Wasser gewaschen, getrocknet und eingedampft. Es werden 750 mg rohes 7α-{5-N-Methyl-N-3-(4,4,5,5,5-pentafluor-pentylthio)-propylamino]-pentyl}-3-(tetrahydropyran-2-yloxy)-estra-1,3,5(10)-trien-17β-ol erhalten.

**k) 7α-{5-[N-Methyl-N-3-(4,4,5,5,5-pentafluor-pentylthio)-propylamino]-pentyl}-estra-1,3,5(10)-trien-3,17β-diol**

Eine Lösung von 750 mg 7α-{5-[N-Methyl-N-3-(4,4,5,5,5-pentafluor-pentylthio)-propylamino]-pentyl}-3-(tetrahydropyran-2-yloxy)-estra-1,3,5(10)-trien-17β-ol in 28 ml Methanol und 2,8 ml Wasser wird mit 350 mg Oxalsäure 17 Stunden bei Raumtemperatur gerührt. Es wird dann mit Essigester verdünnt, mit Natriumhydrogencarbonatlösung und Wasser gewaschen, getrockent und eingedampft. Der Rückstand wird an Kieselgel chromatographiert und es werden 640 mg 7α-{5-[N-Methyl-N-3-(4,4,5,5,5-pentafluor-pentylthio)-propylamino]-pentyl}-estra-1,3,5(10)-trien-3,17β-diol als Öl erhalten. $[\alpha]^{22}_{D}$ = +24,0° (c = 0,515% in Chloroform)

**l) 7α-{5-[N-Methyl-N-3-(4,4,5,5,5-pentafluor-pentylthio)-propylamino]-pentyl}-3-(tetrahydropyran-2-yloxy)-estra-1,3,5(10)-trien-17-on**

Eine Lösung von 900 mg 7α-{5-[N-Methyl-N-3-(4,4,5,5,5-pentafluor-pentylthio)-propylamino]-pentyl}-3-(tetrahydropyran-2-yloxy)-estra-1,3,5(10)-trien-17β-ol wird in 30 ml Toluol und 9,6 ml Cyclohexanon mit einer Lösung von 900 mg Aluminiumisopropylat in 16 ml Toluol versetzt und 30 Minuten unter langsamen abdestillieren erhitzt. Die Reaktions-lösung wird dann mit Essigester verdünnt, mit 20%iger Kaliumnatriumtartratlösung gewaschen, getrocknet und eingedampft. Der Rückstand wird an Kieselgel mit Hexan / Essigester chromatographiert. Man erhält 715 mg 7α-{5-[N-Methyl-N-3-(4,4,5,5,5-pentafluorpentylthio)-propylamino]-pentyl}-3-(tetrahydropyran-2-yloxy)-estra-1,3,5(10)-trien-17-on als Öl.

**m) 17α-Trifluormethyl-3-(tetrahydropyran-2-yloxy)-7α-{5-[N-methyl-N-3-(4,4,5,5,5-pentafluorpentylthio)-propylamino]-pentyl}-estra-1,3,5(10)-trien-17β-ol**

Eine Lösung von 500 mg 7α-{5-[N-Methyl-N-3-(4,4,5,5,5-pentafluorpentylthio)-propylamino]-pentyl}-3-(tetrahydropyran-2-yloxy)-estra-1,3,5(10)-trien-17-on (s. Ausw.-Pat. Beisp. 2a) in 5 ml abs. Tetrahydrofuran wird bei 0 °C Badtemperatur mit 0,27 ml (Trifluormethyl)-trimethylsilan versetzt. Anschließend werden 0,2 ml einer 1,1 molaren Tetrabutylammoniumfluorid-Lösung langsam zugetropft und 45 Minuten gerührt, nochmal 1 ml der 1,1 molaren Tetrabutylammoniumfluorid-Lösung zugegeben und weitere 30 Minuten gerührt. Dann wird mit Essigester verdünnt, mit Wasser gewaschen, über Natriumsulfat getrocknet, i.Vak. eingeengt und an Kieselgel mit Dichlormethan / Methanol chromatographiert. Man erhält 285 mg 17α-Trifluormethyl-3-(tetrahydropyran-2-yloxy)-7α-{5-[N-methyl-N-3-(4,4,5,5,5-pentafluorpentylthio)-propylamino]-pentyl}-estra-1,3,5(10)-trien-17β-ol als Öl.

**n) 17α-Trifluormethyl-7α-{5-[N-methyl-N-3-(4,4,5,5,5-pentafluorpentylthio)-propylamino]-pentyl}-estra-1,3,5 (10)-trien-3,17β-diol**

Eine Lösung von 280 mg 17α-Trifluormethyl-3-(tetrahydropyran-2-yloxy)-7α-{5-[N-methyl-N-3-(4,4,5,5,5-pentafluorpentylthio)-propylamino]-pentyl}-estra-1,3,5(10)-trien-17β-ol in 5,6 ml Methanol und 0,56 ml Wasser wird mit 140 mg Oxalsäure 18 Stunden bei Raumtemperatur gerührt. Dann wird mit Essigester verdünnt, mit Natriumhydrogencarbonat und Wasser gewaschen, über Natriumsulfat getrocknet, i.Vak. eingeengt und an Kieselgel mit Dichlormethan / Methanol chromatographiert. Man erhält 215 mg 17α-Trifluormethyl-7α-{5-[N-methyl-N-3-(4,4,5,5,5-pentafluorpentylthio)-propylamino]-pentyl}-estra-1,3,5(10)-tries-3,17β-diol als Öl.

Beispiel 6

**15β,16β-Methano-17α-methyl-7α-{5-[N-methyl-N-3-(4,4,5,5,5-pentafluorpentylthio)-propylamino]-pentyl}-estra-1,3,5(10)-trien-3,17β-diol**

**a) 3-Benzyloxy-17,17-ethylendioxy-7α-(5-hydroxypentyl)-estra-1,3,5(10),15-tetraen**

Eine Lösung von 32,7 g 17,17-Ethylendioxy-7α-(5-hydroxypentyl)-estra-1,3,5(10),15-tetraen-3-ol (s. Beisp. 1h) in 327 ml Dimethylformamid wird bei Raumtemperatur mit 5,73 g Lithiumhydroxid versetzt und mit 15,1 ml Benzylbromid 2 Stunden bei 60 °C gerührt. Dann wird mit Essigester verdünnt, mit Wasser gewaschen, über Natriumsulfat getrocknet, i.Vak. eingeengt und an Kieselgel mit Dichlormethan / Methanol chromatographiert. Man erhält 32,8 g 3-Benzyloxy-17,17-ethylendioxy-7α-(5-hydroxypentyl)-estra-1,3,5(10),15-tetraen.

**b) 3-Benzyloxy-7α-(5-hydroxypentyl)-estra-1,3,5(10),15-tetraen-17-on**

Eine Lösung von 32,8 g 3-Benzyloxy-17,17-ethylendioxy-7α-(5-hydroxypentyl)-estra-1,3,5(10),15-tetraen in 328 ml Aceton und 32,8 ml Wasser wird bei Raumtemperatur mit 1,033 g *para*-Toluolsulfonsäure 2 Stunden gerührt. Dann wird mit Diethylether verdünnt, mit Natriumhydrogencarbonat und Wasser gewaschen, über Natriumsulfat getrocknet, i.Vak. eingeengt und an Kieselgel mit Dichlormethan / Aceton chromatographiert. Man erhält 25,4 g 3-Benzyloxy-7α-(5-hydroxypentyl)-estra-1,3,5(10),15-tetraen-17-on.

**c) 3-Benzyloxy-7α-(5-hydroxypentyl)-15β,16β-methano-estra-1,3,5(10),15-tetraen-17-on**

In 65 ml Dimethylsulfoxid werden 2,861 g Trimethylsulfoxoniumiodid mit 345 mg 80%igem Natriumhydrid über 2 Stunden bei Raumtemperatur umgesetzt. In diese Lösung werden 4,44 g 3-Benzyloxy-7α-(5-hydroxypentyl)-estra-1,3,5(10),15-tetraen-17-on gegeben und 45 Minuten bei Raumtemperatur gerührt Der ausgefallene Niederschlag wird abfiltriert und mit Wasser gewaschen. Dieser Niederschlag wird mit Essigester aufgenommen, mit Wasser gewaschen, über Natriumsulfat getrocknet, i.Vak. eingeengt und an Kieselgel mit Hexan / Aceton chromatographiert. Man erhält 2,8 g 3-Benzyloxy-7α-(5-hydroxypentyl)-15β,16β-methano-estra-1,3,5(10),15-tetraen-17-on. $[\alpha]_D^{22}$ = +13,1° (c = 0.525% in Chloroform).

**d) 3-Benzyloxy-15β,16β-methano-7α-(5-tosyloxypentyl)-estra-1,3,5(10),15-tetraen-17-on**

Eine Lösung von 2,5 g 3-Benzyloxy-7α-(5-hydroxypentyl)-15β,16β-methano-estra-1,3,5(10),15-tetraen-17-on in 25 ml Pyridin wird bei Raumtemperatur mit 2,5 g *para*-Toluolsulfonsäureanhydrid versetzt und 30 Minuten stehengelassen, unter Eiskühlung mit 1 ml Wasser versetzt und weitere 45 Minuten stehengelassen. Dann wird mit

Diethylether verdünnt, mit Wasser gewaschen, über Natriumsulfat getrocknet und i.Vak. eingeengt. Man erhält 3,4 g rohes 3-Benzyloxy-15β,16β-methano-7α-(5-tosyloxypentyl)-estra-1,3,5(10),15-tetraen-17-on.

**e) 3-Benzyloxy-15β,16β-methano-7α-{5-[N-methyl-N-3-(4,4,5,5,5-pentafluor-pentylthio)-propylamino]-pentyl}-estra-1,3,5(10),15-tetraen-17-on**

Zu einer Lösung von 3,4 g rohem 3-Benzyloxy-15β,16β-methano-7α-(5-tosyloxypentyl)-estra-1,3,5(10),15-tetraen-17-on in 34 ml Dimethylformamid wird eine Lösung von 2,1 g Methyl-[3-(4,4,5,5,5-pentafluorpentylthio)-propyl]-amin in 1 ml Dimethylformamid gegeben und 3,5 Stunden bei 100 °C gerührt. Dann wird mit Essigester verdünnt, mit Wasser gewaschen, über Natriumsulfat getrocknet, i.Vak. eingeengt und an Kieselgel mit Dichlormethan / Methanol chromatographiert. Man erhält 2,6 g 3-Benzyloxy-15β,16β-methano-7α-{5-[N-methyl-N-3-(4,4,5,5,5-pentafluor-pentylthio)-propylamino]-pentyl}estra-1,3,5(10),15-tetraen-17-on als Öl.

**f) 15β,16β-Methano-7α-{5-[N-methyl-N-3-(4,4,5,5,5-pentafluor-pentylthio)-propylamino]-pentyl}-3-hydroxy-estra-1,3,5(10),15-tetraen-17-on**

Eine Lösung von 2,3 g 3-Benzyloxy-15β,16β-methano-7α-{5-[N-methyl-N-3-(4,4,5,5,5-pentafluor-pentylthio)-propylamino]-pentyl}-estra-1,3,5(10),15-tetraen-17-on in 53 ml Dichlormethan wird bei 0 °C Badtemperatur mit 1,12 ml N,N-Dimethylanilin und 1,64 g Aluminiumchlorid (wasserfrei) 4,5 Stunden gerührt. Dann wird mit Essigester verdünnt, mit 30%iger Kalium-Natriumtartratlösung und Wasser gewaschen, über Natriumsulfat getrocknet, i.Vak. eingeengt und an Kieselgel mit Dichlormethan / Methanol chromatographiert. Man erhält 1,52 g 15β,16β-Methano-7α-{5-[N-methyl-N-3-(4,4,5,5,5-pentafluor-pentylthio)-propylamino]-pentyl}-3-hydroxy-estra-1,3,5(10),15-tetraen-17-on als Öl. $[\alpha]_D^{22}$ = -2,5° (c = 0.505% in Chloroform).

**g) 15β,16β-Methano-17α-methyl-7α-{5-[N-methyl-N-3-(4,4,5,5,5-pentafluor-pentylthio)-propylamino]-pentyl}-estra-1,3,5(10)-trien-3,17β-diol**

Zu einer Lösung von 515 mg 15β,16β-Methano-7α-{5-[N-methyl-N-3-(4,4,5,5,5-pentafluor-pentylthio)-propylamino]-pentyl}-3-hydroxy-estra-1,3,5(10),15-tetraen-17-on in 10 ml Tetrahydrofuran werden bei 0 °C Badtemperatur 1,8 ml einer 3 molaren Methylmagnesiumbromidlösung getropft und 2 Stunden bei Raumtemperatur gerührt. Dann wird mit Essigester verdünnt, mit gesättigter Ammoniumchloridlösung und Wasser gewaschen, über Natriumsulfat getrocknet, i.Vak. eingeengt und an Kieselgel mit Dichlormethan / Methanol chromatographiert. Man erhält 395 mg 15β,16β-Methano-17α-methyl-7α-{5-[N-methyl-N-3-(4,4,5,5,5-pentafluor-pentylthio}-propylamino]-pentyl}-estra-1,3,5(10)-trien-3,17β-diol als Öl. $[\alpha]_D^{22}$ = -5,1° (c = 0.52% in Chloroform)

Beispiel 7

**15β,16β-Methano-17α-methyl-7α-{5-[N-methyl-N-3-(4,4,5,5,5-pentafluor-pentansulfinyl)-propylamino]-pentyl}-estra-1,3,5(10)-trien-3,17β-diol**

Eine Lösung von 115 mg 15β,16β-Methano-17α-methyl-7α-{5-[N-methyl-N-3-{4,4,5,5,5-pentafluor-pentylthio)-propylamino]-pentyl}-estra-1,3,5(10)-trien-3,17β-diol (s. Beisp. 6) in 5 ml Methanol wird mit 80 mg Natriumperiodat 3 Stunden bei Raumtemperatur gerührt. Dann wird mit Essigester verdünnt, mit Wasser gewaschen, über Natriumsulfat getrocknet, i.Vak. eingeengt und an Kieselgel mit Dichlormethan / Methanol chromatographiert. Man erhält 65 mg 15β,16β-Methano-17α-methyl-7α-{5-[N-methyl-N-3-(4,4,5,5,5-pentafluor-pentansulfinyl)-propylamino]-pentyl}-estra-1,3,5(10)-trien-3,17β-diol als Öl. $[\alpha]_D^{22}$ = 13,3° (c = 0.27% in Chloroform)

Beispiel 8

**15β,16β-Methano-7α-{5-[N-methyl-N-3-(4,4,5,5,5-pentafluor-pentylthio)-propylamino]-pentyl}-estra-1,3,5(10)-trien-3,17β-diol**

Eine Lösung von 500 mg 15β,16β-Methano-7α-{5-[N-methy]-N-3-(4,4,5,5,5-pentafluor-pentylthio)-propylamino]-pentyl}-3-hydroxy-estra-1,3,5(10),15-tetraen-17-on (s. Beisp. 6f) in 10 ml Methanol und 1 ml Wasser wird mit 100 mg Natriumborhydrid 3 Stunden bei Raum-temperatur gerührt. Dann wird mit Essigester verdünnt, mit Wasser gewaschen, über Natriumsulfat getrocknet, i.Vak. eingeengt und an Kieselgel mit Dichlormethan / Methanol chromatographiert. Man erhält 340 mg 15β,16β-Methano-7α-{5-[N-methyl-N-3-(4,4,5,5,5-pentafluor-pentylthio)-propylamino]-pentyl}-estra-1,3,5(10)-trien-3,17β-diol als Öl $[\alpha]_D^{22}$ = +11,9° (c = 0.52% in Chloroform)

Beispiel 9

**15β,16β-Methano-7α-{5-[N-methyl-N-3-(4,4,5,5,5-pentafluor-pentansulfinyl)-propylamino]-pentyl}-estra-1,3,5(10)-trien-3,17β-diol**

Eine Lösung von 100 mg 15β,16β-Methano-7α-{5-[N-methyl-N-3-(4,4,5,5,5-pentafluor-pentylthio)-propylamino)-pentyl}-estra-1,3,5(10)-trien-3,17β-diol (s. Beisp. 8) in 5 ml Methanol wird mit 80 mg Natriumperiodat 3 Stunden bei Raumtemperatur gerührt. Dann wird mit Essigester verdünnt, mit Wasser gewaschen, über Natriumsulfat getrocknet, i.Vak. eingeengt und an Kieselgel mit Dichlormethan / Methanol chromatographiert. Man erhält 70 mg 15β,16β-Methano-7α-{5-[N-methyl-N-3-(4,4,5,5,5-pentafluor-pentansulfinyl)-propylamino]-pentyl}-estra-1,3,5(10)-trien-3,17β-diol als Öl. $[\alpha]_D^{22}$ = +12,2° (c = 0.515% in Chloroform).

Beispiel 10

**15β-Methyl-7α-{5-[N-methyl-N-3-(4,4,5,5,5-pentafluor-pentylthio)-propylamino]-pentyl}-estra-1,3,5(10)-trien-3,17β-diol**

**a) 3-Benzyloxy-7α-(5-hydroxypentyl)-15β-methyl-estra-1,3,5(10)-trien-17-on**

Zu einer eisgekühlten Lösung von 11,9 ml einer 3 molaren Methylmagnesiumbromidlösung in 68 ml Tetrahydrofliran werden unter einem Stickstoffstrom 5,35 g Kupfer-I-iodid gegben. Anschliesend wird eine Lösung von 4,26 g 3-Benzyloxy-7α-(5-hydroxypentyl)-estra-1,3,5(10),15-tetraen-17-on (s. Beisp. 6b) in 50 ml Tetrahydrofuran zugetropft und 30 Minuten bei 0 °C Badtemperatur gerührt. Dann wird mit gesättigter Ammoniumchloridlösung das überschüssige Reagenz zersetzt, mit Essigester verdünnt, mit Ammoniumchloridlösung und Wasser gewaschen, über Natriumsulfat getrocknet, i.Vak. eingeengt und an Kieselgel mit Hexan / Aceton chromatographiert. Man erhält 3,6 g 3-Benzyloxy-7α-(5-hydroxypentyl)-15β-methyl-estra-1,3,5(10)-trien-17-on als Öl. $[\alpha]_D^{22}$ = +66,4° (c = 0.515% in Chloroform).

**b) 3-Benzyloxy-15β-methyl-7α-(5-tosyloxypentyl)-estra-1,3,5(10)-trien-17-on**

Eine Lösung von 3,6 g 3-Benzyloxy-7α-(5-hydroxypentyl)-15β-methyl-estra-1,3,5(10)-trien-17-on wird wie im Beisp. 6d beschieben tosyliert. Man erhält 4,9 g rohes 3-Benzyloxy-15β-methyl-7α-(5-tosyloxypentyl)-extra-1,3,5(10)-trien-17-on.

**c) 3-Benzyloxy-15β-methyl-7α-(5-N-methylaminopentyl)-extra-1,3,5(10)-trien-17-on**

In eine Lösung von 4,9g 3-Benzyloxy-15β-methyl-7α-(5-tosyloxypentyl)-estra-1,3,5(10)-trien-17-on in 30 ml Tetrahydrofuran im Druckreaktor werden unter Kühlung 3,8 g Methylamin kondensiert, der geschlossene Reaktor wird 5,5 Stunden auf 80 °C erhitzt, abgekühlt und geöffnet. Dann wird mit Essigester verdünnt, mit Wasser gewaschen, über Natriumsulfat getrocknet, i. Vak. eingeengt und an Kieselgel mit Dichlormethan / Methanol / 1% Triethylamin chromatographiert. Man erhält 3,15 g 3-Benzyloxy-15β-methyl-7α-(5-N-methylaminopentyl)-estra-1,3,5(10)-trien-17-on als Öl.

**d) 3-Benzyloxy-15β-methyl-7α-{5-[N-methyl-N-3-(4,4,5,5,5-pentafluor-pentylthio)-propylamino]-pentyl}-estra-1,3,5(10)-trien-17-on**

Eine Lösung von 3,15 g 3-Benzyloxy-15β-methyl-7α-(5-N-methylaminopentyl)-estra-1,3,5(10)-trien-17-on in 31,5 ml abs. Dimethylformamid wird mit 228 mg 80%igen Natriumhydrid versetzt und 5 Stunden bei Raumtemperatur gerührt, mit 2,6 g 3-Chlorpropyl-4,4,5,5,5-pentafluorpentylsulfid in 2ml abs. Dimethylformamid versetzt und 24 h bei 80 °C gerührt. Dann wird mit Essigester verdünnt, mit Natriumhydrogencarbonat und Wasser gewaschen, über Natriumsulfat getrocknet, i.Vak. eingeengt und an Kieselgel mit Dichlormethan / Methanol chromatographiert. Man erhält 3,1 g 3-Benzyloxy-15β-methyl-7α-{5-[N-methyl-N-3-(4,4,5,5,5-pentafluor-pentylthio)-propylamino]-pentyl}-estra-1,3,5(10),15-trien-17-on als Öl.

**e) 3-Hydroxy-15β-methyl-7α-{5-[N-methyl-N-3-(4,4,5,5,5-pentafluor-pentylthio)-propylamino]-pentyl}-estra-1,3,5(10)-trien-17-on**

Eine Lösung von 3,1 g 3-Benzyloxy-15β-methyl-7α-{5-[N-methyl-N-3-(4,4,5,5,5-pentafluor-pentylthio)-propylamino]-pentyl}-estra-1,3,5(10)-trien-17-on wird wie im Beisp. 6f beschrieben debenzyliert. Man erhält 830 mg 3-Hydroxy-15β-methyl-7α-{5-[N-methyl-N-3-(4,4,5,5,5-pentafluor-pentylthio)-propylamino]-pentyl}-estra-1,3,5(10)-trien-17-on als Öl.

**f) 15β-Methyl-7α-{5-[N-methyl-N-3-(4,4,5,5,5-pentafluor-pentylthio)-propylamino]-pentyl}-estra-1,3,5(10)-trien-3,17β-diol**

Eine Lösung von 460 mg 3-Hydroxy-15β-methyl-7α-{5-[N-methyl-N-3-(4,4,5,5,5-pentafluor-pentylthio)-propylamino]-pentyl}-estra-1,3,5(10)-trien-17-on wird wie im Beisp. 8 beschrieben reduziert. Man erhält 200 mg 15β-Methyl-7α-{5-[N-methyl-N-3-(4,4,5,5,5-pentafluor-pentylthio)-propylamino]-pentyl}-estra-1,3,5(10)-trien-3,17β-diol als Öl. $[\alpha]_D^{22}$ = +4,5° (c = 0.51% in Chloroform).

Beispiel 11

**15β,17α-Dimethyl-7α-{5-[N-methyl-N-3-(4,4,5,5,5-pentafluor-pentylthio)-propylamino]-pentyl}-estra-1,3,5(10)-trien-3,17β-diol**

In 15 ml Tetrahydrofuran werden 1,44 g getrocknetes Cer(III)chlorid 2 Stunden bei Raumtemperatur gerührt, unter Eiskühlung mit 3,75 ml einer 3-molaren Methylmagnesiumbromidlösung versetzt, 15 Minuten bei Kühlung und 30 Minuten bei Raumtemperatur gerührt, eine Lösung von 650 mg 3-Hydroxy-15β-methyl-7α-{5-[N-methyl-N-3-(4,4,5,5,5-pentafluor-pentylthio)-propylamino]-pentyl}-estra-1,3,5(10)-trien-17-on (s. Beisp. 10 e) in 7 ml Tetrahydrofuran zugetropft, 2 Stunden bei Raumtemperatur gerührt und mit gesättigter Ammoniumchloridlösung überschüssiges Reagenz zersetzt. Dann wird mit Essigester verdünnt, mit gesättigter Ammoniumchloridlösung und Wasser gewaschen, über Natriumsulfat getrocknet, i.Vak. eingeengt und an Kieselgel mit Dichlormethan / Methanol chromatographiert. Man erhält 145 mg 15β,17α-Dimethyl-7α-{5-[N-methyl-N-3-(4,4,5,5,5-pentafluor-pentylthio)-propylamino]-pentyl}-estra-1,3,5(10)-trien-3,17β-diol als Öl. $[\alpha]_D^{22}$ = -7,3° (c = 0.505% in Chloroform).

Beispiel 12

**11β-Fluor-7α-{5-(N-methyl-N-3-(4,4,5,5,5-pentafluorpentylthio)-propytamino]-pentyl}-estra-1,3,5(10)-trien-3,17β-diol**

**a) 11β-Fluor-estr-4-en-3,17-dion**

Zu 5,0 g 11α-Hydroxy-estr-4-en-3,17-dion in 100 ml Toluol und 7,3 ml 1,8-Diazabicyclo[5,4,0]undec-7-en werden bei 0°C 4,6 ml Perfluorbutan-1-sulfonsäurefluorid getropft. Nach 30 min verdünnt man die Lösung mit Essigester, wäscht mit gesättigter Natriumchlordlösung, trocknet und engt im Vakuum ein. Nach Chromatographieren des Rohproduktes an Kieselgel mit einem Hexan-Essigester -Gradienten werden 3,8 g 11β-Fluor-estr-4-en-3,17-dion vom Schmelzpunkt 173-174°C erhalten.

**b) 11β-Fluor-3-methoxy-estra-3,5-dien-17-on**

7,8 g 11β-Fluor-estr-4-en-3,17-dion werden in 40 ml 2,2-Dimethoxypropan mit 780 mg Pyridinium-toluol-4-sulfonat 5 h bei 80°C gerührt. Anschließend setzt man 1,5 ml Triethylamin zu, verdünnt mit Essigester und wäscht mit gesättigter Natriumchlorilösung. Nach Kristallisieren aus Methanol weren 5,3 g 11β-Fluor-3-methoxy-estra-3,5-dien-17-on vom Schmelzpunkt 173°C erhalten.

**c) 11β-Fluor-estra-4,6-dien-3,17-dion**

Zu 5,0 g 11β-Fluor-3-methoxy-estra-3,5-dien-17-on in 50 ml DMF gibt man bei 0°C nacheinander 5 ml einer 10 proz. Natriumacetatlöung und portionsweise 2,5 g 1,3-Dibrom-5,5-dimethylhydantoin. Nach 30 min setzt man 2,3 g Natriumsulfit und anschließend 2,5 g Lithiumbromid und 2,0 g Lithiumcarbonat zu.und rührt 2 h bei 100°C. Das Reaktionsgemisch wird in Eis-Wasser eingerührt. Man saugt das ausgefallene Produkt ab, löst in Essigester, wäscht mit Wasser, trocknet und engt im Vakuum ein. Nach Umkristallisieren aus Essigester werden 3,6g 11β-Fluor-estra-4,6-dien-3,17-dion vom Schmelzpunkt 198°C erhalten.

**d) 11β-Fluor-7α-(5-*tert.*-butyl-dimethylsilyloxypentyl)-estr-4-en-3,17-dion**

7,9 g Magnesium in 40 ml THF werden unter Stickstofif mit einer Lösung von 95,3 g 1-Brom-5-*tert.*-butyl-dimethylsilyloxypentan [Tetrahedron Letters 1982, 4147-4150] in 260 ml THF zum Grignardreagenz umgestzt. Bei -30°C gibt man 32 g Kupfer(I)-iodid und anschließend tropfenweise 29 g 11β-Fluor-estra-4,6-dien-3,17-dion in 290 ml THF zu. Nach beendeter Reaktion versetzt man mit 20,4 ml Eisessig und rührt das Reaktionsgemisch in Eis-Wasser ein. Das ausgefallene Produkt wird abgesaugt, in Essigester gelöst, mit Wasser neutral gewaschen und getrocknet. Nach Chromatographieren des Rohproduktes an Kieselgel mit einem Hexan-Essigester-Gradienten werden 23,9 g 11β-Fluor-7α-(5-*tert*.-butyldimethylsilyloxypentyl)-estr-4-en-3,17-dion als Schaum erhalten.

**e) 11β-Fluor-7α-(5-hydroxypentyl)-estr-4-en-3,17-dion**

Eine Lösung von 23,1 g 11β-Fluor-7α-(5-*tert*.-butyl-dimethylsilyloxypentyl)-estr-4-en-3,17-dion in 115 ml THF und 64 ml Wasser werden mit 128 ml Eisessig 2,5 h bei 50°C gerührt. Das Reaktionsgemisch wird im Vakuum eingeengt, in Essigester aufgenommen, mit Wasser gewaschen und getrocknet. Man erhält 20,4 g 11β-Fluor-7α-(5-hydroxypentyl)-estr-4-en-3,17-dion als Schaum.

**f) 7α-(5-Acetoxypentyl)-11β-fluor-estr-4-en-3,17-dion**

20 g 11β-Fluor-7α-(5-hydroxypentyl)-estr-4-en-3,17-dion in 100 ml Pyridin läßt man 2 h mit 50 ml Acetanhydrid bei 25°C reagieren. Anschließend setzt man bei 0°C 5 ml Wasser zu und rührt 45 min. Man extrahiert mit Diethylether, wäscht mit 2N Schwefelsäure pyridinfrei und neutralisiert die Lösung nacheinander mit gesättigter Natriumhydrogencarbonatlösung und Wasser. Nach Trocknen und Einengen im Vakuum wird das Rohprodukt an Kieselgel mit einem Hexan-Essigester-Gradienten chromatographiert. Es werden 17 g 7α-(5-Acetoxypentyl)-11β-fluor-estr-4-en-3,17-dion vom Schmelzpunkt 78,4°C erhalten.

**g) 7α-(5-Acetoxypentyl)-11β-fluor-3-hydroxy-estra-1,3,5(10)-trien-17-on**

Zu 16,5 g 7α-(5-Acetoxypentyl)-11β-fluor-estr-4-en-3,17-dion in 190 ml Acetonitril werden bei 80°C 18,6 Kupfer(II)-bromid und 3,6 g Lithiumbromid gegeben. Nach 15 min wird das Reaktionsgemisch in natriumhydrogencarbonathaltiges Eis-Wasser eingerührt. Das ausgefallene Produkt wird abgesaugt, in Essigester gelöst, mit Wasser gewaschen, getrocknet und im Vakuum eingeengt. Nach Chromatographieren des Rohproduktes an Kieselgel mit einem Hexan-Essigester-Gradienten werden 8,5 g 7α-(5-Acetoxypentyl)-11β-fluor-3-hydroxy-estra-1,3,5(10)-trien-17-on als Schaum erhalten.

**h) 7α-(5-Acetoxypentyl)-11β-fluor-3-(tetrahydropyran-2-yloxy)-estra-1,3,5(10)-trien-17-on**

8,2 g 7α-(5-Acetoxypentyl)-11β-fluor-3-hydroxy-estra-1,3,5(10)-trien-17-on in 86 ml THF werden mit 8,6 ml 3,4-Dihydro-2H-pyran und 820 mg p-Toluolsulfonsäure Hydrat 2,5 h bei RT gerührt. Anschließend setzt man 0,5 ml Triethylamin zu, verdünnt mit Essigester, wäscht mit gesättigter Natriumchloridlösung, trocknet und engt im Vakuum ein. Nach Chromatographieren des Rohproduktes an Kieselgel mit einem Hexan-Essigester-Gradienten werden 7,8 g 7α-(5-Acetoxypentyl)-11β-fluor-3-(tetrahydropyran-2-yloxy)-estra-1,3,5(10)-trien-17-on als Schaum erhalten.

**i) 11β-Fluor-7α-(5-hydroxypentyl)-3-(tetrahydropyran-2-yloxy)-estra-1,3,5(10)-trien-17-on**

7,4 g 7α-(5-Acetoxypentyl)-11β-fluor-3-(tetrahydropyran-2-yloxy)-estra-1,3,5(10)-trien-17-on in 370 ml Methanol und 37 ml Wasser werden bei Raumtemperatur mit 1,8 g Kaliumcarbonat gerührt. Nach 3 h gibt man das Reaktionsgemisch in Eis-Wasser. Das ausgefallene Produkt wird abgesaugt, in Essigester gelöst, mit Wasser neutral gewaschen, getrocknet und im Vakuum eingeengt. Es werden 7,0 g 11β-Fluor-7α-(5-hydroxypentyl)-3-(tetrahydropyran-2-yloxy)-estra-1,3,5(10)-trien-17-on als Schaum erhalten.

**j) 11β-Fluor-3-(tetrahydropyran-2-yloxy)-7α-(5-p-toluolsulfonyloxypentyl)-estra-1,3,5(10)-trien-17-on**

6,7 g 11β-Fluor-7α-(5-hydroxypentyl)-3-(tetrahydropyran-2-yloxy)-estra-1,3,5(10)-trien-17-on in 70 ml Pyridin werden bei Raumtemperatur mit 6,0 g p-Toluolsulfonsäureanhydrid 3 h gerührt. Die Lösung wird mit Essigester verdünnt, mit gesättiger Natriumchloridlösung gewaschen. getrocknet und im Vakuum eingeengt. Das Rohprodukt wird an Kieselgel mit einem Hexan-Essigester-Gradienten chromatographiert. Man erhält 5,7 g 11β-Fluor-3-(tetrahydropyran-2-yloxy)-7α-(5-p-toluolsulfonyloxy-pentyl)-estra-1,3,5(10)-trien-17-on als Schaum.

**k) 11β-Fluor-7α-{5-[N-methyl-N-3-(4,4,5,5,5-pentafluorpentylthio)-propylamino]-pentyl}-3-(tetrahydrapyran-2-yloxy)-estra-1,3,5(10)-trien-17-on**

2,0 g 11β-Fluor-3-(tetrahydropyran-2-yloxy)-7α-(5-p-toluolsulfonyloxypentyl)-estra-1,3,5(10)-trien-17-on in 44 ml DMF werden bei 80°C mit 1,2 g Methyl-[3-(4,4,5,5,5-pentafluorpentyl-thio)-propyl]-amin gerührt. Nach 6,5 h versetzt man das Reaktionsgemisch mit Wasser. Man extrahiert mit Essigester, wäscht mit gesättigter Natriumchloridlösung und engt im Vakuum ein. Nach Chromatographieren des Rohproduktes an Kieselgel mit einem Methylenchlorid-Methanol-Gradienten werden1,3 g 11β-Fluor-7α-{5-[N-methyl-N-3-(4,4,5,5,5-pentafluorpentylthio)-propylamino]-pentyl}-3-(tetrahydrapyran-2-yloxy)-estra-1,3,5(10)-trien-17-on als Öl erhalten.

**l) 11β-Fluor-7α-{5-[N-methyl-N-3-(4,4,5,5,5-pentafluorpentylthio)-propylamino]-pentyl}-3-(tetrahydrapyran-2-yloxy)-estra-1,3,5(10)-trien-17β-ol**

Zu 2,0 g 11β-Fluor-7α-{5-[N-methyl-N-3-(4,4,5,5,5-pentafluor-pentylthio)-propylamino]pentyl}-3-(tetrahydrapyran-2-yloxy)-estra-1,3,5(10)-trien-17-on in 17 ml THF, 10 ml Ethanol und 4,2 ml Wasser gibt man bei 0°C portionsweise 350 mg Natriumborhydrid. Nach 30 min rührt man das Reaktions-gemisch in Eis-Wasser ein, extrahiert mit Essigester, wäscht mit gesättigter Natriumchloridlösung und engt im Vakuum ein. Man erhält 1,7 g rohes 11β-Fluor-7α-{5-[N-methyl-N-3-(4,4,5,5,5-pentafluorpentylthio)-propylamino]-pentyl}-3-(tetrahydrapyran-2-yloxy)-estra-1,3,5(10)-trien-17β-ol als Schaum.

**m) 11β-Fluor-7α-{5-[N-methyl-N-3-(4,4,5,5,5-pentafluorpentylthio)-propylamino]-pentyl}-estra-1,3,5(10)-trien-3,17β-diol**

Eine Lösung von 1,6 g 11β-Fluor-7α-{5-[N-methyl-N-3-(4,4,5,5,5-pentafluorpentylthio)-propylamino]-pentyl}-3-(tetrahydrapyran-2-yloxy)-estra-1,3,5(10)-trien-17β-ol in 20 ml Methanol und 2 ml Wasser wird mit 1,0 g Oxalsäure gerührt. Nach 3 h wird das Reaktionsgemisch in Eis-Wasser gegeben. Man extrahiert mit Methylenchlorid, wäscht mit gesättigter Natriumchloridlösung, trocknet und engt im Vakuum ein. Nach Chromatographieren des Rohproduktes an Kieselgel mit einem Methylenchlorid-Methanol-Gradienten werden 1,1g 11β-Fluor-7α-{5-[N-methyl-N-3-(4,4,5,5,5-pentafluorpentylthio)-propylamino]-pentyl}-estra-1,3,5(10)-trien-3,17β-diol vom Schmelzpunkt 95°C erhalten.

Beispiel 13

**11β-Fluor-7α-{5-[N-methyl-N-3-(4,4,5,5,5-pentafluorpentansulfinyl)-propylamino]-pentyl}-estra-1,3,5(10)-trien-3,17β-diol**

580 mg 11β-Fluor-7α-{5-[N-methyl-N-3-(4,4,5,5,5-pentafluorpentylthio)-propylamino]-pentyl}-estra-1,3,5(10)-trien-3,17β-diol in 24 ml Methanol und 1,1 ml Wasser werden bei Raumtemperatur mit 350 mg Natriumperiodat gerührt. Nach 1,5 h wird mit Methylenchlorid verdünnt, mit gesättigter Natriumchloridlösung gewaschen, getrocknet und im Vakuum eingeengt. Das Rohprodukt wird an Kieselgel mit einem Methylenchlorid-Methanol-Gradienten chromatographiert. Es werden 287 mg 11β-Fluor-7α-{5-[N-methyl-N-3-(4,4,5,5,5-pentafluorpentansulfinyl)-propylamino]-pentyl}-estra-1,3,5(10)-trien-3,17β-diol vom Schmelzpunkt 87°C erhalten.

Beispiel 14

**11β-Fluor-7α-{5-[N-methyl-N-3-(4,4,5,5,5-pentafluorpentansulfonyl)-propylamino]-pentyl}-estra-1,3,5(10)-trien-3,17β-diol**

**a) 11β-Fluor-7α-{5-[N-methyl-N-3-(4,4,5,5,5-pentafluorpentansulfonyl)-propylamino]-pentyl}-3-(tetrahydrapyran-2-yloxy)-estra-1,3,5(10)-trien-17-on**

2,0 g 11β-Fluor-3-(tetrahydropyran-2-yloxy)-7α-(5-p-toluolsulfonyloxypentyl)-estra-1,3,5(10)-trien-17-on in 40 ml DMF werden bei 80°C mit 2,1g Methyl-[3-(4,4,5,5,5-pentafluor-pentansulfonyl)-propyl]-amin gerührt. Nach 7 h gibt man das Reaktionsgemisch in Eis-Wasser, extrahiert mit Essigester, wäscht mit gesättigter Natriumchloridlösung, trocknet und engt im Vakuum ein. Nach Chromatographieren des Rohproduktes an Kieselgel mit einem Methylenchlorid-Methanol-Gradienten werden 1,1 g 11β-Fluor-7α-{5-[N-methyl-N-3-(4,4,5,5,5-pentafluorpentansulfonyl)-propylamino]-penty}-3-(tetrahydrapyran-2-yloxy)-estra-1,3,5(10)-trien-17-on als Öl erhalten.

**b) 11β-Fluor-7α-{5-[N-methyl-N-3-(4,4,5,5,5-pentafluorpentansulfonyl)-propylamino]-pentyl}-3-(tetrahydrapyran-2-yloxy)-estra-1,3,5(10)-trien-17β-ol**

1,5g11β-Fluor-7α-{5-[N-methyl-N-3-(4,4,5,5,5-pentafluorpentansulfonyl)-propylamino]-pentyl}-3-(tetrahydrapyran-2-yloxy)-estra-1,3,5(10)-trien-17-on werden bei 0°C in einem Gemisch aus 13 ml THF, 7,5 ml Ethanol und 3,2 ml Wasser portionsweise mit 270 mg Natriumborhydrid versetzt. Nach 90 min gibt man Wasser zu, extrahiert mit Essigester, wäscht mit gesättigter Natriumchloridlösung, trocknet und engt im Vakuum ein. Es werden 1,5 g 11β-Fluor-7α-{5-[N-methyl-N-3-(4,4,5,5,5-pentafluorpentansulfonyl)-propylamino]-pentyl}-3-(tetrahydrapyran-2-yloxy)-estra-1,3,5(10)-trien-17β-ol als Rohprodukt erhalten.

**c) 11β-Fluor-7α-{5-[N-methyl-N-3-(4,4,5,5,5-pentafluorpentansulfonyl)-propylamino]-pentyl}-estra-1,3,5(10)-trien-3,17β-diol**

1,4g11β-Fluor-7α-{5-[N-methyl-N-3-(4,4,5,5,5-pentafluorpentansulfonyl)-propylamino]-pentyl}-3-(tetrahydrapyran-2-yloxy)-estra-1,3,5(10)-trien-17β-ol werden in 18 ml Methanol und 1,8 ml Wasser bei Raumtemperatur mit 900 mg Oxalsäure gerührt. Nach 4 h wird das Reaktionsgemisch in Eis-Wasser eingerühert. Das ausgefallene Produkt wird in Methylenchlorid aufgenommen, mit Wasser gewaschen, getrocknet und im Vakuum eingeengt. Nach Chromatographieren des Rohproduktes an Kieselgel mit einem Methylenchlorid-Methanol-Gradienten werden 325 mg 11β-Fluor-7α-{5-[N-methyl-N-3-(4,4,5,5,5-pentafluorpentansulfonyl)-propylamino]-pentyl}-estra-1,3,5(10)-trien-3,17β-diol vom Schmelzpunkt 70°C erhalten.

Beispiel 15

**16α-Fluor-17α-methyl-7α-{5-[N-methyl-N-3-(4,4,5,5,5-pentafluorpentylthio)-propylamino]-pentyl}-estra-1,3,5 (10)-trien-3,17β-diol**

**a) 7α-(5-Chlorpentyl)-estr-4-en-3,17-dion**

In 150 ml wasserfreiem Tetrahydrofuran suspendiert man 11,6 g Magnesiumspäne und bereitet mit 58,8 ml 1-Brom-5-chlor-pentan in 40 ml wasserfreiem Tetrahydrofuran das Grignardreagenz. Man rührt 30 Minuten bei Raumtemperatur, verdünnt mit weiteren 100 ml wasserfreiem Tetrahydrofuran und kühlt auf -70 °C ab. Dann tropft man die Lösung von 3,04 g Kupfer-I-bromid-dimethylsulfid-Komplex in 72 ml 1,3-Dimethyltetrahydro-2(1H)-pyrimidinon (DMPU) und 150 ml Tetrahydrofuran bei -65 bis -70 °C Innentemperatur zu. Schließlich wird innerhalb von 2,5 Stunden die Lösung von 50 g Estra-4,6-dien-3,17-dion (*Steroids* Vol. 1, **1963**, 223) und 75 ml Trimethylchlorsilan in 700 ml wasserfreiem Tetrahydrofuran zugegeben. Die Mischung läßt man im Kühlbad unter Rühren langsam auf Raumtemperatur erwärmen, säuert am nächsten Morgen mit 48 ml Essigsäure unter Eiskühlung an und versetzt nach 15 Minuten rühren mit Essigester. Die organische Phase wird dreimal mit gesättigter Ammoniumchloridlösung, die wäßrige Phase mit Essigester extrahiert, darauf die vereinigten organischen Phasen mit Natriumbicarbo-natlösung gewaschen, mit Magnesiumsulfat getrocknet und im Vakuum eingedampft. Der Rückstand von 53 g wird an Kieselgel mit Hexan/Dichlormethan und Essigester chromatogra-phiert, Ausbeute 35 g 7α-(5-Chlorpentyl)-estr-4-en-3,17-dion

Das gleiche Produkt erhält man aus 8,05 g 7α-(5-Hydroxypentyl)-estr-4-en-3,17-dion (Beispiel 1b) durch 3,5-stündige Reaktion mit 8,66 g Triphenylphosphin in 85 ml Tetrachlorkohlenstoff und 30 ml Acetonitril bei Raumtemperatur. Die Reaktionsmischung wird mit Dichlormethan verdünnt, mit gesättigter Bicarbonatlösung und mit gesättigter Kochsalzlösung ausgeschüttelt, mit Natriumsulfat getrocknet und im Vakuum eingedampft. Der Rückstand wird an Kieselgel mit Dichlormethan und Essigester chromatographiert, wobei man 4,08 g 7α-(5-Chlorpentyl)-estr-4-en-3,17-dion erhält, $[\alpha]_D^{22}$ = +68 ° (c=0,5 % in Chloroform)

**b) 7α-(5-Chlorpentyl)-3-hydroxy-estra-1,3,5(10)-trien-17-on**

18,96 g 7α-(5-Chlorpentyl)-estr-4-en-3,17-dion werden in 350 ml wasserfreiem Acetonitril gelöst und unter Inertgas bei 80 °C mit einer Lösung von 4,36 g Lithiumbromid und 22,5 g Kupfer-II-bromid in 390 ml wasserfreiem Acetonitril versetzt. Man rührt noch 5 Minuten nach, kühlt die Mischung im Eisbad ab und versetzt mit Wasser und Essigester. Die organische Phase wird mit gesättigter Natriumbicarbonatlösung, die wäßrige Phase mit Essigester, die vereinigten organischen Phasen mit gesättigter Kochsalzlösung ausgeschüttelt, mit Natriumsulfat getrocknet und im Vakuum eingedampft. Die Rohausbeute von 19,1 g wird an Kieselgel in Hexan und Essigester chromatographiert, wobei man 10,0 g öliges 7α-(5-Chlorpentyl)-3-hydroxy-estra-1,3,5(10)-trien-17-on erhält, das aus Hexan/Dichlormethan kristallisiert werden kann.
Schmelzpunkt 143,5 °C, $[\alpha]_D^{22}$ = +112 ° (c=0,5 % in Chloroform)

**c) Bis-3,17-trimethylsilyloxy-7α-(5-chlorpentyl)-estra-1,3,5(10),16-tetraen**

In 75 ml Benzol löst man 5,65 g 7α-{5-Chlorpentyl)-estra-1,3,5(10)-trien-3-ol-17-on, 9 ml Triethylamin und 9 ml Trifluormethansulfonsäuretrimethylsilylester und erhitzt 2 Stunden unter Rückfluß. Nach Abkühlen wird mit Hexan verdünnt, die obere Phase mit gesättigter Natriumbicarbonatlösung und mit gesättigter Kochsalzlösung ausgeschüttelt, mit Natriumsulfat getrocknet und im Vakuum eingedampft und liefert Bis-3,17-trimethylsilyloxy-7α-(5-chlorpentyl)-estra-1,3,5(10),16-tetraen als Öl.

**d) 7α-(5-Chlorpentyl)-16α-fluor-estra-1,3,5(10)-trien-3-ol-17-on**

Das Produkt des vorstehenden Versuches wird in 150 ml wasserfreiem Dichlormethan gelöst und mit 14,2 g N-Fluorbenzolsulfonimid versetzt. Nach 2 Stunden Rühren bei Raumtemperatur gibt man 50 ml 8%ige Schwefelsäure zu, rührt intensiv weitere 3 Stunden und trennt dann die Phasen. Die Wasseiphase wird zweimal mit Dichlormethan, die organische Phase nacheinander mit gesättigter Natriumbicarbonat- und Natriumchloridlösung ausgeschüttelt, mit Natriumsulfat getrocknet und im Vakuum eingedampft. Das Rohprodukt von 15,3 g wird an Kieselgel mit Dichlormethan/Diisopropylether chromatographiert, wobei man 2,92 g 7α-(5-Chlorpentyl)-16α-fluor-estra-1,3,5(10)-trien-3-ol-17-on als Öl erhält. Die Substanz läßt sich aus Diisopropylether kristallisieren, Schmelzpunkt 176 °C, $[\alpha]_D^{22}$ = +114 ° (c=0,5 % in Chloroform)

**e) 7α-(5-Chlorpentyl)-16α-fluor-17α-methyl-estra-1,3,5(10)-tries-3,17β-diol**

3,0 g 7α-(5-Chlorpentyl)-16α-fluor-estra-1,3,5(10)-trien-3-ol-17-on werden in 300 ml wasserfreiem Toluol unter Inertgas gelöst und mit 3 Portionen von je 20 ml einer 1,5 molaren Lösung von Methyllithium und Lithiumbromid in Ether bei Raumtemperatur im Abstand von 15 Minuten versetzt. Nach weiteren 30 Minuten wird die Reaktionsmischung in eine halbgesättigte Ammoniumchloridlösung unter Eiskühlung eingerührt, mit 8%iger Schwefelsäure angesäuert und mit Essigester extrahiert. Die vereinigten organischen Phasen werden mit Natriumchloridlösung ausgeschüttelt, mit Natriumsulfat getrocknet und im Vakuum eingedampft. Das Rohprodukt von 3,2 g wird an Kieselgel mit Hexan und Methyl-*tert*.-butylether chromatographiert. Die polare Fraktion von 1,21 g ist 7α-(5-Chlorpentyl)-16α -fluor-17α-methyl-estra-1,3,5(10)-trien-3,17β-diol, das aus Diisopropylether/Hexan kristallisiert, Schmelzpunkt 70 °C, $[\alpha]_D^{22}$ = +7 ° (c=0,5 % in Chloroform)

**f) 16α-Fluor-17α-methyl-7α-{5-[N-methyl-N-3-(4,4,5,5,5-pentafluorpentylthio)-propylamino]-pentyl}-estra-1,3,5 (10)-trien-3,17β-diol**

Man löst 40,8 mg 7α-(5-Chlorpentyl)-16α-fluor-17α-methyl-estra-1,3,5(10)-trien-3,17β-diol in 0,5 ml Dimethylformamid und gibt die Lösung von 58 mg 3-(N-Methylamino)-propyl-4,4,5,5,5-pentafluorpentyl-sulfid und 13 mg Lithiumiodid zu und erhitzt 17 Stunden auf 100 °C unter Inertgas. Nach dem Abkühlen versetzt man die Mischung mit Essigester, schüttelt nacheinander mit gesättigter Natriumhydrogencarbonat- und Kochsalzlösung aus, trocknet mit Natriumsulfat und dampft das Lösungsmittel im Vakuum ab. Der Rückstand wird an Kieselgel mit Essigester/Methanol chromatographiert, Ausbeute 25 mg 16α-Fluor-17α-methyl-7α-{5-[N-methyl-N-3-(4,4,5,5,5-pentafluorpentylthio)-propylamino]-pentyl}-estra-1,3,5(10)-trien-3,17β-diol als Öl.

Beispiel 16

**16α-Fluor-17β-methyl-7α-{5-[N-methyl-N-3-(4,4,5,5,5-pentafluorpentylthio)-propylamino]-pentyl}-estra-1,3,5 (10)-trien-3,17α-diol**

Als unpolares Produkt der Chromatographie im Beispiel 15e) fällt 7α-(5-Chlorpentyl)-16α-fluor-17β-methyl-estra-1,3,5(10)-trien-3,17α-diol in einer Menge von 0,48 g an. Durch Kristallisation aus Diisopropylether/Hexan erhält man Kristalle mit dem Schmelzpunkt 65 °C und $[\alpha]_D^{22}$ = +5 ° (c=0,5 % in Chloroform).

Davon werden 41 mg wie im Beispiel 15f) beschrieben umgesetzt, wobei man 23 mg 16α-Fluor-17β-methyl-7α-{5-[N-methyl-N-3-(4,4,5,5,5-pentafluorpentylthio)-propylamino]-pentyl}-estra-1,3,5(10)-trien-3,17α-diol erhält.

Beispiel 17

**16α-Fluor-17α-methyl-7α-{5-[N-methyl-N-3-(4,4,5,5,5-pentafluorpentansulfinyl)-propylamino]-pentyl}-estra-1,3,5(10)-trien-3,17β-diol**

Durch Oxidation mit Natriumperiodat erhält man, wie im Beispiel 2 beschrieben, aus 72 mg 16α-Fluor-17α-methyl-7α-{5-[N-methyl-N-3-(4,4,5,5,5-pentafluorpentylthio)-propylamino]-pentyl)-estra-1,3,5(10)-trien-3,17β-diol, 38 mg 16α-Fluor-17α-methyl-7α-{5-[N-methyl-N-3-(4,4,5,5,5-pentafluorpentansulfinly)-propylamino]-pentyl}-estra-1,3,5 (10)-trien-3,17β-diol.

Beispiel 18

**16α-Fluor-17α-methyl-7α-{5-[N-methyl-N-3-(4,4,5,5,5-pentafluorpentansulfonyl)-propylamino]-pentyl}-estra-1,3,5(10)-trien-3,17β-diol**

Wie im Beispiel 15f) beschrieben werden 40,8 mg 7α-(5-Chlorpentyl)-16α-fluor-17α-methyl-estra-1,3,5(10)-trien-3,17β-diol in Dimethylformamid mit 70 mg 3-(N-Methylamino)-propyl-4,4,5,5,5,-pentafluorpentylsulfon umgesetzt. Nach Aufarbeitung erhält man 21 mg 16α-Fluor-17α-methyl-7α-{5-[N-methyl-N-3-(4,4,5,5,5-pentafluorpentansulfonly)-propylamino]-pentyl}-estra-1,3,5(10)-trien-3,17β-diol als Öl.

Beispiel 19

**16α-Fluor-7α-{5-[N-methyl-N-3-(4,4,5,5,5-pentafluorpentylthio)-propylamino]-pentyl}-estra-1,3,5(10)-trien-3,17β-diol**

**a) 7α-(5-Chlorpentyl)-16α-fluor-estra-1,3,5(10)-trien-3,17β-diol und 7α-(5-Chlorpentyl)-16α-fluor -estra-1,3,5 (10)-trien-3,17α-diol**

Man löst 392 mg 7α-(5-Chlorpentyl)-16α-fluor-estra-1,3,5(10)-trien-3-ol-17-on, Beispiel 15d), in 2 ml wasserfreiem Tetrahydrofuran und gibt 1,1 ml einer 1 molaren Lösung von Lithiumtri-*tert*.-butoxyaluminiumhydrid in Tetrahydrofuran unter Kühlung im Eisbad zu. Man läßt 30 Minuten bei 0 °C rühren, versetzt dann mit Wasser und 8%iger Schwefelsäure bis zur schwach sauren Reaktion und extrahiert dreimal mit Essigester. Die organische Phase wird mit gesättigter Natirumchloridlösung gewaschen, mit Natriumsulfat getrocknet und im Vakuum zur Trockene eingedampft. Das Rohprodukt wird an Kieselgel mit Hexan/Essigester chromatographiert, wobei man 118 mg 7α-(5-Chlorpentyl)-16α-fluor-estra-1,3,5(10)-trien-3,17β-diol und 138 mg 7α-(5-Chlorpentyl)-16α-fluor-estra-1,3,5(10)-trien-3,17α-diol erhält, beides als fester Schaum.

**b) 16α-Fluor-7α-{5-[N-methyl-N-3-(4,4,5,5,5-pentafluorpentylthio)-propylamino]-pentyl}-estra-1,3,5(10)-trien-3,17β-diol**

Durch Umsetzung von 100 mg 7α-(5-Chlorpentyl)-16α-fluor-estra-1,3,5(10)-trien-3,17β-diol mit 3-(N-Methylamino)-propyl-4,4,5,5,5-pentafluorpentylsulfid, wie im Beispiel 15f) beschrieben, erhält man 83 mg 16α-fluor-7α-{5-[N-methyl-N-3-(4,4,5,5,5-pentafluorpentylthio)-propylamino]-pentyl}-estra-1,3,5(10)-trien-3,17β-diol als festen Schaum.

Beispiel 20

**16α-Fluor-7α-{5-[N-methyl-N-3-(4,4,5,5,5-pentafluorpentylthio)-propylamino]-pentyl}-estra-1,3,5(10)-trien-3,17α-diol**

Analog erhält man aus 100 mg 7α-(5-Chlorpentyl)-16α-fluor-estra-1,3,5(10)-trien-3,17α-diol in gleicher Weise 75 mg 16α-Fluor-7α-{5-[N-methyl-N-3-(4,4,5,5,5-pentafluorpentylthio)-propyl-amino]-pentyl}-estra-1,3,5(10)-trien-3,17α-diol als Schaum.

Beispiel 21

**16α-Fluor-7α-{5-[N-methyl-N-3-(4,4,5,5,5-pentafluorpentansulfinly)-propylamino]-pentyl}-estra-1,3,5(10)-trien-3,17β-diol**

Durch Oxidation von 85 mg 16α-Fluor-7α-{5-[N-methyl-N-3-(4,4,5,5,5-pentafluorpentylthio)-propylamino]-pentyl}-estra-1,3,5(10)-trien-3,17β-diol mit Natriumperiodat, wie im Beispiel 17 beschrieben, erhält man 47 mg 16α-Fluor-7α-{5-[N-methyl-N-3-(4,4,5,5,5-pentafluorpentyl-sulfinyl)-propylamino]-pentyl}-estra-1,3,5(10)-trien-3,17β-diol als Schaum.

Beispiel 22

**16α-Fluor-7α-{5-[N-methyl-N-3-(4,4,5,5,5-pentafluorpentansulfonly)-propylamino]-pentyl}-estra-1,3,5(10)-trien-3,17β-diol**

Wie im Beispiel 15f) beschrieben setzt man 65 mg 7α-5-(Chlorpentyl)-16α-fluor-estra-1,3,5(10)-trien-3,17β-diol mit 90 mg 3-(N-Methylamino)-propyl-4,4,5,5,5-pentafluorpentylsulfon um und erhält 53 mg 16α-Fluor-7α-{5-[N-methyl-N-3-(4,4,5,5,5-pentafluorpentansulfonly)-propylamino]-pentyl}-estra-1,3,5(10)-trien-3,17β-diol als Öl.

Beispiel 23

**7α-{5-[N-Methyl-N-3-(4,4,5,5,5-pentafluorpentylthio)-propylamino]-pentyl}-estra-1,3,5(10),14-tetraen-3,17β-diol**

**a) 7α-(5-*tert.*-Butyl-dimethylsilyloxypentyl)-estr-4-en-3,17-dion**

In 34 ml absolutem Tetrahydrofuran werden 8,9 g Magnesiumspäne mit 103 g 1-Brom-5-*tert*.-butyldimethylsilyloxypentan, gelöst in 110 ml Tetrahydrofuran, zum Grignardreagenz umgesetzt. Zu dieser Lösung werden bei -70 °C bis -65 °C 2,5 g Kupfer-I-bromid-Dimethylsulfid-Komplex und eine Mischung aus 60 ml 1,3-Dnnethyl-3,4,5,6-tetrahydro-2(1H)-pyrimidinon und 70 ml Tetrahydrofuran gegeben und die erhaltene Suspension noch 30 Minuten bei -70 °C unter Argonatmosphäre gerührt.

Anschließend wird bei -70 °C bis -65 °C eine Lösung von 50 g Estra-4,6-dien-3,17-dion [Steroids Vol. 1, 1963, 233-249] in 730 ml absolutem Tetrahydrofuran und 62,7 ml Chlortrimethylsilan zugetropft, und die Mischung über Nacht bei -70 °C gerührt. Zur Aufarbeitung wird die Reaktionsmischung bei -15 °C mit 48 ml Eisessig versetzt und nach 15-minütigem Rühren bei dieser Temperatur in eine Mischung aus gesättigter Ammoniumchloridlösung und Ethylacetat gegossen. Die organische Phase wird abgetrennt und nacheinander mit gesättigter Ammoniumchloridlösung, gesättigter Natriumhydrogencarbonatlösung und schließlich mit gesättigter Kochsalzlösung neutral gewaschen, über Natriumsulfat getrocknet und eingeengt.

Der Rückstand wird an Kieselgel mit Dichlormethan/Aceton chromatographiert. Es werden 47 g 7α-(5-*tert.* -Butyl-dimethylsilyloxypentyl)-estr-4-en-3,17-dion als gelbes Öl erhalten. $[\alpha]_D^{22}$ = +62,2 ° (c=0,545 in Chloroform).

**b) 3-Hydroxy-7α-(5-hydroxypentyl)-estra-1,3,5(10),15-tetraen-17-on**

Nach dem im Beispiel 1b-1i angegebenen Verfahrensweisen werden 62,7 g 7α-(5-*tert*.-Butyldimethylsilyloxypentyl)-estr-4-en-3,17-dion zu 15,8 g 3-Hydroxy-7α-(5-hydroxypentyl)-estra-1,3,5 (10),15-tetraen-17-on umgesetzt.

**c) 3-Benzyloxy-7α-(5-hydroxypentyl)-estra-1,3,5(10),15-tetraen-17-on**

Eine Lösung von 2,85 g 3-Hydroxy-7α-(5-hydroxypentyl)-estra-1,3,5(10),15-tetraen-17-on in 57 ml Aceton wird mit 3,25 g Cäsiumcarbonat und 1,14 ml Benzylbromid versetzt und die Mischung 1 Stunde unter Rückfluß erhitzt. Die Reaktionsmischung wird eingeengt, der Rückstand mit Wasser versetzt, mit Ethylacetat ausgeschüttelt, die organische Phase über Natriumsulfat getrocknet und eingeengt. Der Rückstand wird an Kieselgel mit Hexan/Ethylacetat chromatographiert. Es werden 3,12 g 3-Benzyloxy-7α-(5-hydroxypentyl)-estra-1,3,5(10),15-tetraen-17-on als Schaum erhalten.

**d) 17-Acetoxy-7α -(5-acetoxypentyl)-3-benzyloxy-estra-1,3,5(10),14,16-pentaen**

Eine Lösung von 6,12 g 3-Benzyloxy-7α-(5-hydroxypentyl)-estra-1,3,5(10),15-tetraen-17-on in 717 ml Acetanhydrid wird mit 920 mg *p*-Toluolsulfonsäure 1 Stunde bei Raumtemperatur gerührt. Die Reaktionsmischung wird nach der im Beispiel 1j angegebenen Verfahrensweise aufgearbeitet und das Rohprodukt an Kieselgel mit Hexan/Ethylacetat chromatographiert. Es werden 4,6 g 17-Acetoxy-7α-(5-acetoxypentyl)-3-benzyloxy-estra-1,3,5(10),14,16-pentaen als Öl erhalten.

**e) 7α-(5-Acetoxypentyl)-3-benzyloxy-estra-1,3,5(10),14-tetraen-17β-ol**

Zu einer Lösung von 4,58 g 17-Acetoxy-7α-(5-acetoxypentyl)-3-benzyloxy-estra-1,3,5(10),14,16-pentaen in 26,8 ml Tetrahydrofuran und 161 ml Ethanol wird bei Raumtemperatur eine Lösung von 1,25 g Natriumborhydrid in 90 ml Ethanol und 18 ml Wasser getropft und die Mischung 1 Stunde gerührt. Die Reaktionsmischung wird mit 4 ml Eisessig versetzt, eingeengt und der Rückstand in Ethylacetat aufgenommen. Die organische Phase wird mit Natriumhydrogen-carbonat gewaschen, über Natriumsulfat getrocknet und eingeengt. Der Rückstand wird an Kieselgel mit Hexan/Ethylacetat chromatographiert. Es werden 2,16 g 7α-(5-Acetoxypentyl)-3-benzyloxy-estra-1,3,5(10),14-tetraen-17β-ol als Schaum erhalten.

**f) 3-Benzyloxy-7α-(5-hydroxypentyl)-estra-1,3,5(10),14-tetraen-17β-ol**

2,16 g 7α-(5-Acetoxypentyl)-3-benzyloxy-estra-1,3,5(10),14-tetraen-17β-ol werden mit 38 ml 1N methanolischer Kaliumhydroxidlösung über Nacht bei Raumtemperatur verseift. Die Reaktionsmischung wird in eiskalte gesättigte Kochsalzlösung gegossen, der Niederschlag abgesaugt, in Dichlormethan aufgenommen, mit gesättigter Kochsalzlösung gewaschen, über Natriumsulfat getrocknet und eingeengt. Es werden 1,86 g 3-Benzyloxy-7α-(5-hydroxypentyl)-estra-1,3,5(10),14-tetraen-17β-ol als Schaum erhalten.

**g) 3-Benzyloxy-7α-(5-tosyloxypentyl)-estra-1,3,5(10),14-tetraen-17β-ol**

Unter den Bedingungen des Beispiels 1o werden 3,03 g 3-Benzyloxy-7α-(5-hydroxypentyl)-estra-1,3,5(10),14-tetraen-17β-ol mit 2,31 g *p*-Toluolsulfonsäureanhydrid in 58 ml Pyridin umgesetzt, aufbereitet und an Kieselgel chromatographiert. Es werden 3 g 3-Benzyloxy-7α-(5-tosyloxy-pentyl)-estra-1,3,5(10),14-tetraen-17β-ol als Schaum erhalten.

**h) 3-Benzyloxy-7α-{5-[N-methyl-N-3-(4,4,5,5,5-pentafluorpentylthio)-propylamino]-pentyl}-estra-1,3,5(10),14-tetraen-17β-ol**

Eine Lösung von 2 g 3-Benzyloxy-7α-(5-tosyloxypentyl)-estra-1,3,5(10),14-tetraen-17β-ol in 28 ml Ethylmethylketon wird mit 1,77 g N-Methyl-3-(4,4,5,5,5-pentafluorpentylthio)-propylamin in Gegenwart von 950 mg Kaliumcarbonat und 230 mg Kaliumiodid 5 Stunden bei 80 °C Badtemperatur gerührt. Die Reaktionsmischung wird in Kochsalzlösung gegossen, mit Ethylacetat extrahiert, mit Kochsalzlösung gewaschen, über Natriumsulfat getrocknet und eingeengt. Der Rückstand wird an Kieselgel mit Hexan/Ethylacetat chromatographiert. Es werden 1,93 g 3-Benzyloxy-7α-{5-[N-methyl-N-3-(4,4,5,5,5-pentafluorpentylthio)-propylamino]-pentyl}-estra-1,3,5(10),14-tetraen-17β-ol als Harz erhalten. $[\alpha]_D^{22}$ = +47,3 ° (c=0,505 in Chloroform).

**i) 7α-{5-[N-Methyl-N-3-(4,4,5,5,5-pentafluorpentylthio)-propylamino]-pentyl}-estra-1,3,5(10),14-tetraen-3,17β-diol**

Zu einer Lösung von 850 mg 3-Benzyloxy-7α-{5-[N-methyl-N-3-(4,4,5,5,5-pentafluorpentylthio)-propylamino]-pentyl}-estra-1,3,5(10),14-tetraen-17β-ol in 10,2 ml Toluol werden bei Raum-temperatur 10,2 ml einer 1,2 molaren Lösung von Diisobutylaluminiumhydrid in Toluol getropft und die Mischung 5 Stunden bei 120 °C Badtemperatur erhitzt. Die Reaktionsmischung wird unter Rühren und Argonatmosphäre in eine Mischung aus gesättigter Kochsalzlösung und 2 N Schwefelsäure getropft, 3 mal mit Ethylacetat extrahiert, die organsiche Phase 2 mal mit 2 N Schwefelsäure und 3 mal mit gesättigter Kochsalzlösung gewaschen, über Natriumsulfat getrocknet und eingeengt. Der Rückstand wird an Kieselgel mit Hexan/ Ethylacetat/0-30 % Methanol chromatographiert. Es werden 670 mg 7α-{5-[N-Methyl-N-3-(4,4,5,5,5-pentafluorpentylthio)-propylamino]-pentyl}-estra-1,3,5(10),14-tetraen-3,17β-diol als Schaum erhalten. $[\alpha]_D^{22}$ = +43 ° (c=0,520 in Chloroform/Methanol).

Beispiel 24

**7α-{5-[N-Methyl-N-3-(4,4,5,5,5-pentafluorpentansulfinyl)-propylamino]-pentyl}-estra-1,3,5(10),14-tetraen-3,17β-diol**

Unter den Bedingungen des Beispiels 2 werden 400 mg 7α-{5-[N-Methyl-N-3-(4,4,5,5,5-pentafluorpentylthio)-propylamino]-pentyl}-estra-1,3,5(10),14-tetraen-3,17β-diol mit 177 mg Natriumperiodat umgesetzt, aufbereitet und das Rohprodukt an Kieselgel mit Ethylacetat/Methanol chromatographiert. Es werden 287 mg der Titelverbindung als Schaum erhalten. $[\alpha]_D^{22}$ = +30,7 ° (c=0,530 in Chloroform / Methanol).

Beispiel 25

**7α-{5-[N-Methyl-N-3-(4,4,5,5,5-pentafluorpentansulfonyl)-propylamino]-pentyl}-estra-1,3,5(10),14-tetraen-3,17β-diol**

**a) 3-Benzyloxy-7-α{5-[N-methyl-N-methyl-N-3-(4,4,5,5,5-pentafluorpentansulfonyl)-propylamino]-pentyl}-estra-1,3,5(10),14-tetraen-17β-ol**

Unter den Bedingungen des Beispiels 23h wird 1 g 3-Benzyloxy-7α-(5-tosyloxypentyl)-estra-1,3,5(10),14-tetraen-17β-ol mit 990 mg N-Methyl-3-(4,4,5,5,5-pentafluorpentansulfonyl)-propyl-amin umgesetzt, aufbereitet und das Rohprodukt an Kieselgel mit Ethylacetat/Methanol chromatographiert. Es werden 960 mg 3-Benzyloxy-7α-{5-[N-methyl-N-3-(4,4,5,5,5-pentafluorpentansulfonyl)-propylamino]-pentyl}-estra-1,3,5(10),14-tetraen-17 β-ol als Öl erhalten. $[\alpha]_D^{22}$ = +48,5 ° (c=0,535 in Chloroform).

**b) 7α-{5-[N-Methyl-N-3-(4,4,5,5,5-pentafluorpentansulfonyl)-propylamino]pentyl}-estra-1,3,5(10),14-tetraen-3,17β-diol**

Eine Lösung von 100 mg 3-Benzyloxy-7α-{5-[N-methyl-N-3-(4,4,5,5,5-pentafluorpentansulfonyl)-propylamino]-pentyl}-estra-1,3,5(10),14-tetraen-17β-ol in 1 ml Dichlormethan wird unter Eisbadkühlung mit 0,2 ml Dimethylanilin und 73 mg Aluminiumchlorid versetzt und 1 Stunde bei dieser Temperatur gerührt. Die Reaktionsmischung wird auf 1 N Salzsäure gegossen, mit Ethylacetat ausgeschüttelt, mit Natriumhydrogencarbonatlösung und gesättigter Kochsalzlösung gewaschen, über Natriumsulfat getrocknet und eingeengt. Das Rohprodukt wird an Kieselgel mit Dichlormethan/ 0-10 % Methanol chromatographiert. Es werden 74 mg der Titelverbindung als Schaum erhalten. $[\alpha]_D^{22}$ = +36 ° (c = 0,525 Chloroform / Methanol).

Beispiel 26

**7α-{5-[(2S)-2-(4,4,5,5,5-Pentafluorpentylthiomethyl)-pyrrolidin-1-yl]-pentyl}-estra-1,3,5(10)-trien-3,17β-diol**

**a) 7α-(5-Acetoxypentyl)-3-benzyloxy-estra-1,3,5(10)-trien-17-on**

Eine Lösung von 6,5 g 7α-(5-Acetoxypentyl)-3-hydroxy-estra-1,3,5(10)-trien-17-on (Beispiel 1d) in 65 ml Dimethylformamid wird mit 3,3 ml Benzylchlorid, 8,7 g Cäsiumcarbonat und 400 mg Natriumiodid versetzt und über Nacht bei Raumtemperatur gerührt. Die Reaktionsmischung wird in Wasser gegossen, mit Ethylacetat ausgeschüttelt, die organische Phase mit gesättigter Kochsalzlösung gewaschen, über Natriumsulfat getrocknet und eingeengt. Der Rückstand wird an Kieselgel mit Hexan/Ethylacetat chromatographiert. Es werden 7,26 g 7α-(5-Acetoxypentyl)-3-benzyloxy-estra-1,3,5(10)-trien-17-on als Öl erhalten.

**b) 3-Benzyloxy-7α-(5-hydroxypentyl)-estra-1,3,5(10)-trien-17-on**

Eine Lösung von 7,26 g α-(5-Acetoxypentyl)-3-benzyloxy-estra-1,3,5(10)-tries-17-on in 80 ml Methanol und 3 ml Tetrahydrofuran wird mit 22,2 ml 2 N Natronlauge über Nacht bei Raumtemperatur verseift. Die Reaktionslösung wird in 2 N Salzsäure gegossen, mit Ethylacetat ausgeschüttelt, die organische Phase mit gesättigter Kochsalzlösung gewaschen, über Natriumsulfat getrocknet und eingeengt. Es werden 6,7 g 3-Benzyloxy-7α-(5-hydroxypentyl)-estra-1,3,5(10)-trien-17-on als Schaum erhalten.

**c) 3-Benzyloxy-7α-(5-tosyloxypentyl)-estra-1,3,5(10)-tries-17-on**

Unter den Bedingungen des Beispiels 1o werden 6,5 g 3-Benzyloxy-7α-(5-hydroxypentyl)-estra-1,3,5(10)-trien mit 7,14 g p-Toluolsulfonsäureanhydrid umgesetzt, aufbereitet und an Kieselgel mit Hexan/Ethylacetat chromatographiert. Es werden 7,45 g 3-Benzyloxy-7α-(5-tosyloxypentyl)-estra-1,3,5(10)-trien-17-on als Schaum erhalten. $[\alpha]_D^{22}$ = +80,6 ° (c=0,620 in Chloroform).

**d) 3-Benzyloxy-7α-{5-[(2S)-2-(4,4,5,5,5-pentafluorpentylthiomethyl)-pyrrolidin-1-yl]-pentyl}-estra-1,3,5(10)-trien-17-on**

Unter den Bedingungen des Beispiels 23h werden 4,58 g 3-Benzyloxy-7α-(5-tosyloxypentyl)-estra-1,3,5(10)-trien-17-on mit 3,17 g (2S)-2-(4,4,5,5,5-Pentafluorpentylthiomethyl)-pyrrolidin umgesetzt, aufbereitet und das Rohprodukt an Kieselgel mit Hexan/Ethylacetat chromatographiert. Es werden 3,9 g 3-Benzyloxy-7α-{5-[(2S)-2-(4,4,5,5,5-pentafluorpentylthiomethyl)-pyrrolidin-1-yl]-pentyl}-estra-1,3,5(10)-trien-17-on als Öl erhalten. $[\alpha]_D^{22}$ = +32,5 ° (c=0,117 in Chloroform).

**e) 3-Hydroxy-7α-{5-[(2S)-2-(4,4,5,5,5-pentafluorpentylthiomethyl)-pyrrolidin-1-yl]-pentyl}-estra-1,3,5(10)-trien-17-on**

Unter den Bedingungen des Beispiels 6f werden 2,05 g 3-Benzyloxy-7α-{5-[(2S)-2-(4,4,5,5,5-pentafluorpentylthiomethyl)-pyrrolidin-1-yl]-pentyl}-estra-1,3,5(10)-trien-17 on debenzyliert. Es werden 1,25 g 3-Hydroxy-7α-{5-[(2S)-2-(4,4,5,5,5-pentafluorpentylthiomethyl)-pyrrolidin-1-yl]-pentyl)-estra-1,3,5(10)-trien-17-on als Öl erhalten. $[\alpha]_D^{22}$ = +22,7 ° (c=0,475 in Chloroform).

**f) 7α-{5-[(2S)-2-(4,4,5,5,5-Pentafluorpentylthiomethyl)-pyrrolidin-1-yl]-pentyl}-estra-1,3,5(10)-trien-3,17β-diol**

Unter den Bedingungen des Beispiels 8 werden 500 mg 3-Hydroxy-7α-{5-[(2S)-2-(4,4,5,5,5-pentafluorpentylthiomethyl)-pyrrolidin-1-yl]-pentyl)-estra-1,3,5(10)-trien-17-on mit 100 mg Natriumborhydrid reduziert. Es werden 325 mg der Titelverbindung als Öl erhalten. $[\alpha]_D^{22}$ = -8,7° (c=0,510 in Methanol).

Beispiel 27

**7α-{5-[(2R)-2-(4,4,5,5,5-Pentafluorpentylthiomethyl)-pyrrolidin-1-yl]-pentyl}-estra-1,3,5(10)-trien-3,17β-diol**

In analoger Weise wie im Beispiel 26d-f beschrieben, werden aus 2,3 g 3-Benzyloxy-7α-(5-tosyloxypentyl)-estra-1,3,5(10)-trien-17-on (Beispiel 26c) und 1,6 g (2R)-2-(4,4,5,5,5-Pentafluor-pentylthiomethyl)-pyrrolidin 612 mg der Titelverbindung als Öl erhalten.

Beispiel 28

**17α-Methyl-7α-{5-[(2S)-2-(4,4,5,5,5-pentafluorpentylthiomethyl)-pyrrolidin-1-yl]-pentyl}-estra-1,3,5(10)-trien-3,17β-diol**

**a) 7α-(5-Chlorpentyl)-17α-methyl-estra-1,3,5(10)-trien-3,17β-diol**

Eine Suspension von 5,21 g wasserfreiem Cer-III-chlorid in 53,2 ml Tetrahydrofuran wird 2 Stunden bei Raumtemperatur gerührt, bei 0 °C 7 ml einer Methylmagnesiumbromidlösung (3M in Diethylether) zugetropft und die Mischung 30 Minuten bei 0 °C und 15 Minuten bei Raumtemperatur gerührt. Anschließend wird eine Lösung von 1 g 7α-(5-Chlorpentyl)-3-hydroxy-estra-1,3,5(10)-trien-17-on (Beispiel 15b) in 24 ml Tetrahydrofuran hinzugefügt und weitere 30 Minuten bei Raumtemperatur gerührt. Die Reaktionsmischung wird auf eiskalte gesättigte Ammoniumchloridlösung gegossen, mit Ethylacetat ausgeschüttelt, die organische Phase über Natriumsulfat getrocknet und eingeengt. Der Rückstand wird an Kieselgel mit Hexan/Ethylacetat chromatographiert. Es werden 753 mg 7α-(5-Chlorpentyl)-17α-methyl-estra-1,3,5(10)-trien-3,17β-diol als Schaum erhalten. $[\alpha]_D^{22}$ = +27,3 ° (c=0,515 in Chloroform).

**b) 7α-(5-Iodpentyl)-17α-methyl-estra-1,3,5(10)-trien-3,17β-diol**

Eine Lösung von 735 mg 7α-(5-Chlorpentyl)-17α-methyl-estra-1,3,5(10)-trien-3,17β-diol in 4 ml Ethylmethylketon wird mit 5,6 g Natriumiodid versetzt und 17 Stunden bei 80 °C Badtemperatur erhitzt. Die Reaktionsmischung

wird in Wasser gegossen, mit Ethylacetat ausgeschüttelt, die organische Phase mit gesättigter Kochsalzlösung gewaschen, über Natriumsulfat getrocknet und eingeengt. Es werden 834 mg 7α-(5-Iodpentyl)-17α-methyl-estra-1,3,5(10)-trien-3,17β-diol als Schaum erhalten. $[\alpha]_D^{22}$ =+20,2 ° (c=0,500 in Chloroform).

**c) 17α-Methyl-7α-{5-[(2S)-2-(4,4,5,5,5-pentafluorpentylthiomethyl)-pyrrolidin-1-yl]-pentyl}-estra-1,3,5(10)-trien-3,17β-diol**

Eine Lösung von 453 mg 7α-(5-Iodpentyl)-17α-methyl-estra-1,3,5(10)-trien-3,17β-diol und 390 mg (2S)-2-(4,4,5,5,5-Pentafluorpentylthiomethyl)-pyrrolidin in 8 ml N-Methyl-2-pyrrolidinon wird 4 Stunden auf 80 °C Badtemperatur erhitzt. Die abgekühlte Reaktionsmischung wird in gesättigte Natriumhydrogencarbonatlösung gegossen, mit Ethylacetat ausgeschüttelt, die organische Phase mit gesättigter Kochsalzlösung gewaschen, über Natriumsulfat getrocknet und eingeengt. Der Rückstand wird an Kieselgel mit Dichlormethan/Ethylacetat chromatographiert. Es werden 413 mg der Titelverbindung als Schaum erhalten. $[\alpha]_D^{22}$ = -25,8 ° (c=0,500 in Chloroform).

Beispiel 29

**11β-Fluor-7α-{5-[2-(4,4,5,5,5-pentafluorpentylthiomethyl)-pyrrolidin-1-yl]-pentyl}-estra-1,3,5(10)-trien-3,17β-diol**

**a) 7α-(5-Chlorpentyl)-11β-fluor-estr-4-en-3,17-dion**

Eine Loesung von 78,7 g 11β-Fluor-7α-(5-hydroxypentyl)-estr-4-en-3,17-dion (Beispiel 12e) in 1,4l Tetrachlorkohlenstoff und 475 ml Acetonitril wird mit 71 g Triphenylphosphin 8,5 Stunden bei Raumtemperatur gerührt. Dann wird mit Wasser, wässriger Natriumhydrogencarbonat- und Kochsalzlösung extrahiert, über Natriumsulfat getrocknet, i.Vak. eingeengt und an Kieselgel mit Hexan / Essigester chromatographiert. Man erhält 48,0 g 7α-(5-Chlorpentyl)-11β-fluor-estr-4-en-3,17-dion als Kristalle vom Schmelzpunkt 51-53 °C. $[\alpha]_D^{22}$ = +78,5° (c = 0.5% in Chloroform).

**b) 7α-(5-Chlorpentyl)-11β-fluor-3-hydroxy-estra-1,3,5(10)-trien-17-on**

Zu einer Lösung von 47g 7α-(5-Chlorpentyl)-1β-fluor-estr-4-en-3,17-dion in 470 ml Acetonitril wird bei 80 °C Badtemperatur eine Lösung von 10,34 g Lithiumbromid und 53,2 g Kupfer(II)bromid in 280 ml Acetonitril zugetropft. Nach einer Zugabe von 20 ml wartet man auf die Entfärbung der Lösung und gibt den Rest der Lösung in 12 Minuten zu und rührt anschließend noch 5 Minuten bei 80 °C Badtemperatur. Dann wird auf 0 °C abgekühlt, mit Natriumhydrogencarbonatlösung versetzt, auf Wasser gegeben, 4 mal mit Essigester extrahiert, neutralgewaschen, über Natriumsulfat getrocknet, i.Vak. eingeengt und an Kieselgel mit Hexan / Essigester chromatographiert. Man erhält 25,3 g 7α-(5-Chlorpentyl)-11β-fluor-3-hydroxy-estra-1,3,5(10)-trien-17-on. $[\alpha]_D^{22}$ = +115,4° (c = 0.5% in Chloroform).

**c) 11β-Fluor-3-hydroxy-7α-{5-[2-(4,4,5,5,5-pentafluorpentylthiomethyl)-pyrrolidin-1-yl]-pentyl}-estra-1,3,5(10)-trien-17-on**

Eine Lösung von 785,9 mg 7α-(5-Chlorpentyl)-11β-fluor-3-hydroxy-estra-1,3,5(10)-trien-17-on in7 ml N-Methyl-2-pyrrolidinon wird mit 535,5 mg Lithiumiodid und 520 mg (2S)-2-(4,4,5,5,5-Pentafluorpentylthiomethyl)-pyrrolidin versetzt und 2,5 Stunden bei 100 °C Badtemperatur gerührt. Dann wird auf Wasser gegeben, 3 mal mit Diethylether extrahiert, neutralgewaschen, über Natriumsulfat getrocknet, i.Vak. eingeengt und an Kieselgel mit Dichlormethan / Methanol chromatographiert. Man erhält 525 mg reines 11β-Fluor-3-hydroxy-7α-{5-[2-(4,4,5,5,5-pentafluorpentylthiomethyl)-pyrrolidin-1-yl]-pentyl}-estra-1,3,5(10)-trien-17-on. $[\alpha]_D^{22}$ = +29,3° (c = 0.5% in Chloroform).

**d} 11β-Fluor-7α-{5-[2-(4,4,5,5,5-pentafluor-pentylthiomethyl)-pyrrolidin-1-yl]-pentyl}-estra-1,3,5(10)-trien-3,17-diol**

Man löst 500 mg 11β-Fluor-3-hydroxy-7α-{5-[2-(4,4,5,5,5-pentafluorpentylthiomethyl)-pyrrolidin-1-yl]-pentyl}-estra-1,3,5(10)-trien-17-on in 5 ml Tetrahydrofuran, 2,75 ml Ethanol sowie 1,1 ml Wasser und gibt bei 0 °C Badtemperatur 100 mg Natriumborhydrid zu und rührt 0,5 Stunden bei Raumtemperatur. Dann wird auf Wasser gegeben, 3 mal mit Essigester extrahiert, mit gesättigter Kochsalzlösung neutralgewaschen, über Natriumsulfat getrocknet, i.Vak. eingeengt und an Kieselgel mit Dichlormethan / Methanol chromatographiert. Man erhält 441,3 mg 11β-Fluor-7α-{5-[2-(4,4,5,5,5-pentafluorpentylthiomethyl)-pyrrolidin-1-yl]-pentyl}-estra-1,3,5(10)-trien-3,17β-diol als Kristalle vom Schmelzpunkt 174-176 °C. $[\alpha]_D^{22}$ = -14,9° (c = 0.5% in Pyridin).

Beispiel 30

**11β-Nitrooxy-7α-(9-[4,4,5,5,5-pentafluorpentansulfonyl]-nonyl)-estra-1,3,5(10)-trien-3,17β-diol**

**a) 3,17β-Diacetyloxy-7α-(9-[4,4,5,5,5-pentafluorpentansulfonyl]-nonyl)-estra-1,3,5(10)-trien**

Man löst 6,28 g 7α-(9-[4,4,5,5,5-pentafluorpentansulfinyl]-nonyl)-estra-1,3,5(10)-trien-3,17βdiol in 30 ml Pyridin, versetzt unter Wasserkühlung mit 15 ml Essigsäureanhydrid und läßt anschließend 5 Stunden bei Raumtemperatur rühren.

Zur Aufarbeitung gießt man die Reaktionslösung auf Eiswasser, extrahiert das Diacetat mit Essigester, wäscht die organische Phase mit verdünnter Schwefelsäure, Wasser, gesättigter Kochsalzlösung und trocknet über Natriumsulfat. Als Rohprodukt erhält man ein Öl, das in 100 ml Essigsäure aufgenommen und mit 15 g Natriumperborat versetzt wird. Nach fünfstündigem Rühren bei Raumtemperatur ist die Umsetzung vollständig.

Das Reaktionsgemisch wird auf Eiswasser gegeben und dann mit Essigester extrahiert. Die organische Phase wird mit wässriger Bicarbonatlösung von Säureresten befreit. Nach dem Trocknen über Natriumsulfat wird filtriert, eingeengt und an Kieselgel chromatografiert (Hexan/Essigester, 7:3). Man erhält 6,83 g Produkt als Schaum.

**b) 3,17β-Diacetyloxy-11β-nitrooxy-7α-(9-[4,4,5,5,5-pentafluorpentansulfonyl]-nonyl)-estra-1,3,5(10)-trien-9-ol**

Zu einer Lösung von 3,0 g 3,17β-Diacetyloxy-7α-(9-[4,4,5,5,5-pentafluorpentansulfonyl]-nonyl)-estra-1,3,5(10)-trien in 50 ml wässriger Essigsäure (90 %) gibt man 18,6 g Cerammonnitrat und rührt 4 Stunden bei Raumtemperatur.

Zur Aufarbeitung gießt man die Reaktionslösung auf Eiswasser, extrahiert mit Essigester und wäscht die organische Phase mit wässriger Bicarbonatlösung neutral. Anschließend wäscht man mit gesättigter Kochsalzlösung und trocknet über Natriumsulfat.

Das Rohprodukt wird an Kieselgel chromatografiert (Hexan/Essigester, 7:3). Man erhält 2,0 g Produkt als gelbgefärbten Schaum.

**c) 3,17β-Diacetyloxy-11β-nitrooxy-7α-(9-[4,4,5,5,5-pentafluorpentansulfonyl]-nonyl)-estra-1,3,5(10)-trien**

Zur reduktiven Entfernung der 9α-Hydroxygruppe in 3,17β-Diacetyloxy-11β-nitrooxy-7α-(9-[4,4,5,5,5-pentafluorpentansulfonyl]-nonyl)-estra-1,3,5(10)-trien-9-ol löst man 2,0 g Ausgangsma-terial in 25 ml Dichlormethan, kühlt auf -15 °C ab und versetzt sukzessiv mit 15 ml Triethylsilan und 2,2 ml Bortrifluorid-Ethorat. Nach einstündigem Rühren bei -15 °C entfernt man das Kältebad und läßt auf Raumtemperatur auftauen.

Zur Aufarbeitung gießt man auf Eiswasser, extrahiert mit Dichlormethan, wäscht die organische Phase mit wässriger Bicarbonatlösung, gesättigter Kochsalzlösung und trocknet über Natriumsulfat.

Das Rohprodukt wird an Kieselgel chromatografiert (Hexan/Essigester, 9:1), Ausbeute 1,3 g Schaum.

**d) 11β-Nitrooxy-7α-(9-[4,4,5,5,5-pentafluorpentansulfonyl]-nonyl)-estra-1,3,5(10)-trien-3,17β-diol**

Zur Verseifung löst man 1,0 g 3,17β-Diacetyloxy-11β-nitrooxy-7α-(9-[4,4,5,5,5-pentafluorpentan-sulfonyl]-nonyl)-estra-1,3,5(10)-trien in 50 ml Methanol, versetzt mit 20 ml 3%iger methanolischer Kalilauge und läßt 4 Stunden bei Raumtemperatur stehen.

Das Reaktionsgemisch wird in zitronensaures Eiswasser eingerührt und anschließend mit Essigester extrahiert. Nach Waschen der organischen Phase mit Wasser und gesättigter Kochsalzlösung wird über Natriumsulfat getrocknet.

Das Rohprodukt wird an Kieselgel chromatografiert (Hexan/Essigester, Gradient bis 3:2), Ausbeute 0,53 g als Öl.

Beispiel 31

**11β-Fluor-17α-methyl-7α-{5-[(2S)-2-(4,4,5,5,5-pentafluorpentylthiomethyl)-pyrrolidin-1-yl]-pentyl}-estra-1,3,5(10)-trien-3,17β-diol**

**a) 7α-(5-Chlorpentyl)-11β-fluor-17α-methyl-estra-1,3,5(10)-trien-3,17β-diol**

Unter den Bedingungen des Beispiels 28a werden 750 mg 7α-(5-Chlorpentyl)-11β-fluor-3-hydroxy-estra-1,3,5(10)-trien-17-on (Beispiel 29b) mit 4,9 ml Methylmagnesiumbromidlösung (3M in Diethylether) umgesetzt, aufbereitet

und chromatographiert. Es werden 561 mg 7α-(5-Chlorpentyl)-11β-fluor-17α-methyl-estra-1,3,5(10)-trien-3,17β-diol als Schaum erhalten. $[\alpha]_D^{22}$ = +51,6 ° (c=0,515 in Chloroform).

**b) 11β-Fluor-17α-methyl-7α-{5-[(2S)-2-(4,4,5,5,5-pentafluorpentylthiomethyl)-pyrrolidin-1-yl]-pentyl}-estra-1,3,5(10)-trien-3,17β-diol**

Unter den Bedingungen des Beispiels 15f werden 408 mg 7α-(5-Chlorpentyl)-11β-fluor-17α-methyl-estra-1,3,5(10)-trien-3,17β-diol in 5 ml N-Methyl-2-pyrrolidinon in Gegenwart von 130 mg Lithiumiodid mit 606 mg (2S)-2-(4,4,5,5,5-Pentafluorpentylthiomethyl)-pyrrolidin umgesetzt, aufbereitet und chromatographiert. Es werden 326 mg der Titelverbindung als Kristalle vom Schmelzpunkt 120-121 °C erhalten.
$[\alpha]_D^{22}$ = -5,8° (c = 0,535% in Chloroform).

Beispiel 32

**11β-Fluor-17α-methyl-7α-{5-[(2S)-2-(4,4,5,5,5-pentafluorpentansulfinylmethyl)-pyrrolidin-1-yl]-pentyl}-estra-1,3,5(10)-trien-3,17β-diol**

Unter den Bedingungen des Beispiels 13 werden 260 mg 11β-Fluor-17α-methyl-7α-{5-[(2S)-2-(4,4,5,5,5-pentafluorpentylthiomethyl)-pyrrolidin-1-yl]-pentyl}-estra-1,3,5(10)-trien-3,17β-diol (Beispiel 31b) mit 151 mg Natriumperiodat umgesetzt, aufbereitet und chromatographiert. Es werden 129 mg der Titelverbindung als Öl erhalten.

Beispiel 33

**3,17β-Diacetoxy-11β-fluor-17α-methyl-7α-{5-[(2S)-2-(4,4,5,5,5-pentafluorpentansulfonylmethyl)-pyrrolidin-1-yl]-pentyl}-estra-1,3,5(10)-trien**

Unter den Bedingungen des Beispiels 3 werden 65 mg 11β-Fluor-17α-methyl-7α-{5-[(2S)-2-(4,4,5,5,5-pentafluorpentylthiomethyl)-pyrrolidin-1-yl]-pentyl}-estra-1,3,5(10)-trien-3,17β-diol (Beispiel 31b) mit Acetanhydrid acetyliert und das Rohprodukt wie in Beispiel 4 beschreiben mit Natriumperborat Tetrahydrat oxidiert, aufbereitet und chromatographiert. Es werden 27 mg der Titelverbindung als Öl erhalten.

Beispiel 34

**7α-{5-[(2S)-2-(4,4,5,5,5-Pentafluorpentansulfinylmethyl)-pyrrolidin-1-yl]-pentyl}-estra-1,3,5(10)-trien-3,17β-diol**

Durch Oxidation mit Natriumperiodat erhält man, wie im Beispiel 2 beschrieben, aus 152 mg 7α-{5-[(2S)-2-(4,4,5,5,5-Pentafluorpentylthiomethyl)-pyrrolidin-1-yl]-pentyl} -estra-1,3,5(10)-trien-3,17β-diol (Beispiel 26f) 66 mg der Titelverbindung als Öl. $[\alpha]_D^{22}$ = +11,8° (c = 0,53% in Methanol).

Beispiel 35

**7α-{5-[(2S)-2-(4,4,5,5,5-Pentafluorpentansulfonylmethyl)-pyrrolidin-1-yl]-pentyl}-estra-1,3,5(10)-trien-3,17β-diol**

Unter den Bedingungen des Beispiels 3 werden 76 mg 7α-{5-[(2S)-2-(4,4,5,5,5-Pentafluorpentylthiomethyl)-pyrrolidin-1-yl]-pentyl}-estra-1,3,5(10)-trien-3,17β-diol (Beispiel 26f) mit Acetanhydrid acetyliert und das Rohprodukt wie in Beispiel 4 beschreiben mit Natriumperborat Tetrahydrat oxidiert, aufbereitet und chromatographiert. Es werden 31 mg der Titelverbindung als Öl erhalten. $[\alpha]_D^{22}$ = +30,6° (c = 0,515% in Methanol).

Beispiel 36

**11β-Fluor-17α-methyl-7α-{5-[N-methyl-N-3-(4,4,5,5,5-pentafluorpentylthio)-propylamino]-pentyl}-estra-1,3,5 (10)-trien-3,17β-diol**

**a) 11β-Fluor-3-hydroxy-17α-methyl-7α-{5-[N-methyl-N-3-(4,4,5,5,5-pentafluorpentylthio)-propylamino]-pentyl}-estra-1,3,5(10)-triem-17-on**

Eine Lösung von 2,0 g 11β-Fluor-7α-{5-[N-methyl-N-3-(4,4,5,5,5-pentafluor-pentylthio)-propylamino]pentyl}-3-(tetrahydrapyran-2-yloxy)-estra-1,3,5(10)-trien-17-on in 20 ml Methanol und 2 ml Wasser wird bei Raumtemperatur mit 1,2 g Oxalsäure gerührt. Nach 2,5 h gibt man auf Eis-Wasser, extrahiert mit Dichlormethan, wäscht mit gesättigter Kochsalzlösung, trocknet über Natriumsulfat, engt i.Vak. ein und chromatographiert an Kieselgel mit Dichlormethan / Methanol. Man erhält 1,2 g 11β-Fluor-3-hydroxy-17α-methyl-7α-{5-[N-methyl-N-3-(4,4,5,5,5-pentafluorpentylthio)-propylamino]-pentyl}-estra-1,3,5(10)-trien-17-on als Schaum. $[\alpha]_D^{22}$ = +69° (c = 0.5% in Chloroform).

**b) 11β-Fluor-17α-methyl-7α-{5-[N-methyl-N-3-(4,4,5,5,5-pentafluorpentylthio)-propylamino]-pentyl}-estra-1,3,5 (10)-trien-3,17β-diol**

Zu 5 g 11β-Fluor-3-hydroxy-17α-methyl-7α-{5-[N-methyl-N-3-(4,4,5,5,5-pentafluorpentylthio)-propylamino]-pentyl}-estra-1,3,5(10)-trien-17-on in 150 ml Tetrahydrofuran tropft man bei Raumtemperatur 33 ml einer 1,6 molaren etherischen Lithiummethylatlösung. Nach 2,5 Stunden gibt man unter Eiskühlung gesättigte Ammoniunchlorid-Lösung zu, extrahiert mit Essigester, wäscht mit gesättigter Kochsalzlösung, trocknet über Natriumsulfat und engt i.Vak. ein. Nach Chromatographie an Kieselgel, das 2 % Triethylamin enthält, mit Dichlormethan / Methanol erhält man 2,0 g 11β-Fluor-17α-methyl-7α-{5-[N-methyl-N-3-(4,4,5,5,5-pentafluorpentylthio)-propylamino]-pentyl}-estra-1,3,5(10)-trien-3,17β-diol als Kristalle vom Schmelzpunkt 83 °C.

Beispiel 37

**11β-Fluor-17α-methyl-7α-{5-[N-methyl-N-3-(4,4,5,5,5-pentafluorpentansulfinyl)-propylamino]-pentyl}-estra-1,3,5(10)-trien-3,17β-diol**

Eine Lösung von 500 mg 11β-Fluor-17α-methyl-7α-{5-[N-methyl-N-3-(4,4,5,5,5-pentafluorpentylthio)-propylamino]-pentyl)-estra-1,3,5(10)-trien-3,17β-diol in 20 ml Methanol und 0,9 ml Wasser wird bei Raumtemperatur mit 355 mg Natriumperiodat 3 Stunden gerührt. Dann wird auf Eiswasser gegeben, mit Dichlormethan extrahiert, mit gesättigter Kochsalzlösung gewaschen, über Natriumsulfat getrocknet, i.Vak. eingeengt und an Kieselgel mit Dichlormethan / Methanol chromatographiert. Man erhält 216 mg 11β-Fluor-17α-methyl-7α-{5-[N-methyl-N-3-(4,4,5,5,5-pentafluorpentansulfinyl)-propylamino]-pentyl}-estra-1,3,5(10)-trien-3,17β-diol als Kristalle vom Schmelzpunkt 83,4 °C.

Beispiel 38

**11β-Fluor-17α-methyl-7α-{5-[N-methyl-N-3-(4,4,5,5,5-pentafluorpentansulfonyl)-propylamino]-pentyl}-estra-1,3,5(10)-trien-3,17β-diol**

**a) 11β-Fluor-3-hydroxy-17α-methyl-7α-{5-[N-methyl-N-3-(4,4,5,5,5-pentafluorpentansulfonyl)-propylamino]-pentyl}-estra-1,3,5(10)-trien-17-on**

Eine Lösung von 3 g 7α-(5-Chlorpentyl)-11β-fluor-3-hydroxy-estra-1,3,5(10)-trien-17-on in 50 ml Dimethylformamid wird mit 1,6 g Lithiumiodid und 6,2 g Methyl-[3-(4,4,5,5,5-Pentafluorpentansulfonyl)-propyl]-amin 22 Stunden bei 100 °C gerührt. Dann wird mit Essigester extrahiert, mit gesättigter Kochsalzlösung gewaschen, über Natriumsulfat getrocknet, i.Vak. eingeengt und an Kieselgel mit Dichlormethan / Methanol chromatographiert. Man erhält 4,5 g 11β-Fluor-3-hydroxy-17α-methyl-7α-{5-[N-methyl-N-3-(4,4,5,5,5-pentafluorpentansulfonyl)-propylamino]-pentyl}-estra-1,3,5 (10)-trien-17-on als Schaum.

**b) 11β-Fluor-17α-methyl-7α-{5-[N-methyl-N-3-(4,4,5,5,5-pentafluorpentansulfonyl)-propylamino]-pentyl}-estra-1,3,5(10)-trien-3,17β-diol**

Eine Suspension von 11,4 g wasserfreiem Cer(III)chlorid in 120 ml Tetrahydrofuran wird 2 Stunden bei Raumtemperatur unter Stickstoff gerührt, bei 0 °C tropfenweise mit 17,5 ml einer 3 molaren, etherischen Methylmagnesiumbromid-Lösung versetzt, 30 Minuten gerührt, eine Lösung von 3,5 g 11β-Fluor-3-hydroxy-17α-methyl-7α-{5-[N-methyl-N-3-(4,4,5,5,5-pentafluorpentansulfonyl)-propylamino]-pentyl}-estra-1,3,5(10)-trien-17-on in 24 ml Tetrahydrofuran zugegeben und weitere 30 Minuten gerührt. Dann gibt man gesättigte Ammoniumchlorid-Lösung zu, verdünnt mit Essigester, wäscht mit gesättigter Kochsalzlösung, trocknet über Natriumsulfat, engt i.Vak. ein und chromatographiert an Kieselgel mit Dichlormethan / Methanol. Man erhält 2,2 g 11β-Fluor-17α-methyl-7α-{5-[Nmethyl-N-3-(4,4,5,5,5-pentafluorpentansulfonyl)-propylamino]-pentyl}-estra-1,3,5(10)-trien-3,17β-diol als Kristalle vom Schmelzpunkt 82,5 °C.

Beispiel 39

**11β-Fluor-7α-{5-[N-methyl-N-2-(4,4,5,5,5-pentafluorpentansulfonyl)-ethylamino]-pentyl}-estra-1,3,5(10)-trien-3,17β-diol**

**a) 7α-(5-Brompentyl)-11β-fluor-estr-4-en-3,17-dion**

Eine Lösung von 33 g 11β-Fluor-7α-(5-hydroxypentyl)-estr-4-en-3,17-dion in 330 ml Dichlormethan wird bei -5 °C mit 28,9 g Triphenylphosphin und 36,7 g Tetrabromkohlenstoff versetzt und 0,5 Stunden gerührt. Anschließend setzt man Dichlormethan zu und wäscht mit Wasser, gesättigter Natriumhydrogencarbonat- und Kochsalzlösung. Die organische Phase wird über Natriumsulfat getrocknet und i.Vak. eingeengt. Dann wird das Rohprodukt an Kieselgel mit einem Hexan-Essigester-Gradienten chromatographiert. Es werden 28,5 g 7α-(5-Brompentyl)-11β-fluor-estr-4-en-3,17-dion vom Schmelzpunkt 75-76 °C erhalten.

**b) 7α-(5-Brompentyl)-11β-fluor-3-hydroxy-estra-1,3,5(10)-trien-17-on**

Zu 27,8 g 7α-(5-Brompentyl)-11β-fluor-estr-4-en-3,17-dion in 190 ml Acetonitril werden bei 80°C 17,0 g Kupfer (II)-bmmid gegeben. Nach 8 Stunden wird das Reaktionsgemisch in Wasser eingerührt, dreimal mit Essigester extrahiert, zweimal mit Ammoniumchlorid, mit Natriumhydrogencarbonat und Kochsalz gewaschen, getrocknet und im Vakuum eingeengt. Nach Chromatographieren des Rohproduktes an Kieselgel mit einem Hexan-Essigester-Gradienten werden 20,4 g 7α-(5-Brompentyl)-11β-fluor-3-hydroxy-estra-1,3,5(10)-trien-17-on als farblose Kristalle vom Schmelzpunkt 178 °C erhalten.

**c) 7α-(5-Brompentyl)-11β-fluor-estra-1,3,5(10)-trien-3,17β-diol**

Eine Lösung von 16,2 g 7α-(5-Brompentyl)-11β-fluor-3-hydroxy-estra-1,3,5(10)-trien-17-on in 162 ml Tetrahydrofuran sowie 90 ml Ethanol und 36 ml Wasser wird bei 0 °C mit 4,7 g Natriumborhydrid portionsweise versetzt und 2 Stunden bei 0 °C gerührt. Dann wird auf Wasser gegeben, viermal mit Essigester extrahiert, mit Wasser und Kochsalzlösung gewaschen, über Natriumsulfat getrocknet und i.Vak. eingeengt. Man erhält 17,1 g Rohprodukt. Nach Chromatographie an Kieselgel mit Hexan / Essigester erhält man 15,6 g reines 7α-(5-Brompentyl)-11β-fluor-estra-1,3,5(10)-trien-3,17β-diol.

**d) 11β-Fluor-7α-[5-(methylamino)-pentyl]-estra-1,3,5(10)-trien-3,17β-diol**

Eine Lösung von 2 g 7α-(5-Brompentyl)-11β-fluor-estra-1,3,5(10)-trien-3,17β-diol in 20 ml Dimethylformamid wird mit 8 ml einer 40%igen wässrigen Methylaminlösung 3,5 Stunden bei 80 °C gerührt. Dann wird auf Wasser gegeben, dreimal mit Essigester extrahiert, mit Wasser und Kochsalzlösung gewaschen, über Natriumsulfat getrocknet und i.Vak. eingeengt. Man erhält 1,77 g 11β-Fluor-7α-[5-(methylamino)-pentyl]-estra-1,3,5(10)-trien-3,17β-diol.

**e) 11β-Fluor-7α-{5-[N-methyl-N-2-(4,4,5,5,5-pentafluorpentansulfonyl)-ethylamino]-pentyl}-estra-1,3,5(10)-trien-3,17β-diol**

Eine Lösung von 440 mg 11β-Fluor-7α-[5-(methylamino)-pentyl]-estra-1,3,5(10)-trien-3,17β-diol in 15 ml Methanol wird mit 500 mg 4,4,5,5,5-Pentafluorpentylvinylsulfon 1 Stunde bei 90 °C gerührt. Dann wird auf Wasser gegeben, dreimal mit Essigester extrahiert, mit Wasser und Kochsalzlösung gewaschen, über Natriumsulfat getrocknet, i. Vak. eingeengt und an Kieselgel mit Dichlormethan / Methanol chromatographiert. Man erhält 488 mg 11β-Fluor-7α -{5-[N-methyl-N-2-(4,4,5,5,5-pentafluorpentansulfonyl)-ethylamino]-pentyl}-estra-1,3,5(10)-trien-3,17β-diol als Kristalle vom Schmelzpunkt 74-76 °C.

Die entschützte Zwischenstufe 12k) ist stark antiestrogen wirksam:

Beispiel 40

**11β-Fluor-7α-{5-[N-methyl-N-3-{4,4,5,5,5-pentafluorpentylthio)-propylamino]-pentyl}-3-hydroxy-estra-1,3,5(10)-trien-17-on**

Eine Lösung von 1,6 g 11β-Fluor-7α-{5-[N-methyl-N-3-(4,4,5,5,5-pentafluorpentylthio)-propylamino]-pentyl}-3-(tetrahydrapyran-2-yloxy)-estra-1,3,5(10)-trien-17-on in 20 ml Methanol und 2 ml Wasser wird mit 1,0 g Oxalsäure gerührt. Nach 3 h wird das Reaktionsgemisch in Eis-Wasser gegeben. Man extrahiert mit Methylenchlorid, wäscht mit gesättigter Natriumchloridlösung, trocknet und engt im Vakuum ein. Nach Chromatographieren des Rohproduktes an Kieselgel mit einem Methylenchlorid-Methanol-Gradienten werden 1,1g 11β-Fluor-7α-{5-[N-methyl-N-3-(4,4,5,5,5-pentafluorpentylthio)-propylamino]-pentyl}-3-hydroxy-estra-1,3,5(10)-trien-17-on erhalten. $[\alpha]_D^{22}$ = +69° (c = 0.5% in Chloroform)

Beispiel 41

**11β-Fluor-7α-{6-[N-methyl-N-3-(4,4,5,5,5-pentafluorpentylthio)-propylamino]-hexyl}-estra-1,3,5(10)-trien-3,17β-diol**

**a) 7α-(6-Chlorhexyl)-11β-fluor-estr-4-en-3,17-dion**

Zu einer Suspension von 6,8 g Magnesiumspänen in 100 ml THF werden unter Stickstoff zunächst 30 ml einer Lösung aus 41 ml 1-Brom-6-chlor-hexan in 270 ml THF zugegeben. Nach dem Anspringen der Reaktion wird die restliche Lösung so zugetropft, daß die Innentemperatur 35 °C nicht überschreitet. In einem zweiten Kolben werden zu einer Suspension von 26,4 g Kupfer(I)iodid in 120 ml THF bei 0 °C 48,1 g Lithiumbromid zugegeben, wobei die Innentemperatur auf 40 °C steigt. Ohne Kühlung werden nun 46,4 ml 1,3-Dimethyl-3,4,5,6-tetrahydro-(1H)-pyrimidin-2-on hinzugefügt und 30 Minuten bei 40 °C gerührt. Man erhält eine klare Lösung, die zu der auf -40 °C gekühlten Grignard-Lösung getropft wird. Anschließend wird 30 Minuten bei -30 °C nachgerührt und bei -50°C tropfenweise mit einer Lösung von 23,5 g 11β-Fluor-estra-4,6-dien-3,17-dion in 230 ml THF, 24,6 ml 1,3-Dimethyl-3,4,5,6-tetrahydro-(1H)-pyrimidin-2-on und 55 ml Trimethylchlorsilan so versetzt, daß die Innentemperatur -40 °C nicht überschreitet. Es wird 1h in der Kälte gerührt, dann 32 ml Eisessig zugetropft, das Kühlbad entfernt und 1 Stunde bei Raumtemperatur nachgerührt. Zur Aufarbeitung wird das Reaktionsgemisch auf 1,51 Wasser gegeben, mit der gleichen Menge Essigester verdünnt, der Niederschlag über Celite abgetrennt, mit Essigester nachgewaschen, die wässrige Phase 3 mal mit Essigester extrahiert, mit Natriumhydrogencarbonat- und Kochsalzlösung gewaschen, über Magnesiumsulfat getrocknet und i. Vak. eingeengt. Nach Chromatographieren des Rohproduktes an Kieselgel mit einem Hexan-Essigester-Gradienten werden 25,2g 7α-(6-Chlorhexyl)-11β-fluor-estr-4-en-3,17-dion erhalten.

**b) 7α-(6-Chlorhexyl)-11β-fluor-3-hydroxy-estra-1,3,5(10)-trien-17-on**

Zu 25,2 g 7α-(6-Chlorhexyl)-11β-fluor-estr-4-en-3,17-dion in 175 ml wasserfreiem Acetonitril werden bei 80 °C 28,1 g Kupfer(II)bromid und 5,4 g Lithiumbromid in 105 ml wasserfreiem Acetonitril gegeben. Nach 15 Minuten wird das Reaktionsgemisch auf 0 °C gekühlt und 250 ml gesättigte Natriumhydrogencarbonat-Lösung zugetropft. Anschließend wird die Lösung in 1 Liter Wasser eingerührt, mit 2N Salzsäure auf pH=6 gebracht, 3 mal mit Essigester extrahiert, mit Kochsalzlösung gewaschen, über Magnesiumsulfat getrocknet und i. Vak. eingeengt. Chromatographie des Rohproduktes an Kieselgel mit einem Hexan-Essigester-Gradienten liefert 5,7 g 7α-(6-Chlorhexyl)-11β-fluor-3-hydroxy-estra-1,3,5(10)-trien-17-on als Schaum.

**c) 11β-Fluor-3-hydroxy-7α-(6-iodhexyl)-estra-1,3,5(10)-trien-17-on**

2,7 g 7α-(6-Chlorhexyl)-11β-fluor-3-hydroxy-estra-1,3,5(10)-trien-17-on werden in 40 ml Ethylmethylketon gelöst, mit 3,0 g Natriumjodid versetzt und über Nacht bei 90 °C Badtemperatur gerührt. Zur Aufarbeitung wird das Reaktionsgemisch auf Raumtemperatur abgekühlt, in Wasser eingerührt, 3 mal mit Essigester extrahiert, mit Kochsalzlösung gewaschen, über Magnesiumsulfat getrocknet und i. Vak. eingeengt. Man erhält 3.4 g 11β-Fluor-3-hydroxy-7α-(6-iodhexyl)-estra-1,3,5(10)-trien-17-on als Rohprodukt, welches ohne weitere Aufreinigung in der nächsten Stufe eingesetzt wird.

**d) 11β-Fluor-3-hydroxy-7α-{6-[N-methyl-N-3-(4,4,5,5,5-pentafluorpentylthio)-propylamino]-hexyl}-estra-1,3,5 (10)-trien-17-on**

2,5 g 11β-Fluor-3-hydroxy-7α-(6-iodhexyl)-estra-1,3,5(10)-trien-17-on in 20 ml wasserfreiem DMF werden bei 100 °C Badtemperatur mit 2,0 g Methyl-[3-(4,4,5,5,5-pentafluorpentylthio)-propyl]-amin gerührt. Nach 2 Stunden gießt man die Reaktionslösung in halbgesättigte Natriumhydrogencarbonat-Lösung, extrahiert 3 mal mit Methylenchlorid, trocknet über Magnesiumsulfat und engt i. Vak ein. Der Rückstand wird an Kieselgel mit einem Methylenchlorid-Methanol-Gradienten chromatographiert. Es werden 3,15g 11β-Fluor-3-hydroxy-7α-{6-[N-methyl-N-3-(4,4,5,5,5-pentafluorpentylthio)-propylamino]-hexyl}-estra-1,3,5(10)-trien-17-on als Schaum erhalten.

**e) 11β-Fluor-7α-{6-[N-methyl-N-3-(4,4,5,5,5-pentafluorpentylthio)-propylamino]-hexyl}-estra-1,3,5(10)-trien-3,17-diol**

250 mg 11β-Fluor-3-hydroxy-7α-{6-[N-methyl-N-3-(4,4,5,5,5-pentafluorpentylthio)-propylamino]-hexyl)-estra-1,3,5(10)-trien-17-on werden in 4,5 ml Methanol gelöst und bei 0 °C portionsweise mit 60 mg Natriumborhydrid versetzt. Nach 3 Stunden Rühren bei Raum-temperatur, wird das Lösungsmittel im Vakuum abgezogen, der Rückstand mit gesättigter Kochsalzlösung versetzt, 3 mal mit Methylenchlorid extrahiert, über Magnesiumsulfät getrocknet und i. Vak. eingeengt. Nach Chromatographie an Kieselgel mit einem Methylenchlorid-Methanol-Gradienten erhält man 165 mg 11β-Floor-7α-{6-[N-methyl-N-3-(4,4,5,5,5-pentafluorpentylthio)-propylamino]-hexyl)-estra-1,3,5(10)-trien-3,17-diol als Schaum, $[\alpha]_D$ = +37 ° (c=1,01 in Chloroform).

Beispiel 42

**11β-Fluor-7α-{6-[N-methyl-N-3-(4,4,5,5,5-pentafluorpentansulfinyl)-propylamino]-hexyl}-estra-1,3,5(10)-trien-3,17-diol**

**a) 11β-Fluor-3-hydroxy-7α-{6-[N-methyl-N-3-(4,4,5,5,5-pentafluorpentansulfinyl)-propylamino]-hexyl}-estra-1,3,5(10)-trien-17-on**

500 mg 11β-Fluor-3-hydroxy-7α-{6-[N-methyl-N-3-(4,4,5,5,5-pentafluorpentylthio)-propylamino]-hexyl}-estra-1,3,5(10)-trien-17-on werden in 17 ml Methanol und 3,3 ml Wasser gelöst, mit 262 mg Natriumperiodat versetzt und 2 Stunden bei Raumtemperatur gerührt. Zur Aufar-beitung wird das Reaktionsgemisch mit halbgesättigter Kochsalzlösung versetzt, 3 mal mit Methylenchlorid extrahiert, über Magnesiumsulfat getrocknet und i. Vak. eingeengt. Der Rückstand wird an Kieselgel mit einem Methylenchlorid-Methanol-Gradienten aufgereinigt. Man isoliert 149 mg 11β-Fluor-3-hydroxy-7α-{6-[N-methyl-N-3-(4,4,5,5,5-pentafluorpentansulfinyl)-propylamino]-hexyl}-estra-1,3,5(10)-trien-17-on als Schaum, $[\alpha]_D$ =+45 ° (c=1,015 in Chloroform).

**b) 11β-Fluor-7α-{6-[N-methyl-N-3-(4,4,5,5,5-pentafluorpentansulfinyl)-propylamino]-hexyl}-estra-1,3,5(10)-trien-3,17-diol**

149 mg 11β-Fluor-3-hydroxy-7α-{6-[N-methyl-N-3-(4,4,5,5,5-pentafluorpentansulfinyl)-propyl-amino]-hexyl}-estra-1,3,5(10)-trien-17-on werden in 3 ml Methanol gelöst und portionsweise mit 35 mg Natriumborhydrid versetzt. Nach 30 Minuten Rühren bei Raumtemperatur wird das Lösungsmittel im Vakuum abgezogen, der Rückstand mit Wasser versetzt, 3 mal mit Methylenchlorid extrahiert, über Magnesiumsulfat getrocknet und i. Vak. eingeengt. Präparative Dünnschichtchromatographie mit Methylenchlorid/Methanol=9/1 als Laufmittel führt zu 109 mg 11β-Fluor-7α-{6-[N-methyl-N-3-(4,4,5,5,5-pentafluorpentansulfinyl)-propylamino]-hexyl}-estra-1,3,5(10)-trien-3,17-diol als Schaum, $[\alpha]_D$ = +24 ° (c=0,51 in Chloroform).

Beispiel 43

**11β-Fluor-7α-(5-{[N-3-(furan-2-ylmethylthio)-propyl]-N-methyl-amino}-pentyl)-estra-1,3,5(10)-trien-3,17β-diol**

**a) 7α-(5-Chlorpentyl)-11β-fluor-estr-4-en-3,17-dion**

Zu einer Suspension von 7,2 g Magnesiumspänen in 100 ml THF werden unter Stickstoff zunächst 20% einer Lösung aus 39 ml 1-Brom-5-chlorpentan in 300 ml THF zugegeben. Nach dem Anspringen der Reaktion, welches durch Zugabe von Iod und Dibrommethan erreicht werden kann, wird die restliche Lösung so zugetropft, daß die Innentemperatur 35 °C nicht überschreitet. In einem zweiten Kolben werden zu einer Suspension von 28,1 g Kupfer(I)iodid in 130 ml THF bei 0 °C 51,2 g Lithiumbromid zugegeben, wobei die Innentemperatur auf 40 °C steigt. Ohne Kühlung werden nun 49,4 ml 1,3-Dimethyl-3,4,5,6-tetrahydro-(1H)-pyrimidin-2-on hinzugefügt und 15 Minuten bei 40 °C gerührt. Man erhält eine klare Lösung, die zu der auf -50 °C gekühlten Grignard-Lösung getropft wird. Anschließend wird 15 Minuten bei -30 °C nachgerührt und bei -70°C tropfenweise mit einer Lösung von 25 g 11β-Fluor-estra-4,6-dien-3,17-dion in 260 ml THF, 26 ml 1,3-Dimethyl-3,4,5,6-tetrahydro-(1H)-pyrimidin-2-on und 59 ml Trimethylchlorsilan so versetzt, daß die Innentemperatur -65 °C nicht überschreitet. Es wird 30 Minuten in der Kälte gerührt, dann 34,7 ml Eisessig zugetropft, das Kühlbad entfernt und 1 Stunde bei Raumtemperatur nachgerührt. Zur Aufarbeitung wird das Reaktionsgemisch auf 1,51 Wasser gegeben, mit der gleichen Menge Essigester verdünnt, der Niederschlag über Celite abgetrennt, mit Essigester nachgewaschen, die wässrige Phase 3 mal mit Essigester extrahiert, mit Natriumhydrogencarbonat- und Kochsalzlösung gewaschen, über Magnesiumsulfat getrocknet und i. Vak. eingeengt. Nach Chromatographieren des Rohproduktes an Kieselgel mit einem Hexan-Essigester-Gradienten werden 22,1 g 7α-(5-Chlorpentyl)-11β-fluor-estr-4-en-3,17-dion erhalten.

**b) 7α-(5-Chlorpentyl)-11β-fluor-3-hydroxy-estra-1,3,5(10)-trien-17-on**

Zu 22,1 g 7α-(5-Chlorpentyl)-11β-fluor-estr-4-en-3,17-dion in 160 ml wasserfreiem Acetonitril werden bei 80 °C 25,4 g Kupfer(II)bromid und 4,9 g Lithiumbromid in 95 ml wasserfreiem Acetonitril gegeben. Nach 20 Minuten wird das Reaktionsgemisch auf 0 °C gekühlt und 200 ml gesättigte Natriumhydrogencarbonat-Lösung zugetropft. Anschließend wird die Lösung in 750 ml Wasser eingerührt, mit 2N Salzsäure auf pH=6 gebracht, 3 mal mit Essigester extrahiert, mit Kochsalzlösung gewaschen, über Magnesiumsulfat getrocknet und i. Vak. eingeengt. Chromatographie des Rohproduktes an Kieselgel mit einem Hexan-Essigester-Gradienten liefert 14,7 g 7α-(5-Chlorpentyl)-11β-fluor-3-hydroxy-estra-1,3,5(10)-trien-17-on.

**c) 11β-Fluor-3-hydroxy-7α-(5-iodpentyl)-estra-1,3,5(10)-trien-17-on**

5,0 g 7α-(5-Chlorpentyl)-11β-fluor-3-hydroxy-estra-13,5(10)-trien-17-on werden in 80 ml Ethylmethylketon gelöst, mit 5,7 g Natriumjodid versetzt und über Nacht bei 90 °C Badtemperatur gerührt. Zur Aufarbeitung wird das Reaktionsgemisch auf Raumtemperatur abgekühlt, in Wasser eingerührt, 3 mal mit Essigester extrahiert, mit Kochsalzlösung gewschen, über Magnesiumsulfat getrocknet und i. Vak. eingeengt. Man erhält 6,8 g 11β-Fluor-3-hydroxy-7α-(5-iodpentyl)-estra-1,3,5(10)-trien-17-on als Rohprodukt, welches ohne weitere Aufreinigung in der nächsten Stufe eingesetzt wird.

**d) 11β-Fluor-3-hydroxy-7α-[5-(methylamino)-pentyl]-estra-1,3,5(10)-trien-17-on**

In eine Lösung von 6,8 g 11β-Fluor-3-hydroxy-7α-(5-iodpentyl)-estra-1,3,5(10)-trien-17-on in 35 ml wasserfreiem Tetrahydrofuran werden bei -78 °C 5,1 g Methylamin kondensiert und über Nacht bei Raumtemperatur im Druckreaktor gerührt. Nachdem der Druckreaktor bei -20 °C geöffnet wurde, läßt man auf Raumtemperatur kommen, um überschüssiges Methylamin abdampfen zu lassen. Anschließend wird die Reaktionslösung in gesättigte Natriumhydrogencarbonat-Lösung gegeben, 3 mal mit Essigester extrahiert, über Magnesiumsulfat getrocknet und i. Vak. eingeengt. Es werden 6,7 g 11β-Fluor-3-hydroxy-7α-[5-(methylamino)-pentyl]-estra-1,3,5(10)-trien-17-on als Rohprodukt erhalten.

**e) 11β-Fluor-7α-(5-{[N-3-(furan-2-ylmethylthio)-propyl]-N-methyl-amino}-pentyl)-3-hydroxy-estra-1,3,5(10)-trien-17-on**

526 mg 11β-Fluor-3-hydroxy-7α-[5-(methylamino)-pentyl]-estra-1,3,5(10)-trien-17-on und 95 mg 2-(3-Chloropropylthiomethyl)-furan werden in 5 ml Ethylmethylketon gelöst, mit 112 mg Natriumiodid und 104 mg Kaliumcarbonat versetzt und 3h bei 90 °C Badtemperatur gerührt. Zur Aufarbeitung wird das Reaktionsgemisch in halbgesättigte Natriumhydrogencarbonat-Lösung gegeben, 3 mal mit Methylenchlorid extrahiert, mit Kochsalzlösung gewaschen, über Magnesiumsulfat getrocknet und i. Vak eingeengt. Chromatographie des Rohproduktes an Kieselgel mit einem Methylenchlorid-Methanol-Gradienten liefert 229 mg 11β-Fluor-7α-(5-{[N-3-(furan-2-ylmethylthio)-propyl]-N-methylamino}-pentyl)-3-hydroxy-estra-1,3,5(10)-trien-17-on als Schaum.

**f) 11β-Fluor-7α-(5-{[N-3-(furan-2-ylmethylthio)-propyl]-N-methyl-amino}-pentyl)-estra-1,3,5(10)-trien-3,17β-diol**

217 mg 11β-Fluor-7α-(5-{[N-3-(furan-2-ylmethylthio)-propyl]-N-methyl-amino}-pentyl)-3-hydroxy-estra-1,3,5(10)-trien-17-on werden in 6 ml Methanol gelöst und portionsweise mit 44 mg Natriumborhydrid versetzt. Nach 1 Stunde

Rühren bei Raumtemperatur wird das Lösungs-mittel im Vakuum abgezogen, der Rückstand mit Kochsalzlösung versetzt, 3 mal mit Methylen-chlorid extrahiert, über Magnesiumsulfat getrocknet und i. Vak. eingeengt. Präparative Säulen-chromatographie mit einem Methylenchlorid/Methanol-Gradienten führt zu 146 mg 11β-Fluor-7α-(5-{[N-3-(furan-2-ylmethylthio)-propyl]-N-methyl-amino}-pentyl)-estra-1,3,5(10)-trien-3,17β-diol als Schaum.

Beispiel 44

**11β-Fluor-7α-(5-{N-methyl-[N-3-(thiophen-2-ylmethylthio)-propyl]-amino}-pentyl)-estra-1,3,5(10)-trien-3,17β-diol**

**a) 11β-Fluor-3-hydroxy-7α-(5-{N-methyl-[N-3-(thiophen-2-ylmethylthio)-propyl]-amino}-pentyl)-estra-1,3,5(10)-trien-17-on**

526 mg 11β-Fluor-3-hydroxy-7α-[5-(methylamino)-pentyl]-estra-1,3,5(10)-trien-17-on und 103 mg 2-(3-Chloro-propylthiomethyl)-thiophen, werden in 5 ml Ethylmethylketon gelöst, mit 112 mg Natriumiodid und 104 mg Kaliumcarbonat versetzt und 4,5h bei 90 °C Badtemperatur gerührt. Zur Aufarbeitung wird das Reaktionsgemisch in halbgesättigte Natriumhydrogencarbo-nat-Lösung gegeben, 3 mal mit Methylenchlorid extrahiert, mit Kochsalzlösung gewaschen, über Magnesiumsulfat getrocknet und i. Vak eingeengt. Chromatographie des Rohproduktes an Kieselgel mit einem Methylenchlorid-Methanol-Gradienten liefert 191 mg 11β-Fluor-3-hydroxy-7α-(5-{N-methyl-[N-3-(thiophen-2-ylmethylthio)-propyl]-amino}-pentyl)-estra-1,3,5(10)-trien-17-on als Schaum.

**b) 11β-Fluor-7α-(5-{N-methyl-[N-3-(thiophen-2-ylmethylthio)-propyl]-amino}-pentyl)-estra-1,3,5(10)-trien-3,17β-diol**

185 mg 11β-Fluor-3-hydroxy-7α-(5-{N-methyl-[N-3-(thiophen-2-ylmethylthio)-propyl]-amino}-pentyl)-estra-1,3,5(10)-trien-17-on werden in 5 ml Methanol gelöst und portionsweise mit 28 mg Natriumborhydrid versetzt. Nach 45 Minuten Rühren bei Raumtemperatur wird das Lösungsmittel größtenteils im Vakuum abgezogen, der Rückstand in Kochsalzlösung gegeben, 3 mal mit Methylenchlorid extrahiert, über Magnesiumsulfat getrocknet und i. Vak. eingeengt. Präparative Säulenchromatographie mit einem Methylenchlorid/Methanol-Gradienten führt zu 93 mg 11β-Fluor-7α-(5-{N-methyl-[N-3-(thiophen-2-ylmethylthio)-propyl]-amino}-pentyl)-estra-1,3,5(10)-trim-3,17β-diol als Schaum.

Beispiel 45

**11β-Fluor-7α-{5-[(2S)-2-(4-trifluormethylphenylthiomethyl)-pyrrolidin-1-yl]-pentyl}-estra-1,3,5(10)-trien-3,17β-diol a) 11β-Fluor-3-hydroxy-7α-{5-[(2S)-2-(4-trifluormethylphenylthiomethyl)-pyrrolidin-1-yl]-pentyl}-estra-1,3,5(10)-trien-17-on**

Eine Lösung von 0,5 g 7α-(5-Chlorpentyl)-11β-fluor-3-hydroxy-estra-1,3,5(10)-trien-17-on in 4 ml Dimethylformamid wird mit 0,55 g (2S)-2-(4-Trifluormethylphenylthiomethyl)-pyrrolidin und 0,32 g Lithiumiodid 2 Stunden bei 100 °C Badtemperatur gerührt. Dann wird auf Natriumhydrogencarbonatlösung gegeben, dreimal mit Essigester extrahiert, mit Kochsalzlösung gewaschen, über Natriumsulfat getrocknet, i.Vak. eingeengt und an Kieselgel mit Dichlormethan / Aceton chromatographiert. Man erhält 0,45 g 11β-Fluor-3-hydroxy-7α-{5-[(2S)-2-(4-trifluormethylphenylthiomemyl)-pyrrolidin-1-yl]-pentyl}-estra-1,3,5(10)-trien-17-on mit $[\alpha]^{22}_{D}$ = +32,7° (c = 0,51 % in Chloroform).

**b) 11β-Fluor-7α-{5-[(2S)-2-(4-trifluormethylphenylthiomethyl)-pyrrolidin-1-yl]-pentyl}-estra-1,3,5(10)-trien-3,17β-diol** Eine Lösung von 0,43 g 11β-Fluor-3-hydroxy-7α-{5-[(2S)-2-(4-trifluormethylphenylthiomethyl)-pyrrolidin-1-yl]-pentyl}-extra-1,3,5(10)-trim-17-on in 4 ml Tetrahydrofuran, 2,3 ml Ethanol und 1 ml Wasser wird bei 0 °C mit 111 mg Natriumborhydrid portionsweise versetzt und 2 Stunden gerührt. Dann wird auf Eiswasser gegeben, dreimal mit Essigester extrahiert, mit Kochsalzlösung gewaschen, über Natriumsulfat getrocknet, i.Vak. eingeengt und an Kieselgel mit Dichlormethan / Aceton chromatographiert. Man erhält 0,32 g 11β-Fluor-7α-{5-[(2S)-2-(4-trifluormethylphenylthiomethyl)-pyrrolidin-1-yl]-pentyl}-estra-1,3,5(10)-trien-3,17β-diol mit $[\alpha]^{22}_{D}$ = + 16,2° (c = 0,51 % in Methanol).

**Beispiel 46**

**11β-Fluor-17α-methyl-7α-{5-[(2S)-2-(4,4,5,5,5-pentafluorpentansulfinylmethyl)-pyrrolidin-1-yl]-pentyl}-estra-1,3,5(10)-trien-3,17β-diol** Eine Lösung von 0,2 g 11β-Fluor-17α-methyl-7α-{5-[(2S)-2-(4,4,5,5,5-pentafluorpentylthiomethyl)-pyrrolidin-1-yl]-pentyl}-estra-1,3,5(10)-trien-3,17β-diol in 5,8 ml Methanol und 2,9 ml Wasser wird mit 82 mg Natriumperiodat 5 Stunden bei Raumtemperatur gerührt. Dann wird auf Wasser gegeben, dreimal mit Dichlormethan extrahiert, neutralgewaschen, über Natriumsulfat getrocknet und i.Vak. eingeengt. Man erhält 210 mg

Rohprodukt, das an Kieselgel mit Dichlormethan / Methanol chromatographiert wird. Man erhält 105 mg reines 14,17-Ethano-7α-{5-[N-Methyl-N-3-(4,4,5,5,5-pentafluorpentansulfinyl)-propylamino]-pentyl}-estra-1,3,5(10)-trien-3,17β-diol IR 1610 und 1190 [$cm^{-1}$].

Beispiel 47

**11β-Fluor-17α-methyl-7α-{5-[(2R)-2-(4,4,5,5,5-pentafluorpentylthiomethyl)-pyrrolidin-1-yl]-pentyl}-estra-1,3,5 (10)-trien-3,17β-diol**

Analog Beispiel 29 erhält man 11β-Fluor-17α-methyl-7α-{5-[(2R)-2-(4,4,5,5,5-pentafluorpentylthiomethyl)-pyrrolidin-1-yl]-pentyl}-estra-1,3,5(10)-trien-3,17β-diol mit $[\alpha]_D^{22}$ = +68,7° (c = 0,74 % in Chloroform).

Beispiel 48

**11β-Fluor-17α-methyl-7α-{5-[(2S)-2-(4,4,5,5,5-pentafluorpentansulfonylmethyl)-pyrrolidin-1-yl]-pentyl}-estra-1,3,5(10)-trien-3,17β-diol** Eine Lösung von 100 mg 3,17 β-Diacetoxy-11β-fluor-17α-methyl-7α-{5-[(2S)-2-(4,4,5,5,5-pentafluorpentansulfonylmethyl)-pyrrolidin-1-yl]-pentyl}-estra-1,3,5(10)-trien in 1,3 ml 0,2 M methanolischer Kaliumhydroxidlösung wird 2 Stunden bei Raumtemperatur gerührt. Dann wird auf Wasser gegeben, dreimal mit Dichlormethan extrahiert, mit Kochsalzlösung gewaschen, über Natriumsulfat getrocknet, i.Vak. eingeengt und an Kieselgel mit Dichlormethan / Aceton chromatographiert. Man erhält 63 mg 11β-Fluor-17α-methyl-7α-{5-[(2S)-2-(4,4,5,5,5-pentafluorpentansulfonylmethyl)-pyrrolidin-1-yl]-pentyl}-estra-1,3,5(10)-trien-3,17β-diol. IR: 1710, 1660, 1610 [$cm^{-1}$].

Beispiel 49

**11β-Fluor-7α-{5-[(2S)-2-(4,4,5,5,5-pentafluorpentansulfinylmethyl)-pyrrolidin-1-yl]-pentyl}-estra-1,3,5 (10)-trien-3,17β-diol** Eine Lösung von 300 mg 11β-Fluor-7α-{5-[(2S)-2-(4,4,5,5,5-pentafluorpentylthiomethyl)-pyrrolidin-1-yl]-pentyl} -estra-1,3,5(10)-trien-3,17β-diol in 4,3 ml Methanol und 2,1 ml Wasser wird mit 131 mg Natriumperiodat 4 Stunden bei Raumtemperatur gerührt. Dann wird auf Wasser gegeben, dreimal mit Essigester extrahiert, mit Kochsalzlösung gewaschen, über Natriumsulfat getrocknet, i.Vak. eingeengt und an Kieselgel mit Dichlormethan / Essigester chromatographiert. Man erhält 203 mg 11β-Fluor-7α-{5-[(2S)-2-(4,4,5,5,5-pentafluorpentansulfinylmethyl)-pyrrolidin-1-yl]-pentyl}-estra-1,3,5(10)-trien-3,17β-diol. $[\alpha]_D^{22}$ = +11,8° (c = 0,53 % in Methanol).

Beispiel 50

**11β-Fluor-7α-{5-[(2S)-2-(4,4,5,5,5-pentafluorpentansulfonylmethyl)-pyrrolidal-1-yl]-pentyl}-estra-1,3,5 (10)-trien-3,17β-diol** Analog wie im Beispiel 48 beschrieben erhält man 11β-Fluor-7α-{5-[(2S)-2-(4,4,5,5,5-pentafluorpentansulfonylmethyl)-pyrrolidin-1-yl]-pentyl}-estra-1,3,5(10)-trien-3,17β-diol. $[\alpha]_D^{22}$ = +30,6° (c = 0,515 % in Methanol).

Herstellung der Ausgangsverbindungen:

**N-Methyl-[3-(4,4,5,5,5-pentafluorpentylthio)-propyl]-amin**

**a) 3-Iodpropyl-4,4,5,5,5-pentafluorpentylsulfid**

Eine Lösung von 22,8 g 3-Chlorpropyl-4,4,5,5,5-pentafluorpentylsulfid in 500 ml Ethylmethylketon wird mit 40 g Natriumiodid 5 Stunden bei 100 °C Badtemperatur unter Stickstoff gerührt. Dann wird i. Vak. zur Trockne eingeengt, auf Wasser gegeben, dreimal mit Essigester extrahiert, neutralgewaschen, und über Natriumsulfat getrocknet und i. Vak. eingeengt. Man erhält 30,6 g 3-Iodpropyl-4,4,5,5,5-pentafluorpentylsulfid.

**b) N-Methyl-[3-(4,4,5,5,5-pentafluorpentylthio)-propyl]-amin**

In eine Lösung von 30,6 g 3-Iodpropyl-4,4,5,5,5-pentafluorpentylsuifid in 200 ml abs. Tetrahydrofuran werden bei -78 °C Badtemperatur 45 g Methylamin kondensiert und 1,5 Stunden bei Raumtemperatur sowie 4 Stunden bei 60 °C im Druckreaktor gerührt. Zur Öffnung des Reaktors läßt man über Nacht auf Raumtemperatur und dann auf -78 °C abkühlen. Dann läßt man auf Raumtemperatur kommen, überschüssiges Methylamin abdampfen, verdünnt mit Essigester, wäscht neutral, trocknet über Natriumsulfat, engt i.Vak. ein und chromatographiert an Kieselgel mit Dichlormethan / Methanol. Man erhält 15,7 g N-Methyl-[3-(4,4,5,5,5-pentafluorpentylthio)-propyl]-amin als Öl.

**N-Methyl-[3-(4,4,5,5,5-pentafluorpentansulfonyl)-propyl]-amin**

**a) 3-Chlorpropyl-4,4,5,5,5-pentafluorpentansulfon**

Eine Lösung von 23 g 3-Chlorpropyl-4,4,5,5,5-pentafluorpentylsulfid in 230 ml Chloroform wird bei 0 °C mit 41,8 g 70 %iger *m*-Chlorperbenzoesäure portionsweise versetzt und 1,5 Stunden bei Raumtemperatur gerührt. Dann wird mit Dichlormethan verdünnt, mit Natriumhydrogensulfit-, Natriumhydrogencarbonat- und Kochsalzlösung gewaschen, über Natriumsulfat getrocknet und i.Vak. eingeengt. Man erhält 23,8 g reines 3-Chlorpropyl-4,4,5,5,5-pentafluorpentansulfon als Kristalle vom Schmelzpunkt 74-76 °C.

**b) 3-Iodpropyl-4,4,5,5,5-pentafluorpentansulfon**

Eine Lösung von 23,5 g 3-Chlorpropyl-4,4,5,5,5-pentafluorpentansulfon in 500 ml Ethylmetylketon wird mit 40 g Natriumiodid 5 Stunden bei 100 °C Badtemperatur unter Stickstoff gerührt. Dann wird i. Vak. zur Trockne eingeengt, auf Wasser gegeben, dreimal mit Essigester extrahiert, neutralgewaschen, und über Natriumsulfat getrocknet und i. Vak. eingeengt. Man erhält 30,6 g 3-Iodpropyl-4,4,5,5,5-pentafluorpentansulfon als Kristalle vom Schmelzpunkt 88-89 °C.

**c) N-Methyl-[3-(4,4,5,5,5-pentafluorpentansulfonyl)-propyl]-amin**

Eine Lösung von 23,5 g 3-Iodpropyl-4,4,5,5,5-pentafluorpentansulfon in 200 ml abs. Tetrahydrofuran werden bei -78 °C Badtemperatur 44 g Methylamin kondesiert und 1,5 Stunden bei Raumtemperatur sowie 4 Stunden bei 60 °C im Druckreaktor gerührt. Zur Öffnung des Reaktors läßt man über Nacht auf Raurntemperatur und dann auf-78 °C abkühlen. Dann läßt man auf Raumtemperatur kommen, überschüssiges Methylamin abdampfen, verdünnt mit Essigester, wäscht neutral, trocknet über Natriumsulfat, engt i.Vak. ein und chromatographiert an Kieselgel mit Dichlormethan / Methanol. Man erhält 14,8 g N-Methyl-[3-(4,4,5,5,5-pentafluorpentansulfonyl)-propyl]-amin als Kristalle vom Schmelzpunkt 55-57 °C.

**1-Brom-5-*tert*.-butyldimethylsilyloxypentan**

**a) 5-Brom-1-pentanol**

Zu einer Lösung von 50 g 5-Brompentylacetat in 1,6 l Methanol werden 50 ml konzentrierte Schwefelsäure getropft und die Mischung 30 Stunden bei Raumtemperatur gerührt. Das Methanol wird im Vakuum abgezogen, der Rückstand in Diethylether aufgenommen, mit gesättigter Kochsalzlösung neutral gewaschen, über Natriumsulfat getrocknet und eingeengt. Es werden 28 g 5-Brom-1-pentanol als Rohprodukt erhalten.

**b) 1-Brom-5-*tert.*-butyldimethylsilyloxypentan**

Eine Lösung von 28 g des rohen 5-Brom-1-pentanols in 144 ml Tetrahydrofuran wird mit 24 g Imidazol versetzt. Anschließend wird eine Lösung von 30,3 g *tert.*-Butyldimethylchlorsilan in 46 ml Tetrahydrofuran zugetropft und 4 Stunden bei Raumtemperatur gerührt. Die Reaktionsmischung wird in Wasser gegossen, mit Diethylether ausgeschüttelt, die organische Phase 4 mal mit Wasser gewaschen, über Natriumsulfat getrocknet und eingeengt. Das Rohprodukt wird an Kieselgel mit Hexan /Diethylether chromatographiert. Es werden 42 g der Titelverbindung als farblose Flüssigkeit erhalten.

**(2S)-2-(4,4,5,5,5-Pentafluorpentylthiomethyl)-pyrrolidin**

**a) N-*tert*.-Butyloxycarbonyl-L-prolinol-*p*-tosylat**

Zu einer Lösung von 10 g N-*tert.*-Butyloxycarbonyl-L-prolinol in 170 ml Pyridin werden bei 0 °C portionsweise 24,2 g p-Toluolsulfonsäureanhydrid gegeben und die Mischung 5 Stunden bei 0 °C gerührt. Die Reaktionsmischung wird auf 2 N Salzsäure gegossen, mit Ethylacetat extrahiert, die organische Phase mit gesättigter Kochsalzlösung gewaschen, über Natriumsulfat getrocknet und eingeengt. Es werden 17,7 g N-*tert*.-Butyloxycarbonyl-L-prolinol-p-tosylat als öliges Rohprodukt erhalten. $[\alpha]_D^{22}$ = -28,0 ° (c=0545 in Chloroform).

**b) N-*tert*.-Butyloxycarbonyl-(2S)-2-(4,4,5,5,5-pentafluorpentylthiomethyl)-pyrrolidin**

Eine Lösung von 3,93 g 4,4,5,5,5-Pentafluorpentylthioacetat in 18 ml Methanol wird mit 2,94 ml einer Natriummethanolatlösung (30%ig in Methanol) versetzt und 30 Minuten bei Raumtemperatur gerührt. Diese Reaktionslösung wird zu einer Lösung von 3,0 g N-*tert*.-Butyloxycarbonyl-L-prolinol-p-tosylat gegeben und die Mischung 3 Stunden bei Raumtemperatur und 3 Stunden bei 50 °C gerührt. Das Reaktionsgemisch wird in Wasser gegossen, mit Ethylacetat extrahiert, mit gesättigter Natriumchloridlösung gewaschen, über Natriumsulfat getrocknet und eingeengt. Der Rückstand wird an Kieselgel mit Hexan/Ethylacetat chromatographiert. Es werden, 2,59 g N-*tert*.-Butyloxycarbonyl-(2S)-2-(4,4,5,5,5-pentafluorpentylthiomethyl)-pyrrolidin als Öl erhalten. $[\alpha]_D^{22}$ = -41,3 ° (c=0,530 in Chloroform).

**c) (2S)-2-(4,4,5,5,5-Pentafluorpentylthiomethyl)-pyrrolidin**

Zu 5,4 ml auf 0 °C gekühlter Trifluoressigsäure werden 2,55 g N-*tert*.-Butyloxycarbonyl-(2S)-2-(4,4,5,5,5-pentafluorpentylthiomethyl)-pyrrolidin gegeben und die Mischung 1,5 Stunden bei 0 °C und 16 Stunden bei Raumtmperatur gerührt. Die Reaktionsmischung wird auf 10%ige Natriumhydrogencarbonatlösung gegossen, mit Ethylacetat extrahiert, mit gesättigter Kochsalzlösung gewaschen, über Natriumsulfat getrocknet und eingeengt. Es werden 1,8 g der Titelverbindung als öliges Rohprodukt erhalten.

**(2R)-2-(4,4,5,5,5-Pentafluorpentylthiomethyl)-pyrrolidin**

In völlig analoger Weise wie bei der Herstellung von (2S)-2-(4,4,5,5,5-Pentafluorpentylthiomethyl)-pyrrolidin beschrieben, werden aus 10 g N-*tert.*-Butyloxycarbonyl-D-prolinol 9,69 g der Titelverbindung als öliges Rohprodukt erhalten.

**(4,4,5,5,5-Pentafluorpentyl)-vinylsulfon**

**a) (4,4,5,5,5-Pentafluorpentyl)-vinylsulfid**

Eine Lösung von 40 g 4,4,5,5,5-Pentafluorpentylthioacetat in 200 ml Methanol wird mit 34 ml einer 30%igen Natriummethylat 1 Stunde bei 25 °C gerührt, mit 21 ml 1,2-Dibromethan tropfenweise versetzt, weitere 2 Stunden bei Raumtemperatur gerührt, mit weiteren 70 ml 30%iger Natriummethylat tropfenweise versetzt und 3 Stunden bei 25 °C gerührt. Dann wird Methanol i.Vak. eingeengt, auf Wasser gegeben, dreimal mit Essigester extrahiert, mit Wasser und Kochsalzlösung gewaschen, über Natriumsulfat getrocknet und i.Vak. eingeengt. Man erhält 34 g (4,4,5,5,5-Pentafluorpentyl)-vinylsulfid.

**b) (4,4,5,5,5-Pentafluorpentyl)-vinylsulfon**

Eine Lösung von 34 g (4,4,5,5,5-Pentafluorpentyl)-vinylsulfid in 74 ml Eisessig wird tropfenweise mit 59 ml 30%igem Wasserstoffperoxid so versetzt, daß die Reaktionstemperatur 70 °C nicht überschreitet. Anschließend wird eine Stunde bei 70 °C Badtemperatur gerührt. Dann wird auf Wasser gegeben, dreimal mit Essigester extrahiert, mit Natriumthiosulfat-, Wasser und Kochsalzlösung gewaschen, über Natriumsulfat getrocknet, i.Vak. eingeengt und an Kieselgel mit Hexan / Essigester chromatographiert. Man erhält 12,3 g (4,4,5,5,5-Pentafluorpentyl)-vinylsulfon als Öl.

**2-(3-Chloro-propylthiomethyl)-furan**

Zu 1,77 ml Furan-2-yl-methanthiol in 18 ml wasserfreiem Acetonitril werden bei 0 °C 3,3 ml einer 30%igen Natriummethylatlösung in Methanol getropft. Nach 5 Minuten erfolgt die tropfenweise Zugabe von 2,6 ml 1-Brom-3-chlorpropan. Anschließend läßt man die Reaktions-lösung fiir 5 Stunden bei Raumtemperatur rühren. Zur Aufarbeitung wird der Ansatz mit Essig-ester verdünnt, mit Wasser und Kochsalzlösung gewaschen, über Magnesiumsulfat getrocknet und i. Vak. eingeent. Chromatographie des Rohproduktes an Kieselgel mit einem Hexan-Essig-ester-Gradienten liefert 1,1 g 2-(3-Chloro-propylthiomethyl)-furan als Öl.

**2-(3-Chloro-propylthiomethyl)-thiophen**

Zu 1,0 g Thiophen-2-yl-methanthiol in 8 ml wasserfreiem Acetonitril werden bei 0 °C 1,5 ml einer 30%igen Natriummethylatlösung in Methanol getropft. Nach 5 Minuten erfolgt die tropfenweise Zugabe von 1,1 ml 1-Brom-3-chlorpropan. Anschließend läßt man die Reaktions-lösung für 5 Stunden bei Raumtemperatur rühren. Zur Aufarbeitung wird der Ansatz mit Essi-gester verdünnt, mit Wasser und Kochsalzlösung gewaschen, über Magnesiumsulfat

getrocknet und i. Vak. eingeent. Chromatographie des Rohproduktes an Kieselgel mit einem Hexan-Essig-ester-Gradienten liefert 1,3 g 2-(3-Chloro-propylthiomethyl)-thiophen als Öl.

**(2S)-2-(4-Trifluormethylphenylthiomethyl)-pyrrolidin**

**a) N-*tert*.-Butyloxycarbonyl-(2S)-2-(4-trifluormethylphenylthiomethyl)-pyrrolidin**

Eine Lösung von 1,65 g 4-Trifluormethylthiophenol in 18 ml Dimethylformamid wird mit 3 g Cäsiumcarbonat und 3 g N-*tert*.-Butyloxycarbonyl-L-prolinol-p-tosylat versetzt und 8 Stunden bei Raumtemperatur gerührt. Das Reaktionsgemisch wird in Wasser gegossen, mit Ethylacetat extrahiert, mit gesättigter Natriumchloridlösung gewaschen, über Natriumsulfat getrocknet und eingeengt. Der Rückstand wird an Kieselgel mit Hexan/Ethylacetat chromatographiert. Es werden, 2,59 g N-*tert*.-Butyloxycarbonyl-(2S)-2-(4-trifluormethylphenylthiomethyl)-pyrrolidin als Öl erhalten.

**b) (2S)-2-(4-Trifluormethylphenylthiomethyl)-pyrrolidin**

Eine Lösung von 2,55 g N-*tert*.-Butyloxycarbonyl(2S)-2-(4-trifluormethylphenylthiomethyl)-pyrrolidin in 5,64 ml Trifluoressigsäure wird 1 Stunde bei 0 °C und anschließend 3,5 Stunden bei Raumtemperatur gerührt. Die Reaktionsmischung wird auf 10%ige Natriumhydrogencarbonatlösung gegossen, mit Ethylacetat extrahiert, zweimal mit 2 M Salzsäure gewaschen, die Wasserphase mit Ether extrahiert, mit Natriumhydrogencarbonat basisch gestellt, dreimal mit Essigester extrahiert, mit Kochsalzlösung gewaschen, über Natriumsulfat getrocknet und eingeengt. Es werden 557 mg (2S)-2-(4-Trifluormethylphenylthiomethyl)-pyrrolidin erhalten.

**Patentansprüche**

1. Substituierte 7α-(ξ-Aminoalkyl) - estratriene der allgemeinen Formel I

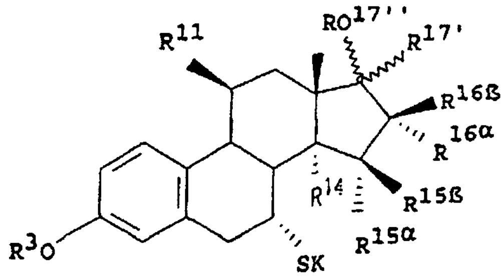

(I)

worin
die Seitenkette SK einen Rest der Teilfonnel

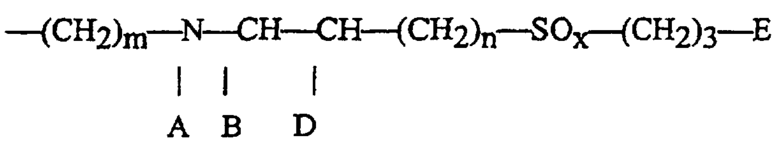

wobei

m 4, 5 oder 6,
n 0, 1 oder 2,
x 0, 1 oder 2,
A ein Wasserstoffatom oder eine $C_{1-5}$-Alkylgruppe,
B und D je ein Wasserstoffatom, oder
A und B gemeinsam eine Alkylengruppe $-(CH_2)_p-$ mit p= 2, 3, 4 oder 5 und D ein Wasserstoffatom oder

A und D gemeinsam eine Alkylengruppe —$(CH_2)_q$— mit q= 2, 3 oder 4 und B ein Wasserstoffatom, und E ein unsubstituierter oder ein- bis fünffach fluorierter Ethylrest ist, oder der terminale Substituent -$(CH_2)_3$-E in der Seitenkette durch einen gegebenenfalls substituierten Aryl- oder Heteroarylrest, der direkt oder über eine Mono-, Di- oder Trimethylengruppe an das Schwefelatom gebunden ist, ersetzt ist,

$R^3$ ein Wasserstoffatom, einen Kohlenwasserstoffrest mit bis zu 8 Kohlenstoffatomen oder einen Rest der Teilformel $R^{3'}$—C(O)—, worin $R^{3'}$ ein Wasserstoffatom oder ein Kohlenwasserstoffrest mit bis zu 8 Kohlenstoffatomen oder ein Phenylrest ist,
$R^{11}$ ein Wasserstoffatom, ein Halogenatom oder eine Nitrooxygruppe —O-$NO_2$,
$R^{14}$, $R^{15\alpha}$, $R^{15}\beta$, $R^{16\alpha}$ und $R^{16\beta}$ je ein Wasserstoffatom oder
$R^{14}$ und $R^{15}\alpha$ eine zusätzliche Bindung oder eine Methylenbrücke, oder
$R^{15\beta}$ eine Methylgruppe und $R^{15\alpha}$ ein Wasserstoffatom, oder
$R^{15\alpha}$ und $R^{15\beta}$ jeweils eine Methylgruppe, oder
$R^{15\beta}$ und $R^{16\beta}$ gemeinsam eine Methylenbrücke, oder
$R^{16\alpha}$ oder $R^{16\beta}$ ein Halogenatom oder
$R^{16\alpha}$ und $R^{16\beta}$ gemeinsam eine Methylidengruppe
und die übrigen der Substituenten $R^{14}$, $R^{15\alpha}$, $R^{15\beta}$, $R^{16\alpha}$ und $R^{16\beta}$ je ein Wasserstoffatom,
$R^{17'}$ in $\alpha$- oder $\beta$-Position ein Wasserstoffatom, eine $C_{1-5}$-Alkyl-, $C_{2-5}$-Alkenyl- oder $C_{2-5}$-Alkinylgruppe oder eine Trifluormethylgruppe und
$R^{17''}$ ein Wasserstoffatom oder einen Rest der Teilformel $R^{17'''}$—C(O)—, worin $R^{17'''}$ ein Wasserstoffatom oder ein Kohlenwasserstoffrest mit bis zu 8 Kohlenstoffatomen ist, oder,

wenn $R^{17'}$ sich in der $\alpha$-Position befindet, $R^{17'}$ gemeinsam mit $R^{14}$ eine Ethanobrücke bedeutet,
mit der Maßgabe, daß, wenn nicht A und B gemeinsam für -$(CH_2)_p$- oder A und D gemeinsam für -$(CH_2)_q$-stehen, mindestens einer der Substituenten $R^{11}$, $R^{14}$, $R^{15\alpha}$, $R^{15\beta}$, $R^{16\alpha}$ und $R^{16\beta}$, nicht ein Wasserstoffatom ist,
sowie deren physiologisch verträgliche Additionssalze mit organischen und anorganischen Säuren.

**2.** Estratriene nach Anspruch 1, worin m 5 ist

**3.** Estratriene nach Anspruch 1, worin n 0 ist.

**4.** Estratriene nach Anspruch 1, worin n 1 ist.

**5.** Estratriene nach Anspruch 1, worin n 2 ist.

**6.** Estratriene nach Anspruch 1, worin A eine Methylgruppe ist.

**7.** Estratriene nach Anspruch 1, worin x 0 ist.

**8.** Estratriene nach Anspruch 1, worin x 1 ist.

**9.** Estratriene nach Anspruch 1, worin x 2 ist.

**10.** Estratriene nach Anspruch 6, worin n 1 ist.

**11.** Estratriene nach Anspruch 1, worin A und B gemeinsam —$(CH_2)_3$— ist.

**12.** Estratriene nach Anspruch 11, worin n 0 ist.

**13.** Estratriene nach Anspruch 12, worin x 0 ist.

**14.** Estratriene nach Anspruch 1, worin E ein Perfluorethylrest ist.

**15.** Estratriene nach Anspruch 1, worin $R^3$ ein Wasserstoffatom ist.

**16.** Estratriene nach Anspruch 1, worin $R^3$ eine Methylgruppe ist.

**17.** Estratriene nach Anspruch 1, worin $R^3$ eine Acetylgruppe ist.

**18.** Estratriene nach Anspruch 1, worin $R^{11}$ ein Wasserstoffatom ist.

**19.** Estratriene nach Anspruch 1, worin $R^{11}$ ein Fluoratom ist.

**20.** Estratriene nach Anspruch 1, worin $R^{11}$ ein Nitrooxygruppe ist.

**21.** Estratriene nach Anspruch 1, worin $R^{14}$ ein Wasserstoffatom ist.

**22.** Estratriene nach Anspruch 1, worin $R^{14}$ gemeinsam mit $R^{15\alpha}$ eine zusätzliche Bindung bildet.

**23.** Estratriene nach Anspruch 1, worin $R^{15\beta}$ eine Methylgruppe ist.

**24.** Estratriene nach Anspruch 1, worin $R^{15\beta}$ und $R^{16\beta}$ gemeinsam eine Methylenbrücke bilden.

**25.** Estratriene nach Anspruch 1, worin $R^{16\alpha}$ ein Halogenatom (F, Cl, Br, I) ist

**26.** Estratriene nach Anspruch 25, worin $R^{16\alpha}$ ein Fluoratom ist.

**27.** Estratriene nach Anspruch 1, worin $R^{16\alpha}$ und $R^{16\beta}$ gemeinsam eine Methylidengruppe bilden.

**28.** Estratriene nach Anspruch 1, worin $R^{17}$ $\alpha$-ständig ist.

**29.** Estratriene nach Anspruch 28, worin $R^{17}$ eine Trifluormethylgruppe ist.

**30.** Estratriene nach Anspruch 28, worin $R^{17}$ eine Methylgruppe ist.

**31.** Estratriene nach Anspruch 28, worin $R^{17}$ ein Wasserstoffatom ist.

**32.** Estratriene nach Anspruch 1, worin $R^{17}$ $\beta$-ständig ist.

**33.** Estratriene nach Anspruch 32, worin $R^{17}$ eine Methylgruppe ist.

**34.** Estratriene nach Anspruch 32, worin $R^{17}$ ein Wasserstoffatom ist.

**35.** Estratriene nach Anspruch 28, worin $R^{14}$ und $R^{17}$ gemeinsam eine Ethanobrücke bilden.

**36.** Estratriene nach Anspruch 1, worin SK ein Rest der Teilformel

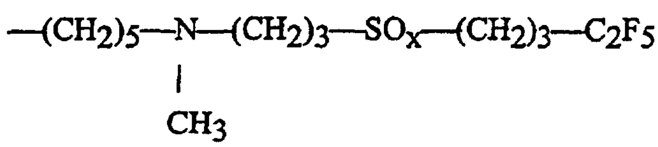

mit x= 0, 1 oder 2 ist.

**37.** Estratriene nach Anspruch 13, worin m=5, E ein Perfluorethylrest und die Konformation des 2-Kohlenstoffatoms des Heterocyclus R ist.

**38.** Estratriene nach Anspruch 13, worin m=5, E ein Perfluorethylrest und die Konformation des 2-Kohlenstoffatoms des Heterocyclus S ist

**39.** Estratriene nach Anspruch 37, worin $R^{17}$ $\alpha$-ständig und ein Wasserstoffatom ist.

**40.** Estratriene nach Anspruch 1, nämlich

14,17-Ethano-7$\alpha$-{5-[N-Methyl-N-3-(4,4,5,5,5-pentafluorpentylthio)-propylamino]-pentyl}-estra-1,3,5(10)-trien-3,17β-diol
14,17-Ethano-7α-{5-[N-Methyl-N-3-(4,4,5,5,5-pentafluorpentansulfinyl)-propylamino]-pentyl}-estra-1,3,5(10)-trien-3,17β-diol
3,17β-Diacetoxy-14α,17α-ethano-7α-{5-[N-methyl-N-3-(4,4,5,5,5-pentafluorpentylthio)-propylamino]-pentyl}-estra-1,3,5(10)-trien
14,17-Ethano-7α-{5-[N-methyl-N-3-(4,4,5,5,5-pentafluorpentansulfonyl)-propylamino]-pentyl}-estra-1,3,5(10)-trien-3,17β-diol
17α-Trifluormethyl-7α-{5-[N-methyl-N-3-(4,4,5,5,5-pentafluorpentylthio)-propylamino]-pentyl}-estra-1,3,5(10)-trien-3,17β-diol
15β,16β-Methano-17α-methyl-7α-{5-[N-methyl-N-3-(4,4,5,5,5-pentafluor-pentylthio)-propylamino]-pentyl}-estra-1,3,5(10)-trien-3,17β-diol
15β,16β-Methano-17α-methyl-7α-{5-[N-methyl-N-3-(4,4,5,5,5-pentafluorpentansulfinyl)-propylamino]-pentyl}-estra-1,3,5(10)-trien-3,17β-diol
15β,16β-Methano-7α-{5-[N-methyl-N-3-(4,4,5,5,5-pentafluor-pentylthio)-propylamino]-pentyl}-estra-1,3,5(10)-trien-3,17β-diol
15β,16β-Methano-7α-{5-[N-methyl-N-3-(4,4,5,5,5-pentafluor-pentansulfinyl)-propylamino]-pentyl}-estra-1,3,5(10)-trien-3,17β-diol
15β-Methyl-7α-{5-[N-methyl-N-3-(4,4,5,5,5-pentafluor-pentylthio)-propylamino]-pentyl)-estra-1,3,5(10)-trien-3,17β-diol
15β,17α-Dimethyl-7α-{5-[N-methyl-N-3-(4,4,5,5,5-pentafluor-pentylthio)-propylamino]-pentyl}-estra-1,3,5(10)-trien-3,17β-diol
11β-Fluor-7α-{5-[N-methyl-N-3-(4,4,5,5,5-pentafluorpentylthio)-propylamino]-pentyl}-estra-1,3,5(10)-trien-3,17β-diol
11β-Fluor-7α-{5-[N-methyl-N-3-(4,4,5,5,5-pentafluorpentansulfinyl)-propylamino]-pentyl}-estra-1,3,5(10)-trien-3,17β-diol
11β-Fluor-7α-{5-[N-methyl-N-3-(4,4,5,5,5-pentafluorpentansulfonyl)-propylamino]-pentyl}-estra-1,3,5(10)-trien-3,17β-diol
16α-Fluor-17α-methyl-7α-{5-[N-methyl-N-3-(4,4,5,5,5-pentafluorpentylthio)-propylamino]-pentyl}-estra-1,3,5(10)-trien-3,17β-diol
16α-Fluor-17β-methyl-7α-{5-[N-methyl-N-3-(4,4,5,5,5-pentafluorpentylthio)-propylamino]-pentyl}-estra-1,3,5(10)-trien-3,17α-diol
16α-Fluor-17α-methyl-7α-{5-[N-methyl-N-3-(4,4,5,5,5-pentafluorpentansulfinyl)-propylamino)-pentyl)-estra-1,3,5(10)-trien-3,17β-diol
16α-Fluor-17α-methyl-7α-{5-[N-methyl-N-3-(4,4,5,5,5-pentafluorpentansulfonyl)-propylamino]-pentyl}-estra-1,3,5(10)-trien-3,17β-diol
16α-Fluor-7α-{5-[N-methyl-N-3-(4,4,5,5,5-pentafluorpentylthio)-propylamino]-pentyl}-estra-1,3,5(10)-trien-3,17β-diol
16α-Fluor-7α-{5-[N-methyl-N-3-(4,4,5,5,5-pentafluorpentylthio)-propylamino]-pentyl}-estra-1,3,5(10)-trien-3,17α-diol
16α-Fluor-7α-{5-[N-methyl-N-3-(4,4,5,5,5-pentafluorpentansulfinyl)-propylamino]-pentyl}-estra-1,3,5(10)-trien-3,17β-diol
16α-Fluor-7α-{5-[N-methyl-N-3-(4,4,5,5,5-pentafluorpentansulfonyl)-propylamino]-pentyl}-estra-1,3,5(10)-trien-3,17β-diol
7α-{5-[N-Methyl-N-3-(4,4,5,5,5-pentafluorpentylthio)-propylamino]-pentyl}-estra-1,3,5(10),14-tetraen-3,17β-diol
7α-{5-[N-Methyl-N-3-(4,4,5,5,5-pentafluorpentansulfinyl)-propylamino]-pentyl}-estra-1,3,5(10),14-tetraen-3,17β-diol
7α-{5-[N-Methyl-N-3-(4,4,5,5,5-pentafluorpentansulfonyl)-propylamino]-pentyl}-estra-1,3,5(10),14-tetraen-3,17β-diol
7α-{5-((2S)-2-(4,4,5,5,5-Pentafluorpentylthiomethyl)-pyrrolidin-1-yl]-pentyl}-estra-1,3,5(10)-trien-3,17β-diol
7α-{5-[(2R)-2-{4,4,5,5,5-Pentafluorpentylthiomethyl)-pyrrolidin-1-yl]-pentyl}-estra-1,3,5(10)-trien-3,17β-diol
17α-Methyl-7α-{5-[2-(4,4,5,5,5-pentafluorpentylthiomethyl)-pyrrolidin-1-yl]-pentyl}-estra-1,3,5(10)-trien-3,17β-diol
11β-Fluor-7α-{5-[2-(4,4,5,5,5-pentafluorpentylthiomethyl)-pyrrolidin-1-yl]-pentyl}-estra-1,3,5(10)-trien-3,17β-diol
11β-Fluor-17α-methyl-7α-{5-[(2S)-2-(4,4,5,5,5-pentafluorpentylthiomethyl)-pyrrolidin-1-yl]-pentyl}-estra-1,3,5(10)-trien-3,17β-diol
11β-Fluor-17α-methyl-7α-{5-[(2S)-2-(4,4,5,5,5-pentafluorpentansulfinylmethyl)-pyrrolidin-1-yl]-pentyl}-estra-1,3,5(10)-trien-3,17β-diol
11β-Fluor-17α-methyl-7α-{5-[(2S)-2-(4,4,5,5,5-pentafluorpentansulfonylmethyl)-pyrrolidin-1-yl]-pentyl}-estra-1,3,5(10)-trien-3,17β-diol
7α-{5-[(2S)-2-(4,4,5,5,5-Pentafluorpentansulfinylmethyl)-pyrrolidin-1-yl]-pentyl-estra-1,3,5(10)-trien-3,17β-diol
7α-{5-[(2S)-2-(4,4,5,5,5-Pentafluorpentansulfonylmethyl)-pyrrolidin-1-yl]-pentyl-estra-1,3,5(10)-trien-3,17β-diol
11β-Fluor-17α-methyl-7α-{5-[N-methyl-N-3-(4,4,5,5,5-pentafluorpentylthio)-propylamino]-pentyl}-estra-1,3,5(10)-trien-3,17β-diol
11β-Fluor-17α-methyl-7α-{5-[N-methyl-N-3-(4,4,5,5,5-pentafluorpentansulfinyl)-propylamino]-pentyl}-estra-1,3,5(10)-trien-3,17β-diol
11β-Fluor-17α-methyl-7α-{5-[N-methyl-N-3-{4,4,5,5,5-pentafluorpentansulfonyl)-propylamino]-pentyl}-estra-1,3,5(10)-trien-3,17β-diol
11β-Fluor-7α-{5-[N-methyl-N-2-(4,4,5,5,5-pentafluorpentansulfonyl)-ethylamino]-pentyl}-estra-1,3,5(10)-trien-3,17β-diol
11β-Fluor-7α-{5-[N-methyl-N-3-(4,4,5,5,5-pentafluorpentylthio)-propylamino)-pentyl}-3-hydroxy-estra-1,3,5(10)-trien-17-on
11β-Fluor-7α-{6-[N-methyl-N-3-(4,4,5,5,5-pentafluorpentylthio)-propylamino]-hexyl}-estra-1,3,5(10)-trien-3,17β-diol
11β-Fluor-7α-{6-[N-methyl-N-3-(4,4,5,5,5-pentafluorpentansulfinyl)-propylamino]-hexyl}-estra-1,3,5(10)-trien-3,17β-diol
11β-Fluor-7α-(5-{[N-3-(furan-2-ylmethylthio)-propyl]-N-methyl-amino}-pentyl)-estra-1,3,5(10)-trien-3,17β-diol
11β-Fluor-7α-(5-{N-methyl-[N-3-(thiophen-2-ylmethylthio)-propyl]-amino}-pentyl)-estra-1,3,5(10)-trien-3,17β-diol
11β-Fluor-7α-{5-[(2S)-2-(4-trifluormethyphenylthiomethyl)-pyrrolidin-1-yl]-pentyl}-estra-1,3,5(10)-trien-3,17β-diol
11β-Fluor-17α-methyl-7α-{5-[(2S)-2-(4,4,5,5,5-pentafluorpentansulfinylmethyl)-pyrrolidin-1-yl]-pentyl}-estra-1,3,5(10)-trien-3,17β-diol
11β-Fluor-17α-methyl-7α-{5-[(2R)-2-(4,4,5,5,5-pentafluorpentylthiomethyl)-pyrrolidin-1-yl]-pentyl}-estra-1,3,5(10)-trien-3,17β-diol
11β-Fluor-7α-{5-[(2S)-2-(4,4,5,5,5-pentafluorpentylthiomethyl)-pyrrolidin-1-yl]-pentyl}-estra-1,3,5(10)-trien-3,17β-diol
11β-Fluor-7α-{5-[(2S)-2-(4,4,5,5,5-pentafluorpentansulfinylmethyl)-pyrrolidin-1-yl]-pentyl}-estra-1,3,5(10)-trien-3,17β-diol
11β-Fluor-7α-{5-[(2S)-2-(4,4,5,5,5-pentafluorpentansulfonylmethyl)-pyrrolidin-1-yl]-pentyl}-estra-1,3,5(10)-trien-3,17β-diol.

**41.** Pharmazeutische Präparate enthaltend mindestens eine Verbindung der allgemeinen Formel 1 gemäß Anspruch 1 sowie einen pharmazeutisch verträglichen Träger.

**42.** Verwendung der Verbindungen der allgemeinen Formel I gemäß Anspruch 1 zur Herstellung von Arzneimitteln.

## Claims

**1.** Substituted 7α-(ξ-aminoalkyl)estratrienes of the general formula I

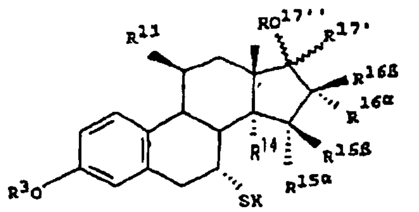

(I)

in which
the side chain SK is a radical of the part-formula

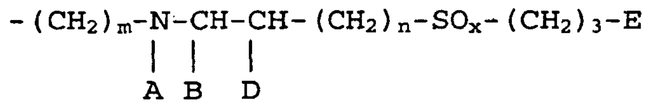

where

m is 4, 5 or 6,
n is 0, 1 or 2,
x is 0, 1 or 2,
A is a hydrogen atom or a $C_{1-5}$-alkyl group,
B and D are each a hydrogen atom, or
A and B together are an alkylene group $-(CH_2)_p-$ with p = 2, 3, 4 or 5 and D is a hydrogen atom or
A and D together are an alkylene group $-(CH_2)_q-$ with q = 2, 3 or 4 and B is a hydrogen atom, and
E is an unsubstituted or mono- to pentafluorinated ethyl radical or

the terminal substituent $-(CH_2)_3$-E in the side chain is replaced by an optionally substituted aryl or heteroaryl radical which is linked directly or via a mono-, di- or trimethylene group to the sulphur atom,

$R^3$ is a hydrogen atom, a hydrocarbon radical with up to 8 carbon atoms or a radical of the part-formula $R^{3'}$-C(O)-, in which $R^{3'}$ is a hydrogen atom or a hydrocarbon radical of up to 8 carbon atoms or a phenyl radical,
$R^{11}$ is a hydrogen atom, a halogen atom or a nitrooxy group $-O-NO_2$,
$R^{14}$, $R^{15\alpha}$, $R^{15\beta}$, $R^{16\alpha}$ and $R^{16\beta}$ are each a hydrogen atom or
$R^{14}$ and $R^{15\alpha}$ are an additional bond or a methylene bridge, or
$R^{15\beta}$ is a methyl group and $R^{15\alpha}$ is a hydrogen atom, or
$R^{15\alpha}$ and $R^{15\beta}$ are each a methyl group, or
$R^{15\beta}$ and $R^{16\beta}$ together are a methylene bridge, or
$R^{16\alpha}$ or $R^{16\beta}$ is a halogen atom or
$R^{16\alpha}$ and $R^{16\beta}$ together are a methylidene group
and the other substituents $R^{14}$ $R^{15\alpha}$, $R^{15\beta}$, $R^{16\alpha}$ and $R^{16\beta}$ are each a hydrogen atom,
$R^{17'}$ in the $\alpha$ or $\beta$ position is a hydrogen atom, a $C_{1-5}$-alkyl, $C_{2-5}$-alkenyl or $C_{2-5}$-alkynyl group or a trifluoromethyl group and
$R^{17''}$ is a hydrogen atom or a radical of the part-formula $R^{17}$-C(O)-, in which $R^{17}$ is a hydrogen atom or a hydrocarbon radical with up to 8 carbon atoms, or,
if $R^{17'}$ is in the $\alpha$ position, $R^{17'}$ together with $R^{14}$ is an ethanol bridge,

with the proviso that if it is not the case that A and B together are $-(CH_2)_p-$ or A and D together are $-(CH_2)_q-$, at least one of the substituents $R^{11}$, $R^{14}$, $R^{15\alpha}$, $R^{15\beta}$, $R^{16\alpha}$ and $R^{16\beta}$ is not a hydrogen atom,
and the physiologically tolerated addition salts thereof with organic and inorganic acids.

**2.** Estratrienes according to Claim 1, in which m is 5.

**3.** Estratrienes according to Claim 1, in which n is 0.

**4.** Estratrienes according to Claim 1, in which n is 1.

**5.** Estratrienes according to Claim 1, in which n is 2.

**6.** Estratrienes according to Claim 1, in which A is a methyl group.

**7.** Estratrienes according to Claim 1, in which x is 0.

**8.** Estratrienes according to Claim 1, in which x is 1.

**9.** Estratrienes according to Claim 1, in which x is 2.

**10.** Estratrienes according to Claim 6, in which n is 1.

**11.** Estratrienes according to Claim 1, in which A and B together is-$(CH_2)_3$.

**12.** Estratrienes according to Claim 11, in which n is 0.

**13.** Estratrienes according to Claim 12, in which x is 0.

**14.** Estratrienes according to Claim 1, in which E is a perfluoroethyl radical.

**15.** Estratrienes according to Claim 1, in which $R^3$ is a hydrogen atom.

**16.** Estratrienes according to Claim 1, in which $R^3$ is a methyl group.

**17.** Estratrienes according to Claim 1, in which $R^3$ is an acetyl group.

**18.** Estratrienes according to Claim 1, in which $R^{11}$ is a hydrogen atom.

**19.** Estratrienes according to Claim 1, in which $R^{11}$ is a fluorine atom.

**20.** Estratrienes according to Claim 1, in which $R^{11}$ is a nitrooxy group.

**21.** Estratrienes according to Claim 1, in which $R^{14}$ is a hydrogen atom.

**22.** Estratrienes according to Claim 1, in which $R^{14}$ together with $R^{15\alpha}$ forms an additional bond.

**23.** Estratrienes according to Claim 1, in which $R^{15\beta}$ is a methyl group.

**24.** Estratrienes according to Claim 1, in which $R^{15\beta}$ and $R^{16\beta}$ together form a methylene bridge.

**25.** Estratrienes according to Claim 1, in which $R^{16\alpha}$ is a halogen atom (F, Cl, Br, I).

**26.** Estratrienes according to Claim 25, in which $R^{16\alpha}$ is a fluorine atom.

**27.** Estratrienes according to Claim 1, in which $R^{16\alpha}$ and $R^{16\beta}$ together form a methylidene group.

**28.** Estratrienes according to Claim 1, in which $R^{17}$ is in the $\alpha$ position.

**29.** Estratrienes according to Claim 28, in which $R^{17}$ is a trifluoromethyl group.

**30.** Estratrienes according to Claim 28, in which $R^{17}$ is a methyl group.

**31.** Estratrienes according to Claim 28, in which $R^{17}$ is a hydrogen atom.

**32.** Estratrienes according to Claim 1, in which $R^{17}$ is in the $\beta$ position.

**33.** Estratrienes according to Claim 32, in which $R^{17}$ is a methyl group.

**34.** Estratrienes according to Claim 32, in which $R^{17}$ is a hydrogen atom.

**35.** Estratrienes according to Claim 28, in which $R^{14}$ and $R^{17}$ together form an ethano bridge.

**36.** Estratrienes according to Claim 1, in which SK is a radical of the part-formula

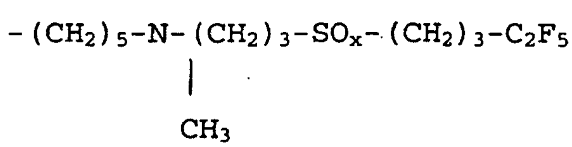

where x = 0, 1 or 2.

**37.** Estratrienes according to Claim 13, in which m is 5, E is a perfluoroethyl radical and the conformation of the 2-carbon atom of the heterocycle is R.

**38.** Estratrienes according to Claim 13, in which m is 5, E is a perfluoroethyl radical and the conformation of the 2-carbon atom of the heterocycle is S.

**39.** Estratrienes according to Claim 37, in which $R^{17}$ is in the α position and is a hydrogen atom.

**40.** Estratrienes according to claim 1, namely

14,17-ethano-7α-{5-[N-methyl-N-3-(4,4,5,5,5-pentafluoropentylthio)propylamino]pentyl}estra-1,3,5(10)-triene-3,17β-diol
14,17-ethano-7α-{5-[N-methyl-N-3-(4,4,5,5,5-pentafluoropentanesulphinyl)propylamino]pentyl}estra-1,3,5(10)-triene-3,17β-diol
3,17β-diacetoxy-14α,17α-ethano-7α-{5-[N-methyl-N-3-(4,4,5,5,5-pentafluoropentylthio)propylamino]pentyl}estra-1,3,5(10)-triene
14, 17-ethano-7α-{5-[N-methyl-N-3-(4,4,5,5,5-pentafluoropentanesulphonyl)propylamino]pentyl}estra-1,3,5(10)-triene-3,17β-diol
17α-trifluoromethyl-7α-{5-[N-methyl-N-3-(4,4,5,5,5-pentafluoropentylthio)propylamino]pentyl}estra-1,3,5(10)-triene-3,17β-diol
15β, 16β-methano-17α-methyl-7α-{5-[N-methyl-N-3-(4,4,5,5,5-pentafluoropentylthio)propylamino]pentyl}estra-1,3,5(10)-triene-3,17β-diol
15β,16β-methano-17α-methyl-7α-{5-[N-methyl-N-3-(4,4,5,5,5-pentafluoropentanesulphinyl)propylamino]pentyl}estra-1,3,5(10)-triene-3,17β-diol
15β,16β-methano-7α-{5-[N-methyl-N-3-(4,4,5,5,5-pentafluoropentylthio)propylamino]pentyl}estra-1,3,5(10)-triene-3,17β-diol
15β,16β-methano-7α-{5-[N-methyl-N-3-(4,4,5,5,5-pentafluoropentanesulphinyl)propylamino]pentyl}estra-1,3,5(10)-triene-3,17β-diol
15β-methyl-7α-(5-[N-methyl-N-3-(4,4,5,5,5-pentafluoropentylthio)propylamino]pentyl}estra-1,3,5(10)-triene-3,17β-diol
15β,17α-dimethyl-7α-{5-[N-methyl-N-3-(4,4,5,5,5-pentafluoropentylthio)propylamino]pentyl}estra-1,3,5(10)-triene-3,17β-diol
11β-fluoro-7α-{5-[N-methyl-N-3-(4,4,5,5,5-pentafluoropentylthio)propylamino]pentyl}estra-1,3,5(10)-triene-3,17β-diol
11β-fluoro-7α-{5-[N-methyl-N-3-(4,4,5,5,5-pentafluoropentanesulphinyl)propylamino]pentyl}estra-1,3,5(10)-triene-3,17β-diol
11β-fluoro-7α-{5-[N-methyl-N-3-(4,4,5,5,5-pentafluoropentanesulphonyl)propylamino]pentyl}estra-1,3,5(10)-triene-3,17β-diol
16α-fluoro-17α-methyl-7α-{5-[N-methyl-N-3-(4,4,5,5,5-pentafluoropentylthio)propylamino]pentyl}estra-1,3,5(10)-triene-3,17β-diol
16α-fluoro-17β-methyl-7α-{5-[N-methyl-N-3-(4,4,5,5,5-pentafluoropentylthio)propylamino]pentyl}estra-1,3,5(10)-triene-3,17α-diol
16α-fluoro-17α-methyl-7α-{5-[N-methyl-N-3-(4,4,5,5,5-pentafluoropentanesulphinyl)propylamino]pentyl}estra-1,3,5(10)-triene-3,17β-diol
16α-fluoro-17α-methyl-7α-{5-[N-methyl-N-3-(4,4,5,5,5-pentafluoropentanesulphonyl)propylamino]pentyl}estra-1,3,5(10)-triene-3,17β-diol
16α-fluoro-7α-{5-[N-methyl-N-3-(4,4,5,5,5-pentafluoropentylthio)propylamino]pentyl}estra-1,3,5(10)-triene-3,17β-diol
16α-fluoro-7α-{5-[N-methyl-N-3-(4,4,5,5,5-pentafluoropentylthio)propylamino]pentyl}estra-1,3,5(10)-triene-3,17α-diol
16α-fluoro-7α-{5-[N-methyl-N-3-(4,4,5,5,5-pentafluoropentanesulphinyl)propylamino]pentyl}estra-1,3,5(10)-triene-3,17β-diol
16α-fluoro-7α-{5-[N-methyl-N-3-(4,4,5,5,5-pentafluoropentanesulphonyl)propylamino]pentyl}estra-1,3,5(10)-triene-3,17β-diol
7α-{5-[N-methyl-N-3-(4,4,5,5,5-pentafluoropentylthio)propylamino]pentyl}estra-1,3,5(10),14-tetraene-3,17β-diol

7α-{5-[N-methyl-N-3-(4,4,5,5,5-pentafluoropentanesulphinyl)propylamino]pentyl}estra-1,3,5(10),14-tetraene-3,17β-diol
7α-{5-[N-methyl-N-3-(4,4,5,5,5-pentafluoropentanesulphonyl)propylamino]pentyl}estra-1,3,5(10),14-tetraene-3,17β-diol
7α-{5-[(2S)-2-(4,4,5,5,5-pentafluoropentylthiomethyl)pyrrolidin-1-yl]pentyl}estra-1,3,5(10)-triene-3,17β-diol
7α-{5-[(2R)-2-(4,4,5,5,5-pentafluoropentylthiomethyl)pyrrolidin-1-yl]pentyl}estra-1,3,5(10)-triene-3,17β-diol
17α-methyl-7α-(5-[2-(4,4,5,5,5-pentafluoropentylthiomethyl)pyrrolidin-1-yl]pentyl}estra-1,3,5(10)-triene-3,17β-diol
11β-fluoro-7α-{5-[2-(4,4,5,5,5-pentafluoropentylthiomethyl)pyrrolidin-1-yl]pentyl}estra-1,3,5(10)-triene-3,17β-diol
11β-fluoro-17α-methyl-7α-{5-[(2S)-2-(4,4,5,5,5-pentafluoropentylthiomethyl)pyrrolidin-1-yl]pentyl}estra-1,3,5(10)-triene-3,17β-diol
11β-fluoro-17α-methyl-7α-{5-[(2S)-2-(4,4,5,5,5-pentafluoropentanesulphinylmethyl)pyrrolidin-1-yl]pentyl}estra-1,3,5(10)-triene-3,17β-diol
11β-fluoro-17α-methyl-7α-{5-[(2S)-2-(4,4,5,5,5-pentafluoropentanesulphonylmethyl)pyrrolidin-1-yl]pentyl}estra-1,3,5(10)-triene-3,17β-diol
7α-{5-[(2S)-2-(4,4,5,5,5-pentafluoropentanesulphinylmethyl)pyrrolidin-1-yl]pentyl}estra-1,3,5(10)-triene-3,17β-diol
7α-{5-[(2S)-2-(4,4,5,5,5-pentafluoropentanesulphonylmethyl)pyrrolidin-1-yl]pentyl}estra-1,3,5(10)-triene-3,17β-diol
11β-fluoro-17α-methyl-7α-{5-[N-methyl-N-3-(4,4,5,5,5-pentafluoropentylthio)propylamino]pentyl}estra-1,3,5(10)-triene-3,17β-diol
11β-fluoro-17α-methyl-7α-(5-[N-methyl-N-3-(4,4,5,5,5-pentafluoropentanesulphinyl)propylamino]pentyl}-estra-1,3,5(10)-triene-3,17β-diol
11β-fluoro-17α-methyl-7α-{5-[N-methyl-N-3-(4,4,5,5,5-pentafluoropentanesulphonyl)propylamino]pentyl}-estra-1,3,5(10)-triene-3,17β-diol
11β-fluoro-7α-{5-[N-methyl-N-2-(4,4,5,5,5-pentafluoropentanesulphonyl)ethylamino]pentyl}estra-1,3,5(10)-triene-3,17β-diol
11β-fluoro-7α-{5-[N-methyl-N-3-(4,4,5,5,5-pentafluoropentylthio)propylamino]pentyl}-3-hydroxy-estra-1,3,5(10)-trien-17-one
11β-fluoro-7α-{6-[N-methyl-N-3-(4,4,5,5,5-pentafluoropentylthio)propylamino]hexyl}estra-1,3,5(10)-triene-3,17β-diol
11β-fluoro-7α-{6-[N-methyl-N-3-(4,4,5,5,5-pentafluoropentanesulphinyl)propylamino]hexyl}estra-1,3,5(10)-triene-3,17β-diol
11β-fluoro-7α-(5-{[N-3-(furan-2-ylmethylthio)propyl]-N-methylamino}pentyl)estra-1,3,5(10)-triene-3,17β-diol
11β-fluoro-7α-(5-{N-methyl-[N-3-(thiophen-2-yl-methylthio)propyl]amino}pentyl)estra-1,3,5(10)-triene-3,17β-diol
11β-fluoro-7α-{5-[(2S)-2-(4-trifluoromethylphenylthiomethyl)pyrrolidin-1-yl]pentyl}estra-1,3,5(10)-triene-3,17β-diol
11β-fluoro-17α-methyl-7α-{5-[(2S)-2-(4,4,5,5,5-pentafluoropentanesulphinylmethyl)pyrrolidin-1-yl]pentyl}estra-1,3,5(10)-triene-3,17β-diol
11β-fluoro-17α-methyl-7α-{5-[(2R)-2-(4,4,5,5,5-pentafluoropentylthiomethyl)pyrrolidin-1-yl]pentyl}estra-1,3,5(10)-triene-3,17β-diol
11β-fluoro-7α-{5-[(2S)-2-(4,4,5,5,5-pentafluoropentylthiomethyl)pyrrolidin-1-yl]pentyl}estra-1,3,5(10)-triene-3,17β-diol
11β-fluoro-7α-{5-[(2S)-2-(4,4,5,5,5-pentafluoropentanesulphinylmethyl)pyrrolidin-1-yl]pentyl}estra-1,3,5(10)-triene-3,17β-diol
11β-fluoro-7α-{5-[(2S)-2-(4,4,5,5,5-pentafluoropentanesulphonylmethyl)pyrrolidin-1-yl]pentyl}estra-1,3,5(10)-triene-3,17β-diol

**41.** Pharmaceutical products comprising at least one compound of the general formula I according to Claim 1 and a pharmaceutically acceptable carrier.

**42.** Use of the compounds of the general formula I according to Claim 1 for producing medicaments.

## Revendications

**1.** 7α-(ξ-Aminoalkyl)estratriènes substitués de formule générale I

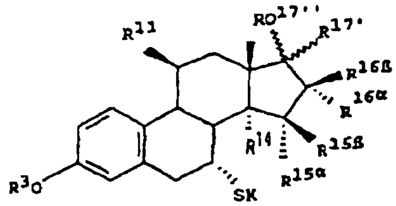

(I)

dans laquelle
la chaîne latérale SK représente un radical de formule partielle

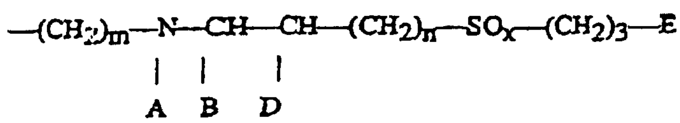

dans laquelle

m vaut 4, 5 ou 6,
n vaut 0, 1 ou 2,
x vaut 0, 1 ou 2,
A représente un atome d'hydrogène ou un groupe alkyle en $C_{1-5}$,
B et D représentent chacun un atome d'hydrogène, ou
A et B représentent conjointement un groupe alkylène $-(CH_2)_p-$ dans lequel p = 2, 3, 4 ou 5 et D représente un atome d'hydrogène ou
A et D représentent conjointement un groupe alkylène $-(CH_2)_q-$ dans lequel q = 2, 3 ou 4 et B représente un atome d'hydrogène, et
E représente un radical éthyle non substitué ou mono- à pentafluoré, ou le substituant terminal $-(CH_2)_3$-E dans la chaîne latérale est remplacé par un radical aryle ou hétéroaryle éventuellement substitué, qui est lié directement ou par l'intermédiaire d'un groupe mono-, di- ou triméthylène à l'atome de soufre,

$R^3$ représente un atome d'hydrogène, un radical hydrocarboné ayant jusqu'à 8 atomes de carbone ou un radical de formule partielle $R^{3'}$-C(O)-, dans laquelle $R^{3'}$ représente un atome d'hydrogène ou un radical hydrocarboné ayant jusqu'à 8 atomes de carbone ou un radical phényle,
$R^{11}$ représente un atome d'hydrogène, un atome d'halogène ou un groupe nitro-oxy-O-$NO_2$,
$R^{14}$, $R^{15\alpha}$, $R^{15\beta}$, $R^{16\alpha}$ et $R^{16\beta}$ représentent chacun un atome d'hydrogène ou
$R^{14}$ et $R^{15\alpha}$ représentent une liaison supplémentaire ou un pont méthylène, ou
$R^{15\beta}$ représente un groupe méthyle et $R^{15\alpha}$ représente un atome d'hydrogène, ou
$R^{15\alpha}$ et $R^{15\beta}$ représentent chacun un groupe méthyle, ou
$R^{15\beta}$ et $R^{16\beta}$ représentent conjointement un pont méthylène, ou
$R^{16\alpha}$ et $R^{16\beta}$ représentent un atome d'halogène ou
$R^{16\alpha}$ et $R^{16\beta}$ représentent conjointement un groupe méthylidène,
et les autres substituants $R^{14}$, $R^{15\alpha}$, $R^{15\beta}$, $R^{16\alpha}$ et $R^{16\beta}$ représentent chacun un atome d'hydrogène,
$R^{17'}$ représente, en position α ou β, un atome d'hydrogène, un groupe alkyle en $C_{1-5}$, un groupe alcényle en $C_{2-5}$ ou un groupe alcynyle en $C_{2-5}$, ou un groupe trifluorométhyle et
$R^{17''}$ représente un atome d'hydrogène ou un radical de formule partielle $R^{17'''}$-C(O)-, dans laquelle $R^{17'''}$ représente un atome d'hydrogène ou un radical hydrocarboné ayant jusqu'à 8 atomes de carbone, ou si $R^{17'}$ se trouve en position α, $R^{17'}$ représente, conjointement avec $R^{14}$, un pont éthanol,

à condition que si A et B ne représentent pas conjointement $-(CH_2)_p-$ ou que A et D ne représentent pas conjointement $-(CH_2)_q-$, au moins l'un des substituants $R^{11}$, $R^{14}$, $R^{15\alpha}$, $R^{15\beta}$, $R^{16\alpha}$ et $R^{16\beta}$ ne représentent pas un atome d'hydrogène,
ainsi que leurs sels d'addition physiologiquement acceptables avec des acides organiques et inorganiques.

**2.** Estratriènes selon la revendication 1, dans lesquels m vaut 5.

**3.** Estratriènes selon la revendication 1, dans lesquels n vaut 0.

**4.** Estratriènes selon la revendication 1, dans lesquels n vaut 1.

**5.** Estratriènes selon la revendication 1, dans lesquels n vaut 2.

**6.** Estratriènes selon la revendication 1, dans lesquels A représente un groupe méthyle.

**7.** Estratriènes selon la revendication 1, dans lesquels x vaut 0.

**8.** Estratriènes selon la revendication 1, dans lesquels x vaut 1.

**9.** Estratriènes selon la revendication 1, dans lesquels x vaut 2.

**10.** Estratriènes selon la revendication 6, dans lesquels n vaut 1.

**11.** Estratriènes selon la revendication 1, dans lesquels A et B représentent conjointement $-(CH_2)_3-$.

**12.** Estratriènes selon la revendication 11, dans lesquels n vaut 0.

**13.** Estratriènes selon la revendication 12, dans lesquels x vaut 0.

**14.** Estratriènes selon la revendication 1, dans lesquels E représente un radical perfluoroéthyle.

**15.** Estratriènes selon la revendication 1, dans lesquels $R^3$ représente un atome d'hydrogène.

**16.** Estratriènes selon la revendication 1, dans lesquels $R^3$ représente un groupe méthyle.

**17.** Estratriènes selon la revendication 1, dans lesquels $R^3$ représente un groupe acétyle.

**18.** Estratriènes selon la revendication 1, dans lesquels $R^{11}$ représente un atome d'hydrogène.

**19.** Estratriènes selon la revendication 1, dans lesquels $R^{11}$ représente un atome de fluor.

**20.** Estratriènes selon la revendication 1, dans lesquels $R^{11}$ représente un groupe nitro-oxy.

**21.** Estratriènes selon la revendication 1, dans lesquels $R^{14}$ représente un atome d'hydrogène.

**22.** Estratriènes selon la revendication 1, dans lesquels $R^{14}$ forme, conjointement avec $R^{15\alpha}$, une liaison supplémentaire.

**23.** Estratriènes selon la revendication 1, dans lesquels $R^{15\beta}$ représente un groupe méthyle.

**24.** Estratriènes selon la revendication 1, dans lesquels $R^{15\beta}$ et $R^{16\beta}$ forment conjointement un pont méthylène.

**25.** Estratriènes selon la revendication 1, dans lesquels $R^{16\alpha}$ représente un atome d'halogène (F, Cl, Br, I).

**26.** Estratriènes selon la revendication 25, dans lesquels $R^{16\alpha}$ représente un atome de fluor.

**27.** Estratriènes selon la revendication 1, dans lesquels $R^{16\alpha}$ et $R^{16\beta}$ forment conjointement un groupe méthylidène.

**28.** Estratriènes selon la revendication 1, dans lesquels $R^{17}$ est en position $\alpha$.

**29.** Estratriènes selon la revendication 28, dans lesquels $R^{17}$ représente un groupe trifluorométhyle.

**30.** Estratriènes selon la revendication 28, dans lesquels $R^{17}$ représente un groupe méthyle.

**31.** Estratriènes selon la revendication 28, dans lesquels $R^{17}$ représente un atome d'hydrogène.

**32.** Estratriènes selon la revendication 1, dans lesquels $R^{17}$ est en position $\beta$.

**33.** Estratriènes selon la revendication 32, dans lesquels $R^{17}$ représente un groupe méthyle.

**34.** Estratriènes selon la revendication 32, dans lesquels $R^{17}$ représente un atome d'hydrogène.

**35.** Estratriènes selon la revendication 28, dans lesquels $R^{14}$ et $R^{17}$ forment conjointement un pont éthanol.

**36.** Estratriènes selon la revendication 1, dans lesquels SK représente un radical de formule partielle

$$-(CH_2)_5-\underset{\underset{CH_3}{|}}{N}-(CH_2)_3-SO_x-(CH_2)_3-C_2F_5$$

dans laquelle x = 0, 1 ou 2.

**37.** Estratriènes selon la revendication 13, dans lesquels m = 5, E représente un radical perfluoroéthyle et la conformation de l'atome de carbone 2 de l'hétérocycle est R.

**38.** Estratriènes selon la revendication 13, dans lesquels m = 5, E représente un radical perfluoroéthyle et la conformation de l'atome de carbone 2 de l'hétérocycle est S.

**39.** Estratriènes selon la revendication 37, dans lesquels $R^{17}$ est en position $\alpha$ et représente un atome d'hydrogène.

**40.** Estratriènes selon la revendication 1, à savoir

le 14,17-éthano-7α-{5-[N-méthyl-N-3-(4,4,5,5,5-pentafluoropenthylthio)-propylamino]-pentyl}-estra-1,3,5(10)-triène-3,17β-diol
le 14,17-éthano-7α-{5-[N-méthyl-N-3-(4,4,5,5,5-pentafluoropentanesulfinyl)-propylamino]-pentyl}-estra-1,3,5(10)-triène-3,17β-diol
le 3,17β-diacétoxy-14α,17α-éthano-7α-{5-[N-méthyl-N-3-(4,4,5,5,5-pentafluoropentylthio)-propylamino]-pentyl}-estra-1,3,5(10)-triène
le 14,17-éthano-7α-{5-[N-méthyl-N-3-(4,4,5,5,5-pentafluoropentanesulfonyl)-propylamino]-pentyl)-estra-1,3,5(10)-triène-3,17β-diol
le 17α-trifluorométhyl-7α-{5-[N-méthyl-N-3-(4,4,5,5,5-pentafluoropentylthio)-propylamino]-pentyl}-estra-1,3,5(10)-triène-3,17β-diol
le 15β,16β-méthano-17α-méthyl-7α-{5-[N-méthyl-N-3-(4,4,5,5,5-pentafluoropentylthio)-propylamino]-pentyl}-estra-1,3,5(10)-triène-3,17β-diol
le 15β,16β-méthano-17α-méthyl-7α-{5-(N-méthyl-N-3-(4,4,5,5,5-pentafluoropentanesulfinyl)-propylamino]-pentyl}-estra-1,3,5(10)-triène-3,17β-diol
le 15β,16β-méthano-7α-{5-[N-méthyl-N-3-(4,4,5,5,5-pentafluoropentylthio)-propylamino]-pentyl}-estra-1,3,5(10)-triène-3,17β-diol
le 15β,16β-méthano-7α-{5-[N-méthyl-N-3-(4,4,5,5,5-pentafluoropentanesulfinyl)-propylamino]-pentyl}-estra-1,3,5(10)-triène-3,17β-diol
le 15β-méthyl-7α-{5-[N-méthyl-N-3-(4,4,5,5,5-pentafluoropentylthio)-propylamino]-pentyl}-estra-1,3,5(10)-triène-3,17β-diol
le 15β,17α-diméthyl-7α-{5-[N-méthyl-N-3-(4,4,5,5,5-pentafluoropentylthio)-propylamino]-pentyl}-estra-1,3,5(10)-triène-3,17β-diol

le 11β-fluoro-7α-{5-[N-méthyl-N-3-(4,4,5,5,5-pentafluoropentylthio)-propylamino]-pentyl}-estra-1,3,5(10)-triène-3,17β-diol

le 11β-fluoro-7α-{5-[N-méthyl-N-3-(4,4,5,5,5-pentafluoropentanesulfinyl)-propylamino]-pentyl}-estra-1,3,5(10)-triène-3,17β-diol

le 11β-fluoro-7α-{5-[N-méthyl-N-3-(4,4,5,5,5-pentafluoropentanesulfonyl)-propylamino]-pentyl}-estra-1,3,5(10)-triène-3,17β-diol

le 16α-fluoro-17α-méthyl-7α-{5-[N-méthyl-N-3-(4,4,5,5,5-pentafluoropentylthio)-propylamino]-pentyl}-estra-1,3,5(10)-triène-3,17β-diol

le 16α-fluoro-17β-méthyl-7α-{5-[N-méthyl-N-3-(4,4,5,5,5-pentafluoropentylthio)-propylamino]-pentyl}-estra-1,3,5(10)-triène-3,17α-diol

le 16α-fluoro-17α-méthyl-7α-{5-[N-méthyl-N-3-(4,4,5,5,5-pentafluoropentanesulfinyl)-propylamino]-pentyl}-estra-1,3,5(10)-triène-3,17β-diol

le 16α-fluoro-17α-méthyl-7α-{5-[N-méthyl-N-3-(4,4,5,5,5-pentafluoropentanesulfonyl)-propylamino]-pentyl}-estra-1,3,5(10)-triène-3,17β-diol

le 16α-fluoro-7α-{5-[N-méthyl-N-3-(4,4,5,5,5-pentafluoropentylthio)-propylamino]-pentyl}-estra-1,3,5(10)-triène-3,17β-diol

le 16α-fluoro-7α-{5-[N-méthyl-N-3-(4,4,5,5,5-pentafluoropentylthio)-propylamino]-pentyl}-estra-1,3,5(10)-triène-3,17α-diol

le 16α-fluoro-7α-{5-[N-méthyl-N-3-(4,4,5,5,5-pentafluoropentanesulfinyl)-propylamino]-pentyl}-estra-1,3,5(10)-triène-3,17β-diol

le 16α-fluoro-7α-{5-[N-méthyl-N-3-(4,4,5,5,5-pentafluoropentanesulfonyl)-propylamino]-pentyl}-estra-1,3,5(10)-triène-3,17β-diol

le 7α-{5-[N-méthyl-N-3-(4,4,5,5,5-pentafluoropentylthio)-propylamino]-pentyl}-estra-1,3,5(10),14-tétraène-3,17β-diol

le 7α-{5-[N-méthyl-N-3-(4,4,5,5,5-pentafluoropentanesulfinyl)-propylamino]-pentyl}-estra-1,3,5(10),14-tétraène-3,17β-diol

le 7α-{5-[N-méthyl-N-3-(4,4,5,5,5-pentafluoropentanesulfonyl)-propylamino]-pentyl}-estra-1,3,5(10),14-tétraène-3,17β-diol

le 7α-{5-[(2S)-2-(4,4,5,5,5-pentafluoropentylthiométhyl)-pyrrolidin-1-yl]-pentyl}-estra-1,3,5(10)-triène-3,17β-diol

le 7α-{5-[(2R)-2-(4,4,5,5,5-pentafluoropentylthiométhyl)-pyrrolidin-1-yl]-pentyl}-estra-1,3,5(10)-triène-3,17β-diol

le 17α-méthyl-7α-{5-[2-(4,4,5,5,5-pentafluoropentylthiométhyl)-pyrrolidin-1-yl]-pentyl}-estra-1,3,5(10)-triène-3,17β-diol

le 11β-fluoro-7α-{5-[2-(4,4,5,5,5-pentafluoropentylthiométhyl)-pyrrolidin-1-yl]-pentyl}-estra-1,3,5(10)-triène-3,17β-diol

le 11β-fluoro-17α-méthyl-7α-{5-[(2S)-2-(4,4,5,5,5-pentafluoropentylthiométhyl)-pyrrolidin-1-yl]-pentyl}-estra-1,3,5(10)-triène-3,17β-diol

le 11β-fluoro-17α-méthyl-7α-{5-[(2S)-2-(4,4,5,5,5-pentafluoropentanesulfinylméthyl)-pyrrolidin-1-yl]-pentyl}-estra-1,3,5(10)-triène-3,17β-diol

le 11β-fluoro-17α-méthyl-7α-{5-[(2S)-2-(4,4,5,5,5-pentafluoropentanesulfonylméthyl)-pyrrolidin-1-yl]-pentyl}-estra-1,3,5(10)-triène-3,17β-diol

le 7α-{5-[(2S)-2-(4,4,5,5,5-pentafluoropentanesulfinylméthyl)-pyrrolidin-1-yl]-pentyl}-estra-1,3,5(10)-triène-3,17β-diol

le 7α-{5-[(2S)-2-(4,4,5,5,5-pentafluoropentanesulfonylméthyl)-pyrrolidin-1-yl]-pentyl}-estra-1,3,5(10)-triène-3,17β-diol

le 11β-fluoro-17α-méthyl-7α-{5-[N-méthyl-N-3-(4,4,5,5,5-pentafluoropentylthio)-propylamino]-pentyl}-estra-1,3,5(10)-triène-3,17β-diol

le 11β-fluoro-17α-méthyl-7α-{5-[N-méthyl-N-3-(4,4,5,5,5-pentafluoropentanesulfinyl)-propylamino]-pentyl}-estra-1,3,5(10)-triène-3,17β-diol

le 11β-fluoro-17α-méthyl-7α-{5-[N-méthyl-N-3-(4,4,5,5,5-pentafluoropentanesulfonyl)-propylamino]-pentyl}-estra-1,3,5(10)-triène-3,17β-diol

le 11β-fluoro-7α-{5-[N-méthyl-N-2-(4,4,5,5,5-pentafluoropentanesulfonyl)-éthylamino]-pentyl}-estra-1,3,5(10)-triène-3,17β-diol

le 11β-fluoro-7α-{5-[N-méthyl-N-3-(4,4,5,5,5-pentafluoropentylthio)-propylamino]-pentyl}-3-hydroxy-estra-1,3,5(10)-trién-17-one

le 11β-fluoro-7α-(6-[N-méthyl-N-3-(4,4,5,5,5-pentafluoropentylthio)-propylamino]-hexyl}-estra-1,3,5(10)-triène-3,17β-diol

le 11β-fluoro-7α-{6-[N-méthyl-N-3-(4,4,5,5,5-pentafluoropentanesulfinyl)-propylamino]-hexyl}-estra-1,3,5(10)-triène-3,17β-diol
le 11β-fluoro-7α-(5-{[N-3-(furan-2-ylméthylthio)-propyl]-N-méthyl-amino}-pentyl)-estra-1,3,5(10)-triène-3,17β-diol
le 11β-fluoro-7α-(5-{N-méthyl-[N-3-(thiophén-2-ylméthylthio)-propyl]-amino}-pentyl)-estra-1,3,5(10)-triène-3,17β-diol
le 11β-fluoro-7α-{5-[(2S)-2-(4-trifluorométhylphénylthiométhyl)-pyrrolidin-1-yl]-pentyl}-estra-1,3,5(10)-trién-3,17β-diol
le 11β-fluoro-17α-méthyl-7α-{5-[(2S)-2-(4,4,5,5,5-pentafluoropentanesulfinylméthyl)-pyrrolidin-1-yl]-pentyl}-estra-1,3,5(10)-triène-3,17β-diol
le 11β-fluoro-17α-méthyl-7α-{5-[(2R)-2-(4,4,5,5,5-pentafluoropentylthiométhyl)-pyrrolidin-1-yl]-pentyl}-estra-1,3,5(10)-triène-3,17β-diol
le 11β-fluoro-7α-{5-[(2S)-2-(4,4,5,5,5-pentafluoropentylthiométhyl)-pyrrolidin-1-yl]-pentyl}-estra-1,3,5(10)-triène-3,17β-diol
le 11β-fluoro-7α-{5-[(2S)-2-(4,4,5,5,5-pentafluoropentanesulfinylméthyl)-pyrrolidin-1-yl]-pentyl}-estra-1,3,5(10)-triène-3,17β-diol
le 11β-fluoro-7α-{5-[(2S)-2-(4,4,5,5,5-pentafluoropentanesulfonylméthyl)-pyrrolidin-1-yl]-pentyl}-estra-1,3,5(10)-triène-3,17β-diol.

**41.** Préparations pharmaceutiques comprenant au moins un composé de formule générale I selon la revendication 1 ainsi qu'un support pharmaceutiquement acceptable.

**42.** Utilisation des composés de formule générale I selon la revendication 1 pour la préparation de médicaments.

DMBA - induzierte Mamma Tumore p.o. Fig. 1